(19) Europäisches Patentamt
European Patent Office
Office européen des brevets

(11) EP 3 548 481 B1

(12) EUROPÄISCHE PATENTSCHRIFT

(45) Veröffentlichungstag und Bekanntmachung des Hinweises auf die Patenterteilung:
**23.08.2023 Patentblatt 2023/34**

(21) Anmeldenummer: **17816571.8**

(22) Anmeldetag: **30.11.2017**

(51) Internationale Patentklassifikation (IPC):
***C07D 403/14*** (2006.01) ***C07D 409/04*** (2006.01)
***C07D 409/10*** (2006.01) ***C07D 471/04*** (2006.01)
***C07D 491/04*** (2006.01) ***C07D 493/04*** (2006.01)
***H10K 85/20*** (2023.01) ***H10K 50/00*** (2023.01)
***H10K 85/60*** (2023.01)

(52) Gemeinsame Patentklassifikation (CPC):
**C07D 403/04; C07D 225/04; C07D 403/10; C07D 403/14; C07D 405/04; C07D 405/10; C07D 405/14; C07D 409/04; C07D 409/10; C07D 409/14; C07D 471/04; C07D 491/048; C07D 493/04; C07D 495/04; C09K 11/06;** (Forts.)

(86) Internationale Anmeldenummer:
**PCT/EP2017/080913**

(87) Internationale Veröffentlichungsnummer:
**WO 2018/100029 (07.06.2018 Gazette 2018/23)**

(54) **HETEROCYCLISCHE VERBINDUNGEN ZUR VERWENDUNG IN ELEKTRONISCHEN VORRICHTUNGEN**

HETEROCYCLIC COMPOUNDS FOR USE IN ELECTRONIC DEVICES

COMPOSÉS HÉTÉROCYCLIQUES À UTILISER DANS DES DISPOSITIFS ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.12.2016 EP 16201964**

(43) Veröffentlichungstag der Anmeldung:
**09.10.2019 Patentblatt 2019/41**

(73) Patentinhaber: **Merck Patent GmbH**
**64293 Darmstadt (DE)**

(72) Erfinder:
- **STOESSEL, Philipp**
  **60389 Frankfurt am Main (DE)**
- **KOENEN, Nils**
  **64347 Griesheim (DE)**
- **HARBACH, Philipp**
  **64367 Muehltal (DE)**
- **JOOSTEN, Dominik**
  **60487 Frankfurt am Main (DE)**
- **PARHAM, Amir**
  **60486 Frankfurt am Main (DE)**
- **JATSCH, Anja**
  **60489 Frankfurt am Main (DE)**

(56) Entgegenhaltungen:
**WO-A1-2011/088877** **WO-A1-2016/078747**
**KR-A- 20160 079 546** **US-A1- 2017 018 721**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)
**H10K 50/00; H10K 85/615; H10K 85/622; H10K 85/626; H10K 85/631; H10K 85/654; H10K 85/6572; H10K 85/6574; H10K 85/6576;**
H10K 50/11; H10K 50/15; H10K 50/18; H10K 85/342; H10K 2101/10; H10K 2101/90; Y02E 10/549

**Beschreibung**

Die vorliegende Erfindung beschreibt heterocyclische Verbindungen, insbesondere zur Verwendung in elektronischen Vorrichtungen. Die Erfindung betrifft ferner ein Verfahren zur Herstellung der erfindungsgemäßen Verbindungen sowie elektronische Vorrichtungen enthaltend diese Verbindungen.

Der Aufbau organischer Elektrolumineszenzvorrichtungen (OLEDs), in denen organische Halbleiter als funktionelle Materialien eingesetzt werden, ist im Stand der Technik allgemein bekannt. Als emittierende Materialien werden häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können zu deutlichen Verbesserungen elektrolumineszierender Vorrichtungen führen.

Gemäß dem Stand der Technik werden als Matrixmaterialien für phosphoreszierende Verbindungen sowie als Elektronentransportmaterialien häufig aromatische oder heteroaromatische Verbindungen eingesetzt, wie zum Beispiel Triarylamin- oder Carbazolderivate. Weiterhin werden als Matrixmaterialien auch Triazinderivate oder Pyrimidinderivate verwendet, wobei auch Verbindungen bekannt sind, die sowohl Carbazol-Strukturen als auch Strukturen aufweisen, die von Triazinen oder Pyrimidinen abgeleitet sind.

Generell besteht bei diesen Materialien, beispielsweise für die Verwendung als Matrixmaterialien noch Verbesserungsbedarf, insbesondere in Bezug auf die Lebensdauer, aber auch in Bezug auf die Effizienz und die Betriebsspannung der Vorrichtung.

Aufgabe der vorliegenden Erfindung ist daher die Bereitstellung von Verbindungen, welche sich für den Einsatz in einer organischen elektronischen Vorrichtung, insbesondere in einer organischen Elektrolumineszenzvorrichtung eignen, und welche bei Verwendung in dieser Vorrichtung zu guten Device-Eigenschaften führen, sowie die Bereitstellung der entsprechenden elektronischen Vorrichtung.

Insbesondere ist es die Aufgabe der vorliegenden Erfindung Verbindungen zur Verfügung zu stellen, die zu hoher Lebensdauer, guter Effizienz und geringer Betriebsspannung führen. Gerade auch die Eigenschaften der Matrixmaterialien haben einen wesentlichen Einfluss auf die Lebensdauer und die Effizienz der organischen Elektrolumineszenzvorrichtung.

Eine weitere Aufgabe der vorliegenden Erfindung kann darin gesehen werden, Verbindungen bereitzustellen, welche sich für den Einsatz in einer phosphoreszierenden oder fluoreszierenden OLED eignen, insbesondere als Matrixmaterial. Insbesondere ist es eine Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen. Ferner sollten fluoreszierende Emitter bereitgestellt werden, die ausgezeichnete Eigenschaften aufweisen.

Weiterhin sollten sich die Verbindungen möglichst einfach verarbeiten lassen, insbesondere eine gute Löslichkeit und Filmbildung zeigen. Beispielsweise sollten die Verbindungen eine erhöhte Oxidationsstabilität und eine verbesserte Glasübergangstemperatur zeigen.

Eine weitere Aufgabe kann darin gesehen werden, elektronische Vorrichtungen mit einer ausgezeichneten Leistungsfähigkeit möglichst kostengünstig und in konstanter Qualität bereitzustellen Weiterhin sollten die elektronischen Vorrichtungen für viele Zwecke eingesetzt oder angepasst werden können. Insbesondere sollte die Leistungsfähigkeit der elektronischen Vorrichtungen über einen breiten Temperaturbereich erhalten bleiben.

Überraschend wurde gefunden, dass bestimmte, nachfolgend näher beschriebene Verbindungen diese Aufgaben lösen. Die Verwendung der Verbindungen führt zu sehr guten Eigenschaften organischer elektronischer Vorrichtungen, insbesondere von organischen Elektrolumineszenzvorrichtungen, insbesondere hinsichtlich der Lebensdauer, der Effizienz und der Betriebsspannung. Elektronische Vorrichtungen, insbesondere organische Elektrolumineszenzvorrichtungen, welche derartige Verbindungen enthalten, sowie die entsprechenden bevorzugten Ausführungsformen sind daher Gegenstand der vorliegenden Erfindung.

Gegenstand der vorliegenden Erfindung ist daher eine Verbindung gemäß Anspruch 1.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende

Schema verdeutlicht.

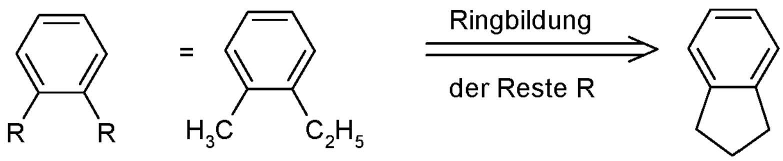


Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

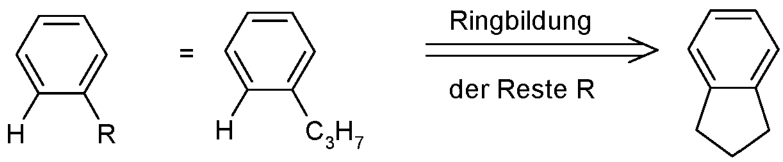


Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindenstens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 60 C-Atome, vorzugsweise 6 bis 40 C-Atome; eine Heteroarylgruppe im Sinne dieser Erfindung enthält 2 bis 60 C-Atome, vorzugsweise 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl-oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome, vorzugsweise 6 bis 40 C-Atome im Ringsystem. Ein heteroaromatisches Ringsystem im Sinne dieser Erfindung enthält 1 bis 60 C-Atome, vorzugsweise 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer $C_1$- bis $C_{20}$-Alkylgruppe, in der auch einzelne H-Atome oder $CH_2$-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1 -Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, vorzugsweise 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Anspruchsgemäß ist es vorgesehen, dass in der Struktur der Formel (I) höchstens 2 der Symbole $X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ ungleich CH oder CD sind.

In einer bevorzugten Ausgestaltung können die erfindungsgemäßen Verbindungen mindestens eine Struktur gemäß den Formeln (I-1) enthalten

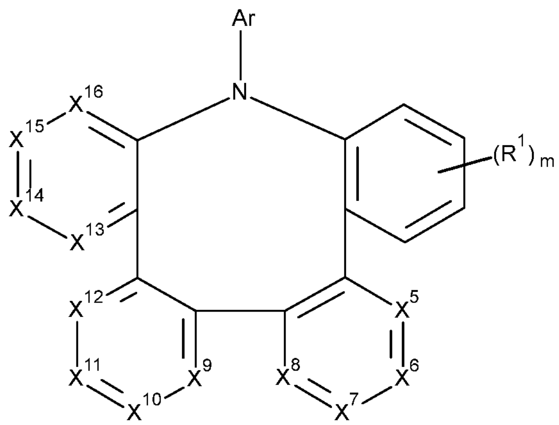

Formel (I-1)

wobei m 0, 1 oder 2 ist und die verwendeten Symbole $X^5$, $X^6$, $X^7$, $X^8$, $X^9$, $X^{10}$, $X^{11}$, $X^{12}$, $X^{13}$, $X^{14}$, $X^{15}$, $X^{16}$, $R^1$ und Ar die zuvor, insbesondere für Formel (I) genannte Bedeutung aufweisen.

Vorzugsweise können die erfindungsgemäßen Verbindungen Strukturen gemäß der Formel (I-2) umfassen

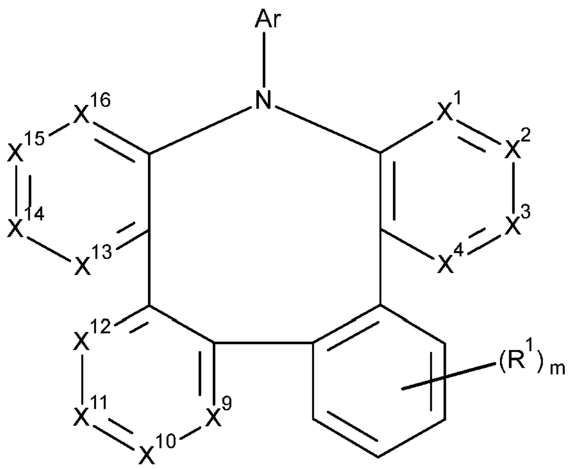

Formel (I-2)

wobei m 0, 1 oder 2 ist und die verwendeten Symbole $X^1$, $X^2$, $X^3$, $X^4$, $X^9$, $X^{10}$, $X^{11}$, $X^{12}$, $X^{13}$, $X^{14}$, $X^{15}$, $X^{16}$ , $R^1$ und Ar die zuvor, insbesondere für Formel (I) genannte Bedeutung aufweisen.

Vorzugsweise können die erfindungsgemäßen Verbindungen Strukturen gemäß der Formel (I-3) umfassen

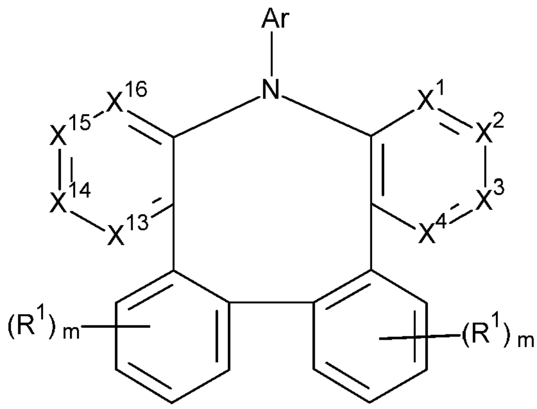

Formel (I-3)

wobei die verwendeten Symbole $X^1$, $X^2$, $X^3$, $X^4$, $X^{13}$, $X^{14}$, $X^{15}$, $X^{16}$, , $R^1$ und Ar die zuvor, insbesondere für Formel (I) dargelegte Bedeutung aufweisen und m jeweils unabhängig für 0, 1 oder 2 steht.

Bevorzugt können die erfindungsgemäßen Verbindungen Strukturen gemäß der Formel (I-4) umfassen

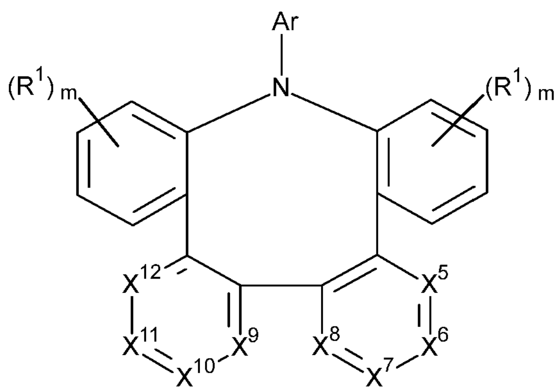

Formel (I-4)

wobei die verwendeten Symbole $X^5$, $X^6$, $X^7$, $X^8$, $X^9$, $X^{10}$, $X^{11}$, $X^{12}$, $R^1$ und Ar die zuvor, insbesondere für Formel (I)

dargelegte Bedeutung aufweisen und m jeweils unabhängig für 0, 1 oder 2 steht.

In einer weiterhin bevorzugten Ausführungsform können die erfindungsgemäßen Verbindungen Strukturen gemäß der Formel (III) aufweisen

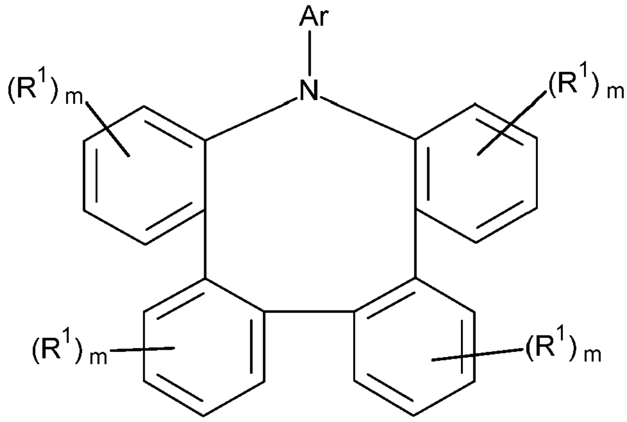

Formel (III)

wobei die verwendeten Symbole $R^1$ und Ar die zuvor, insbesondere für Formel (I) genannte Bedeutung aufweisen und m bei jedem Auftreten jeweils unabhängig für 0, 1 oder 2 steht.

Anspruchsgemäß beträgt die Summe der Indices m in der Struktur der Formel (III) maximal 2 und speziell bevorzugt 0.

In einer bevorzugten Ausführungsform kann eine erfindungsgemäße Verbindung zwei oder mehr Struktureinheiten der Formel (I) beziehungsweise deren bevorzugten Ausfühurngsformen aufweisen. In diesem Fall kann die Struktureinheit anstatt eines Wasserstoffatoms oder eines Substituenten ein oder mehrere Bindungen zu einer weiteren Gruppe mit einer Struktureinheit der Formel (I) aufweisen.

Vorzugsweise kann eine verbindende Gruppe jeweils mit dem in Formel (I) dargestellten Stickstoffatom des Heterocyclus verbunden sein, so dass die verbindenden Gruppe eine Teilstruktur der Gruppe Ar darstellt oder die Gruppe Ar durch die verbindende Gruppe darstellbar ist, wobei an diese verbindende Gruppe ein weiterer Heterocyclus mit mindestens 4 aromatischen oder heteroaromatischen Ringen und einem Stickstoffatom gebunden ist. Die verbindende Gruppe ist vorzugsweise ein aromatisches oder heteroaromatisches Ringsystem mit 5 bis 30 aromatischen oder heteroaromatischen Ringatomen, vorzugsweise ein aromatisches Ringsystem mit 6 bis 12 Kohlenstoffatomen, welches durch einen oder mehrere Reste $R^1$ substituiert sein kann.

Anspruchsgemäß ist vorgesehen, dass die Substitutenten $R^1$ des heteroaromatischen Ringssystems gemäß den zuvorgenannten Formeln mit den Ringatomen des heteroaromatischen Ringssystems kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten $R^2$ ein, die an die Reste $R^1$ gebunden sein können. Anspruchsgemäß ist vorgesehen, dass die Substitutenten $R^1$ des heteroaromatischen Ringssystems gemäß den vorgenannten Formeln mit den Ringatomen des heteroaromatischen Ringssystems kein Ringsystem bilden. Dies schließt die Bildung eines Ringsystems mit möglichen Substituenten $R^2$ ein, die an die Reste $R^1$ gebunden sein können.

Gemäß einer bevorzugten Ausgestaltung sind erfindungsgemäße Verbindungen durch Strukturen der Formel (I), (I-1), (I-2), (I-3), (I-4), (III) darstellbar. Dementsprechend sind Verbindungen gemäß einer Struktur der Formel (I), (I-1), (I-2), (I-3), (I-4), (III) bevorzugt. Vorzugsweise weisen Verbindungen, umfassend Strukturen gemäß Formel (I), (I-1), (I-2), (I-3), (I-4), (III) ein Molekulargewicht von kleiner oder gleich 5000 g/mol, bevorzugt kleiner oder gleich 4000 g/mol, insbesondere bevorzugt kleiner oder gleich 3000 g/mol, speziell bevorzugt kleiner oder gleich 2000 g/mol und ganz besonders bevorzugt kleiner oder gleich 1200 g/mol auf.

Weiterhin zeichnen sich bevorzugte erfindungsgemäße Verbindungen dadurch aus, dass diese sublimierbar sind. Diese Verbindungen weisen im Allgemeinen eine Molmasse von weniger als ca. 1200 g/mol auf.

In einer bevorzugten Ausführungsform kann die Gruppe Ar und/oder der Rest $R^1$ eine Elektronentransportgruppe umfassen, vorzugsweise darstellen. Elektronentransportgruppen sind in der Fachwelt weithin bekannt und fördern die Fähigkeit von Verbindungen Elektronen zu transportieren und/oder zu leiten.

Vorzugsweise steht das Symbol $Ar^1$ für einen Aryl- oder Heteroarylrest, so dass eine aromatische oder heteroaromatische Gruppe eines aromatischen oder heteroaromatischen Ringsystems direkt, d.h. über ein Atom der aromatischen oder heteroaromatischen Gruppe, an das jeweilige Atom der weiteren Gruppe gebunden ist.

In einer weiteren bevorzugten Ausführungsform der Erfindung steht $Ar^1$ gleich oder verschieden bei jedem

Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste $R^2$ substituiert sein kann, bevorzugt aber unsubstituiert ist, wobei $R^2$ die zuvor, insbesondere in Formel (I) dargestellte Bedeutung aufweisen kann. Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^3$ substituiert sein können, bevorzugt aber unsubstituiert sind.

Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist. Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Anspruchsgemäß sind die Substituenten $R^1$ ausgewählt aus der Gruppe bestehend aus H, D, F, CN, $N(Ar^1)_2$, einer geradkettigen Alkylgruppe mit 1 bis 8 C-Atomen, bevorzugt mit 1, 2, 3 oder 4 C-Atomen, oder einer ver-zweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevor-zugt mit 2, 3 oder 4 C-Atomen, die jeweils mit einem oder mehreren Resten $R^2$ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten $R^1$ substituiert sein kann, bevorzugt aber unsubstituiert ist.

Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Anspruchsgemäß ist vorgesehen, dass in der Struktur gemäß Formel (I), (I-1), (I-2), (I-3), (I-4), (III) mindestens ein Rest Ar, für eine Gruppe steht, die ausgewählt ist aus den Formeln ($R^1$-1) bis ($R^1$-95)

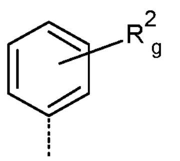

Formel ($R^1$-1)

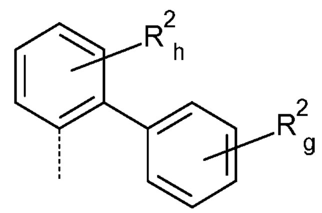

Formel ($R^1$-2)

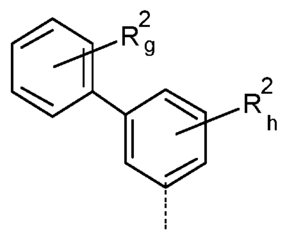

Formel ($R^1$-3)

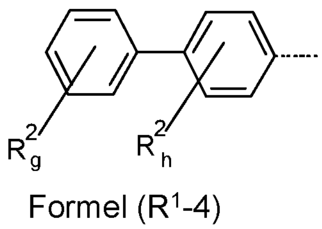

Formel ($R^1$-4)

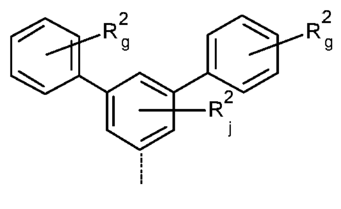

Formel ($R^1$-5)

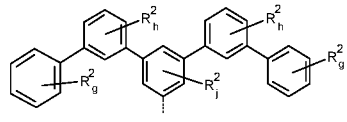

Formel ($R^1$-6)

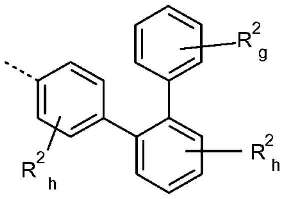

Formel ($R^1$-7)

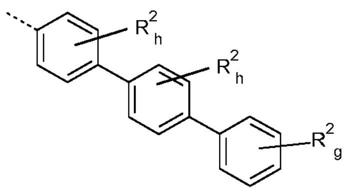

Formel ($R^1$-8)

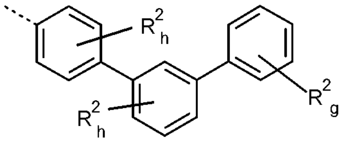

Formel ($R^1$-9)

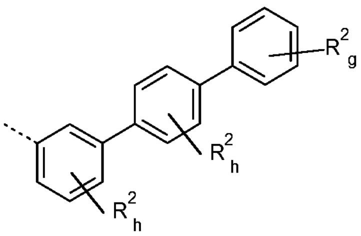

Formel ($R^1$-10)

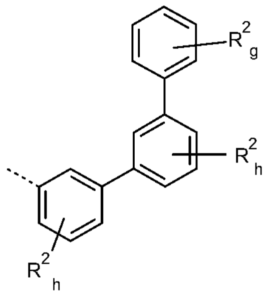

Formel ($R^1$-11)

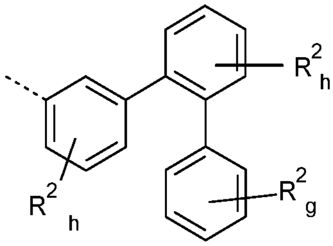

Formel ($R^1$-12)

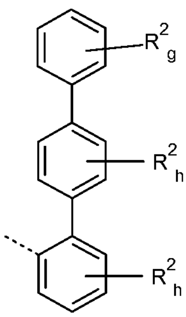

Formel ($R^1$-13)

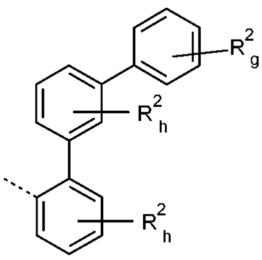

Formel ($R^1$-14)

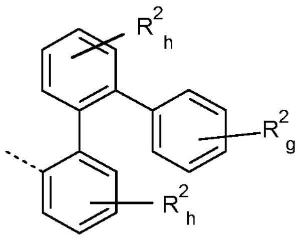

Formel ($R^1$-15)

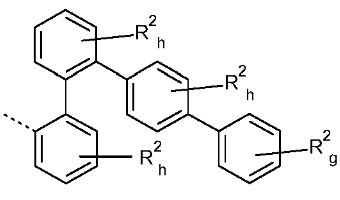

Formel ($R^1$-16)

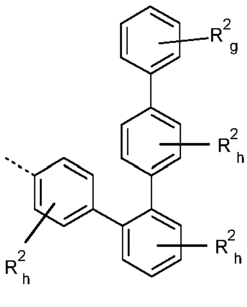

Formel ($R^1$-17)

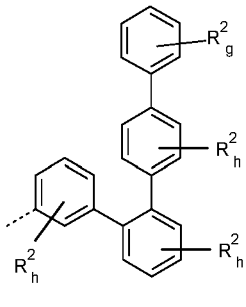

Formel ($R^1$-18)

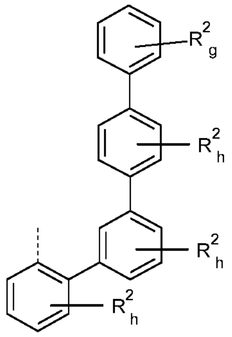

Formel ($R^1$-19)

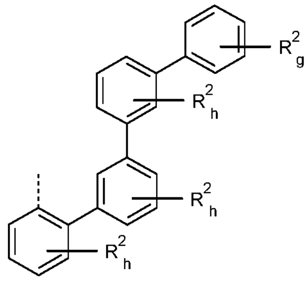

Formel ($R^1$-20)

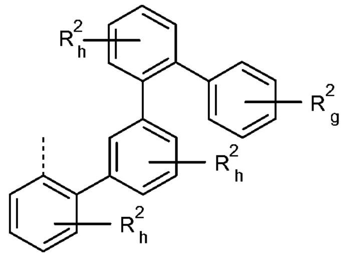

Formel ($R^1$-21)

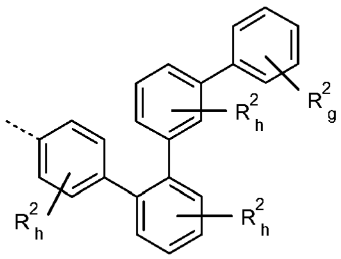

Formel ($R^1$-22)

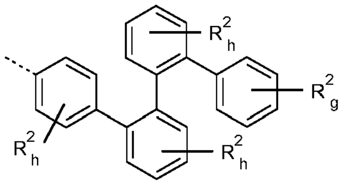

Formel ($R^1$-23)

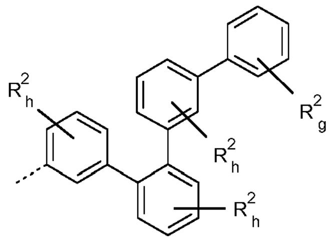

Formel ($R^1$-24)

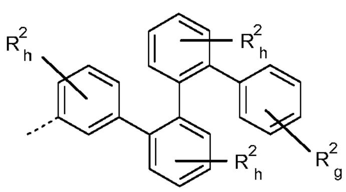

Formel ($R^1$-25)

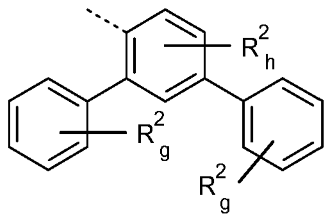

Formel ($R^1$-26)

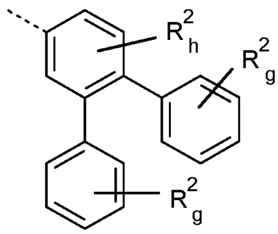

Formel ($R^1$-27)

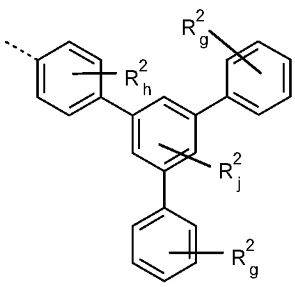

Formel ($R^1$-28)

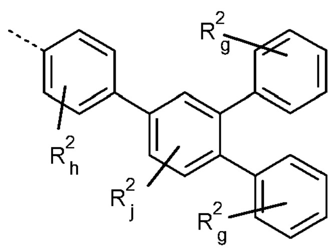

Formel ($R^1$-29)

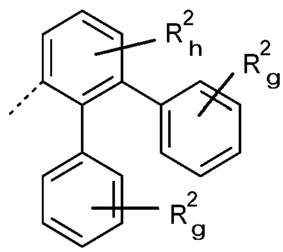

Formel ($R^1$-30)

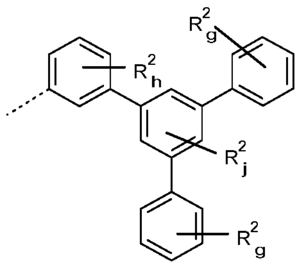

Formel ($R^1$-31)

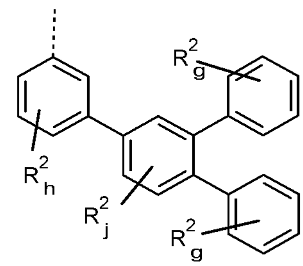

Formel ($R^1$-32)

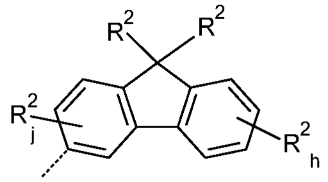

Formel ($R^1$-33)

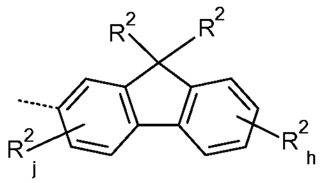

Formel ($R^1$-34)

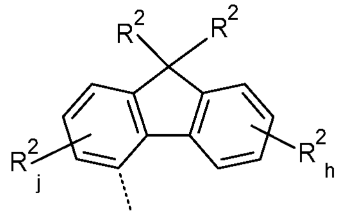

Formel ($R^1$-35)

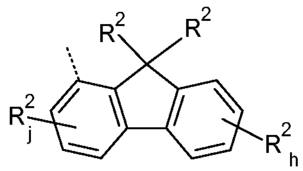

Formel ($R^1$-36)

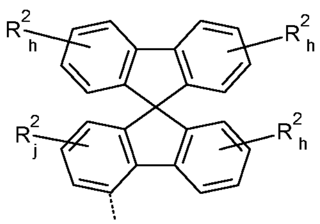

Formel ($R^1$-37)

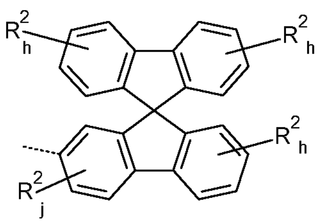

Formel ($R^1$-38)

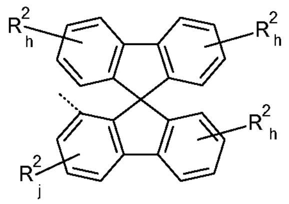

Formel ($R^1$-39)

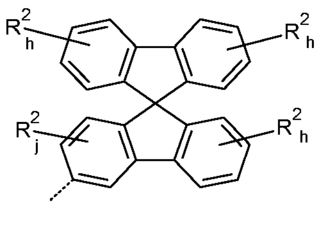

Formel ($R^1$-40)

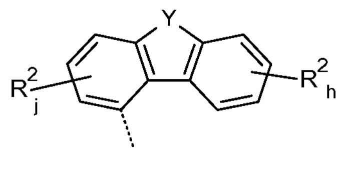

Formel ($R^1$-41)

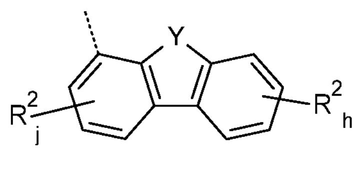

Formel ($R^1$-42)

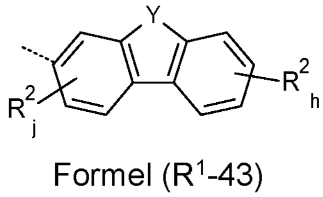

Formel ($R^1$-43)

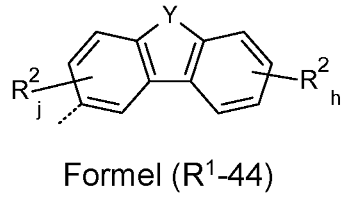

Formel ($R^1$-44)

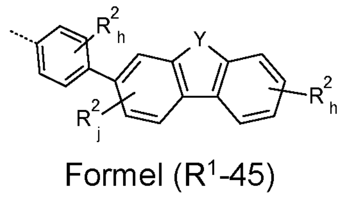

Formel ($R^1$-45)

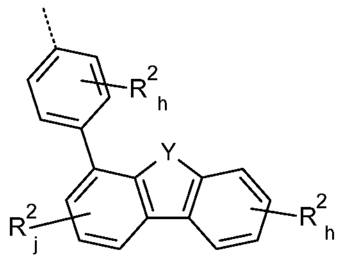

Formel ($R^1$-46)

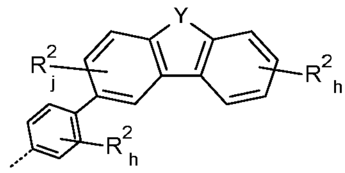

Formel ($R^1$-47)

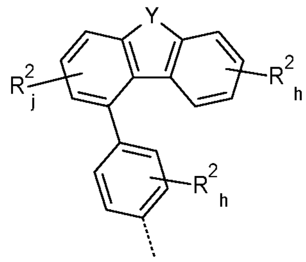

Formel ($R^1$-48)

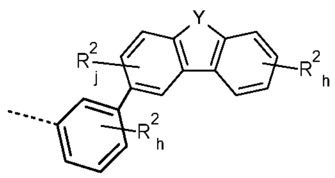

Formel ($R^1$-49)

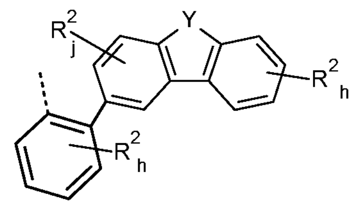

Formel ($R^1$-50)

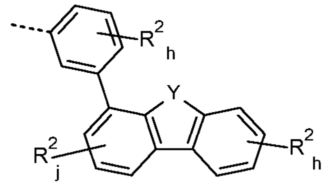

Formel ($R^1$-51)

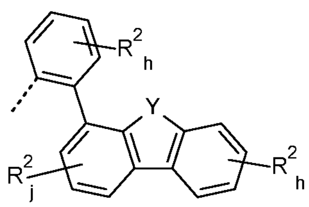

Formel ($R^1$-52)

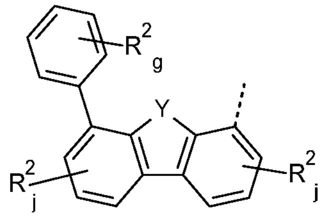

Formel ($R^1$-53)

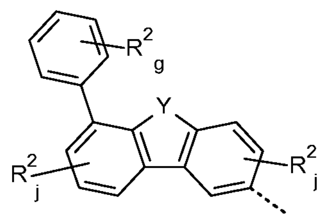

Formel ($R^1$-54)

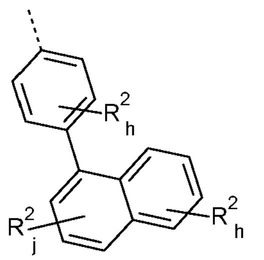

Formel ($R^1$-55)

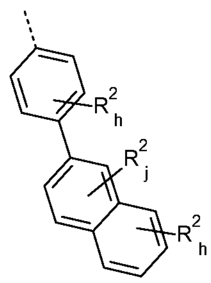

Formel ($R^1$-56)

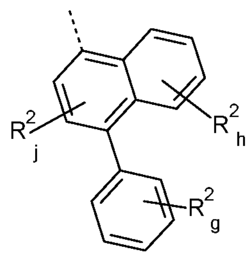

Formel ($R^1$-57)

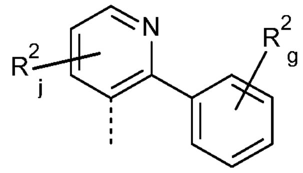

Formel ($R^1$-58)

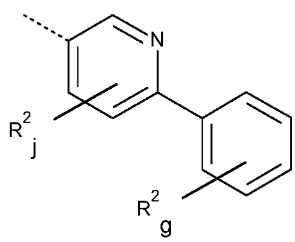

Formel ($R^1$-59)

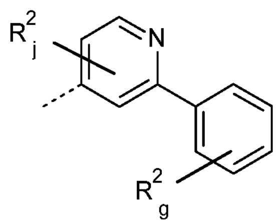

Formel ($R^1$-60)

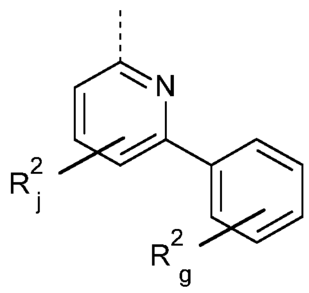

Formel ($R^1$-61)

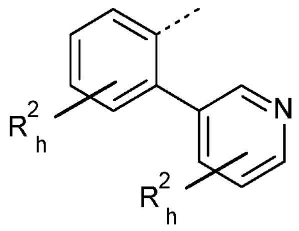

Formel ($R^1$-62)

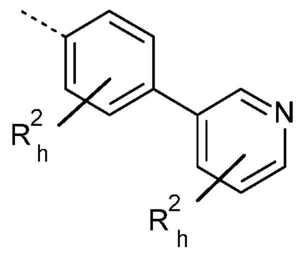

Formel ($R^1$-63)

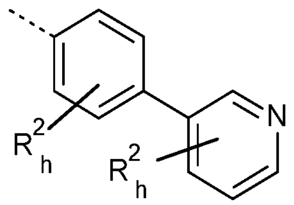

Formel ($R^1$-64)

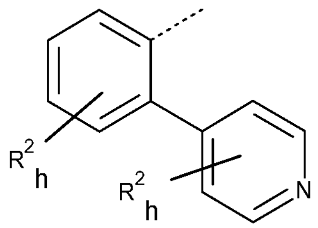

Formel ($R^1$-65)

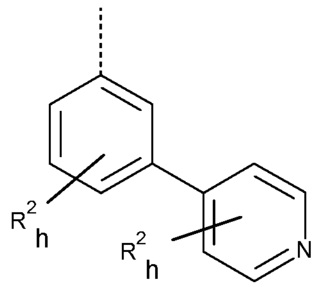

Formel ($R^1$-66)

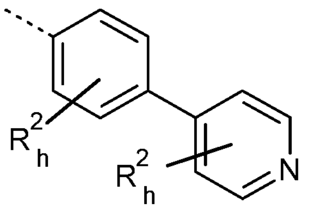

Formel ($R^1$-67)

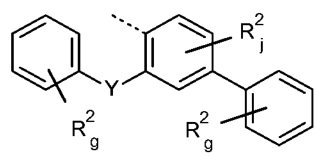

Formel ($R^1$-68)

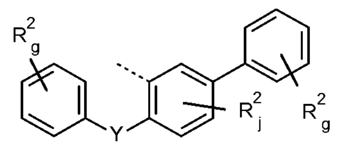

Formel ($R^1$-69)

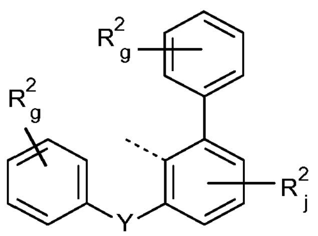

Formel ($R^1$-70)

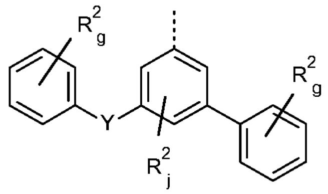

Formel ($R^1$-71)

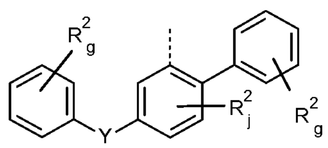

Formel ($R^1$-72)

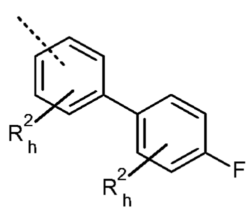

Formel ($R^1$-73)

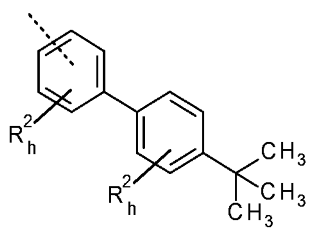

Formel ($R^1$-74)

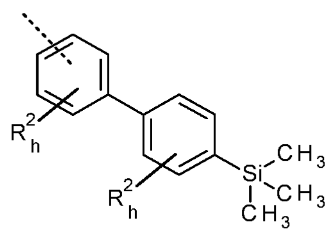

Formel ($R^1$-75)

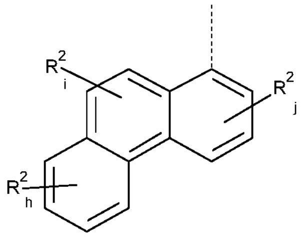
Formel ($R^1$-76)

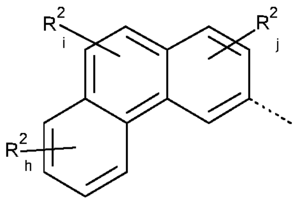
Formel ($R^1$-77)

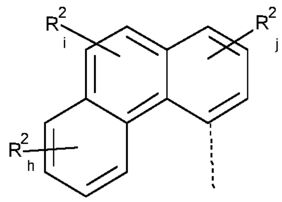
Formel ($R^1$-78)

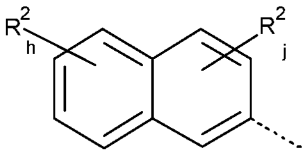
Formel ($R^1$-79)

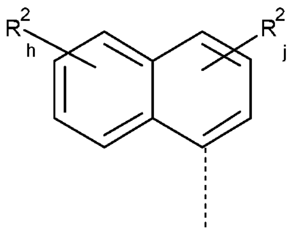
Formel ($R^1$-80)

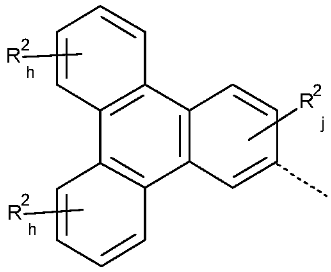
Formel ($R^1$-81)

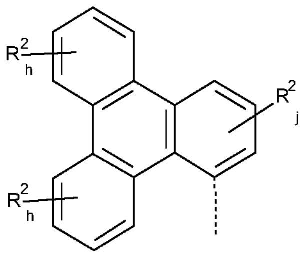
Formel ($R^1$-82)

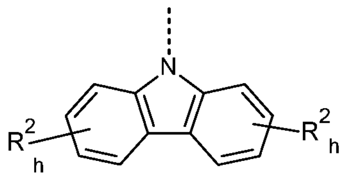
Formel ($R^1$-83)

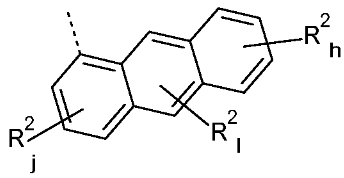
Formel ($R^1$-84)

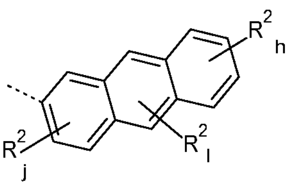
Formel ($R^1$-85)

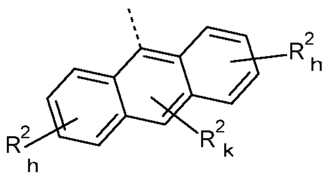
Formel ($R^1$-86)

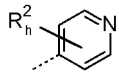
Formel ($R^1$-87)

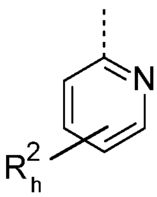
Formel ($R^1$-88)

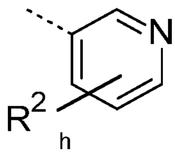
Formel ($R^1$-89)

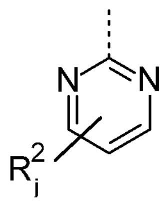
Formel ($R^1$-90)

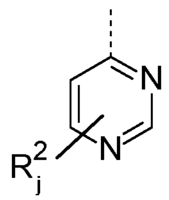

Formel ($R^1$-91)

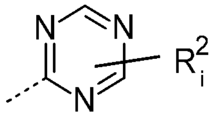

Formel ($R^1$-92)

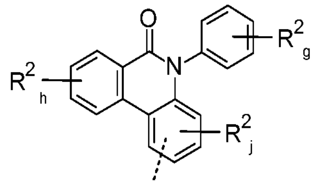

Formel ($R^1$-93)

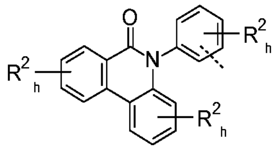

Formel ($R^1$-94)

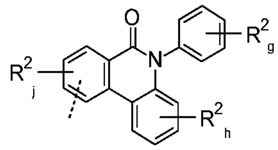

Formel ($R^1$-95)

wobei für die verwendeten Symbole gilt:

- Y ist O, S oder $NR^2$, vorzugsweise O oder S;
- i ist bei jedem Auftreten unabhängig 0, 1 oder 2;
- j ist bei jedem Auftreten unabhängig 0, 1, 2 oder 3;
- h ist bei jedem Auftreten unabhängig 0, 1, 2, 3 oder 4;
- g ist bei jedem Auftreten unabhängig 0, 1, 2, 3, 4 oder 5;
- $R^2$ kann die zuvor genannte, insbesondere für Formel (I) genannte Bedeutung aufweisen und

die gestrichelte Bindung markiert die Anbindungsposition.

Vorzugsweise kann vorgesehen sein, dass die Summe der Indices i, j, h und g in den Strukturen der Formel ($R^1$-1) bis ($R^1$-95) jeweils höchstens 3, vorzugsweise höchstens 2 und besonders bevorzugt höchstens 1 beträgt.

Anspruchsgemäß bilden die Reste $R^2$ in den Formeln ($R^1$-1) bis ($R^1$-95) mit den Ringatomen der Arylgruppe oder Heteroarylgruppe, an die die Reste $R^2$ gebunden sind, kein kondensiertes Ringsystem.

Anspruchsgemäß ist vorgesehen, dass in der Struktur der Formel (I) höchstens 2 der Symbole $X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ ungleich CH oder CD sind, und die Summe der Indices m in der Struktur der Formel (III) maximal 2 beträgt.

Anspruchsgemäß ist vorgesehen, dass in der Struktur der Formel (I) höchstens 2 der Symbole $X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ ungleich CH oder CD sind, vorzugsweise die Summe der Indices m in der Struktur der Formel (III) maximal 2 beträgt, wobei der Rest Ar ausgewählt ist aus den zuvor dargelegten Gruppen der Formeln ($R^1$-1) bis ($R^1$-95), vorzugsweise ($R^1$-1) bis ($R^1$-54), besonders bevorzugt ($R^1$-3) und/oder ($R^1$-46).

Wenn die erfindungsgemäße Verbindung mit aromatischen oder heteroaromatischen Gruppen $R^1$ bzw. $R^2$ substituiert ist, so ist es, insbesondere bei deren Ausgestaltung als Hostmaterial, Elektronentransportmaterial oder Lochtransportmaterial, bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Bei Ausgestaltung der erfindungsgemäßen Verbindungen zur Verwendung als fluoreszierender Emitter können bevorzugte Verbindungen entsprechende Gruppen, beispielsweise Fluoren-, Anthracen- und/oder Pyren-Gruppen enthalten, die mit Gruppen $R^2$ substituiert sein können oder die durch entsprechende Substitution der Gruppen ($R^1$-1) bis ($R^1$-95), vorzugsweise ($R^1$-33) bis ($R^1$-57) und ($R^1$-76) bis ($R^1$-86), oder ($R^1$-71) bis ($R^1$-91), mit den Substituenten $R^2$ gebildet werden.

Anspruchsgemäß ist $R^2$, beispielsweise bei einer Struktur gemäß Formel (I) sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Beispiele für geeignete erfindungsgemäße Verbindungen sind die nachstehend gezeigten Strukturen gemäß den folgenden Formeln 1 bis 180, wobei nicht anspruchsgemäße Verbindungen mit # gekennzeichnet sind:

| | | |
|---|---|---|
| 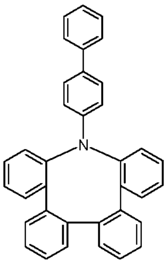 | 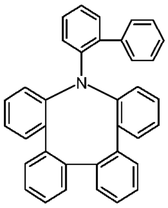 | 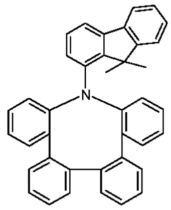 |
| Formel 1 | Formel 2 | Formel 3 |
| 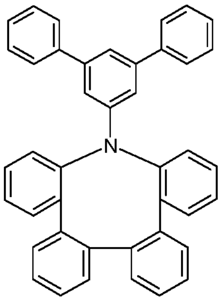 | 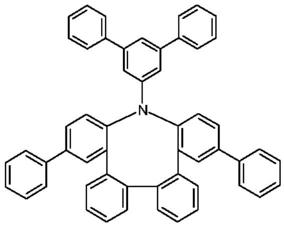 | 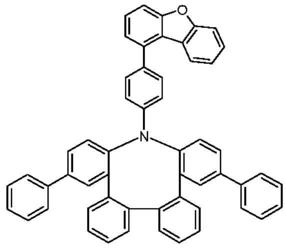 |
| Formel 4 | Formel 5 | Formel 6 |
| 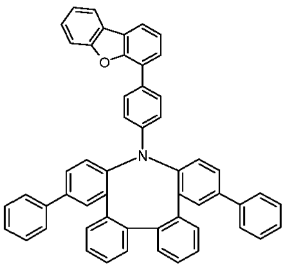 | 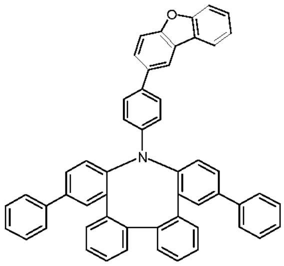 | 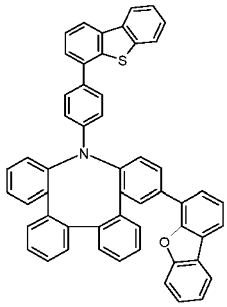 |
| Formel 7 | Formel 8 | Formel 9 |
| 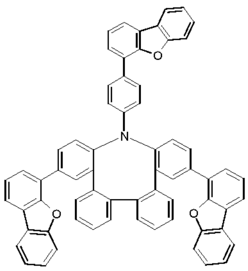 | 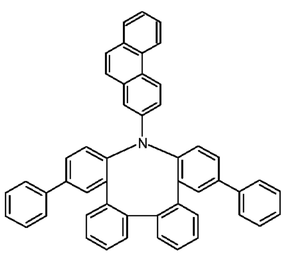 | 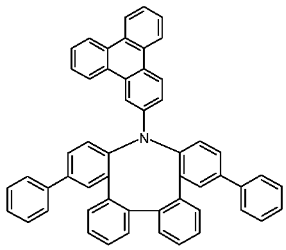 |
| Formel 10 | Formel 11 | Formel 12 |

(fortgesetzt)

| | | |
|---|---|---|
| 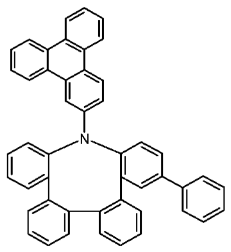 | 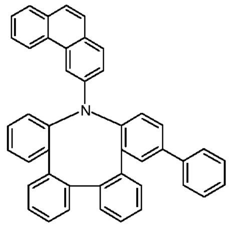 | 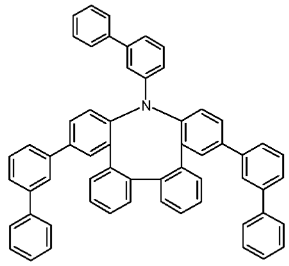 |
| Formel 13 | Formel 14 | Formel 15 |
| 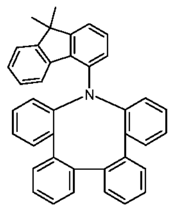 | 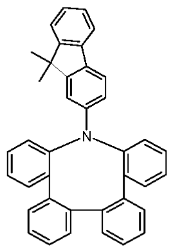 | 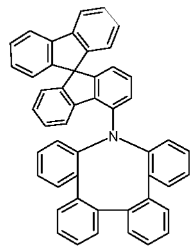 |
| Formel 16 | Formel 17 | Formel 18 |
| | | |
| 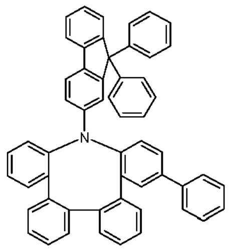 | 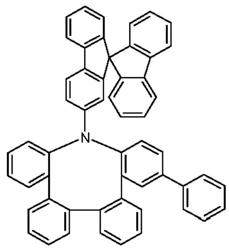 | 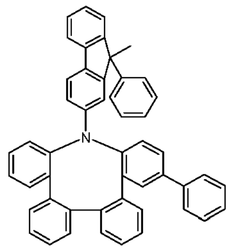 |
| Formel 19 | Formel 20 | Formel 21 |
| 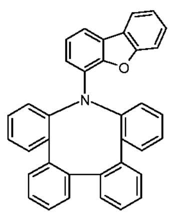 | 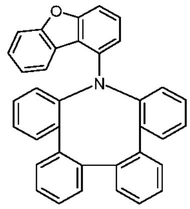 | 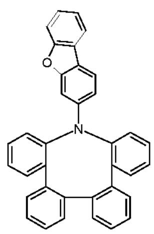 |
| Formel 22 | Formel 23 | Formel 24 |
| 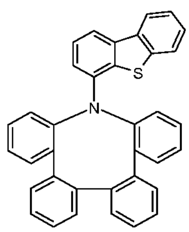 | 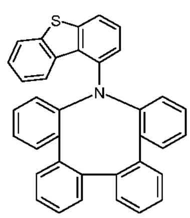 | 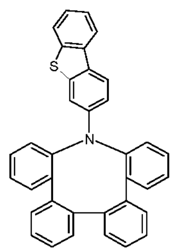 |
| Formel 25 | Formel 26 | Formel 27 |

(fortgesetzt)

| | | |
|---|---|---|
| 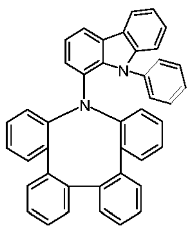 | 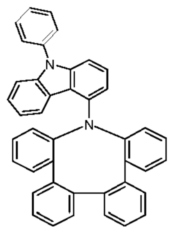 | 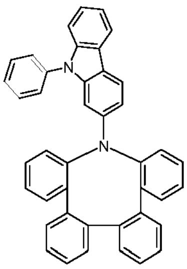 |
| Formel 28 | Formel 29 | Formel 30 |
| 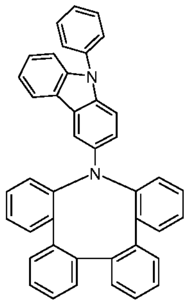 | 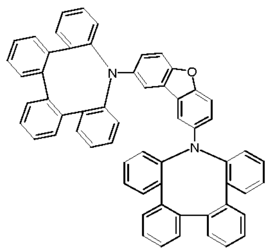 | 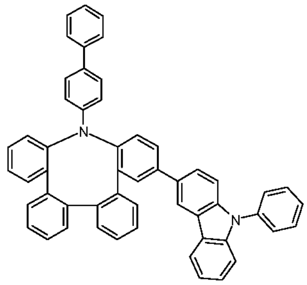 |
| Formel 31 | Formel 32 | Formel 33 |
| 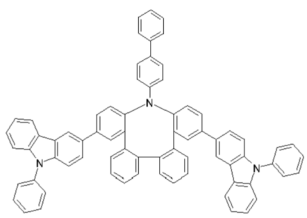 | 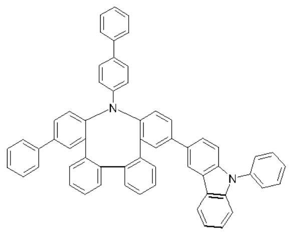 | 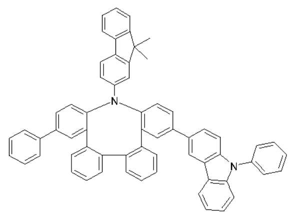 |
| Formel 34 | Formel 35 | Formel 36 |
| 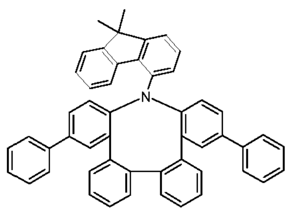 | 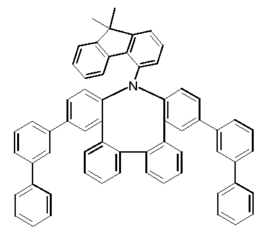 | 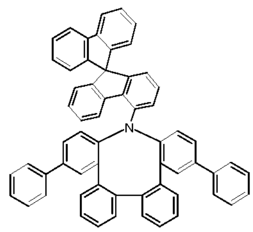 |
| Formel 37 | Formel 38 | Formel 39 |
| 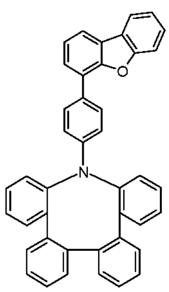 | 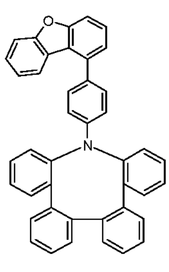 | 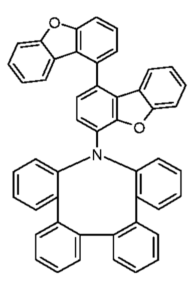 |
| Formel 40 | Formel 41 | Formel 42 |

(fortgesetzt)

| | | |
|---|---|---|
| 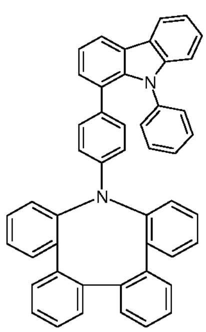 | 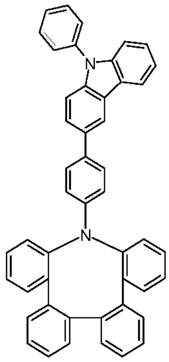 | 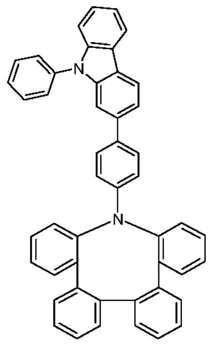 |
| Formel 43 | Formel 44 | Formel 45 |
| 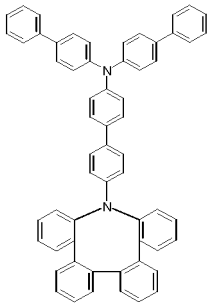 | 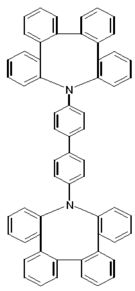 | 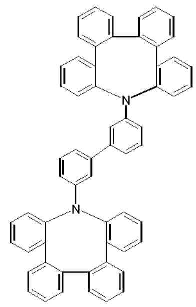 |
| Formel 46 | Formel 47 | Formel 48 |
| 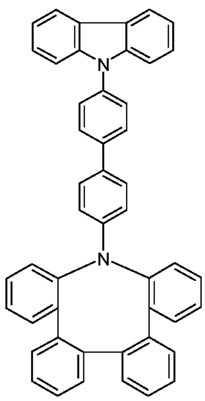 | 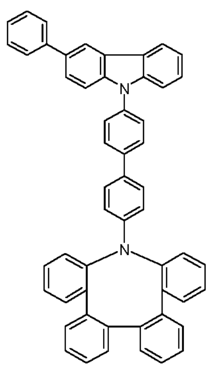 | 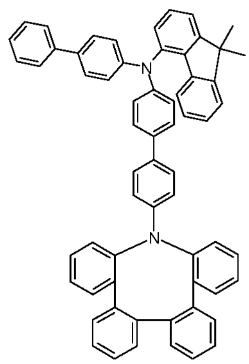 |
| Formel 49 | Formel 50 | Formel 51 |
| 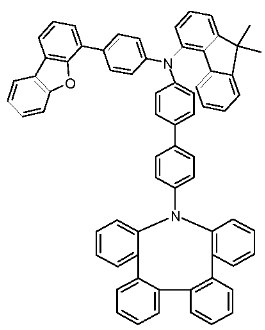 | 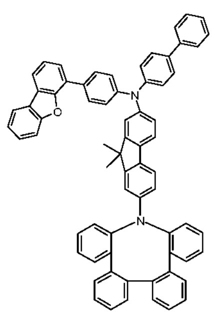 | 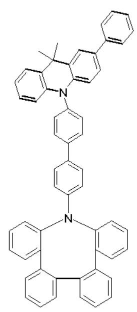 |
| Formel 52 | Formel 53 | Formel 54 |

(fortgesetzt)

| | | |
|---|---|---|
| 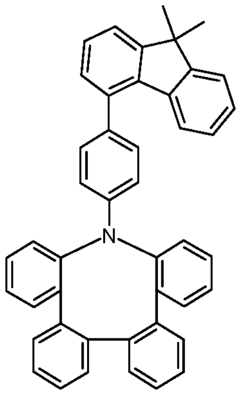 | 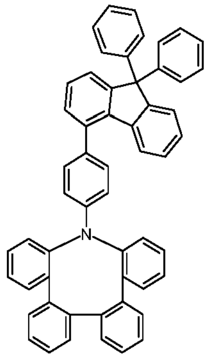 | 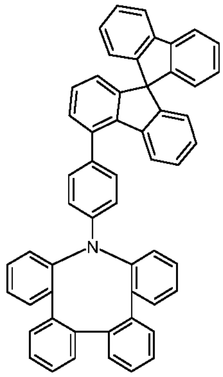 |
| Formel 55 | Formel 56 | Formel 57 |
| 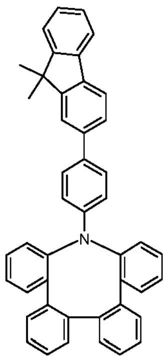 | 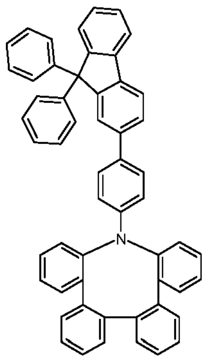 | 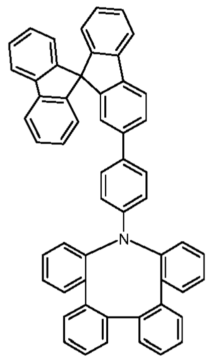 |
| Formel 58 | Formel 59 | Formel 60 |
| 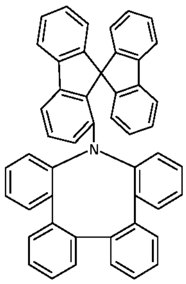 | 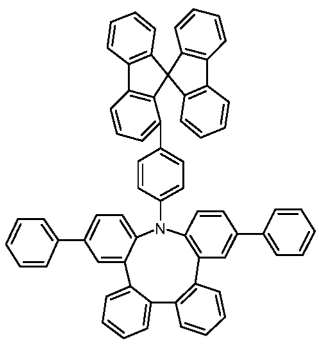 | 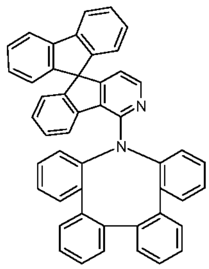 |
| Formel 61 | Formel 62 | Formel 63 |
| 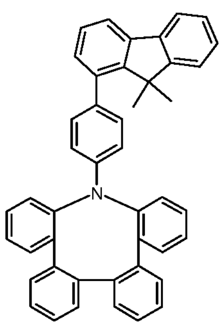 | 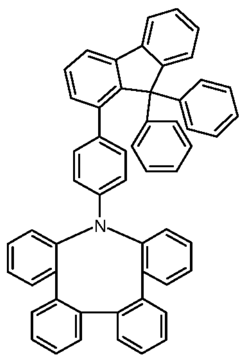 | 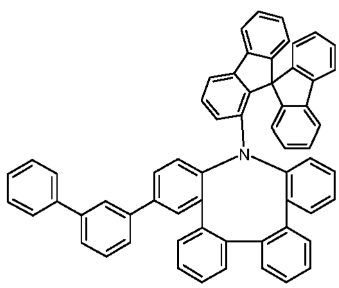 |
| Formel 64 | Formel 65 | Formel 66 |

(fortgesetzt)

| | | |
|---|---|---|
| 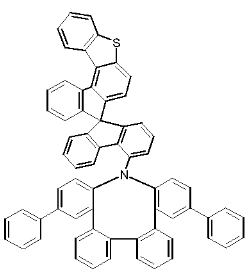 | 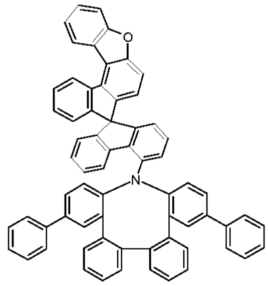 | 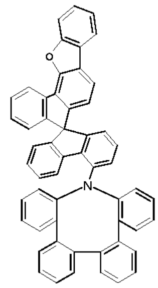 |
| Formel 67 | Formel 68 | Formel 69 |
| 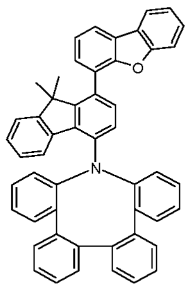 | 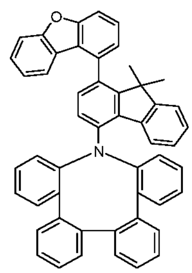 | 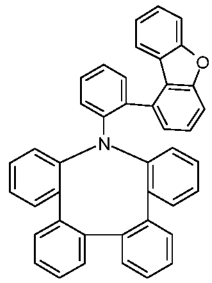 |
| Formel 70 | Formel 71 | Formel 72 |
| 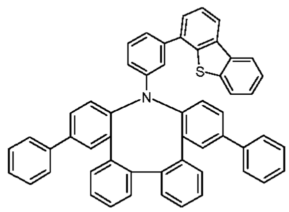 | 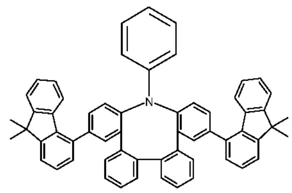 | 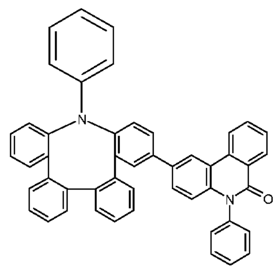 |
| Formel 73 | Formel 74 | Formel 75 |
| 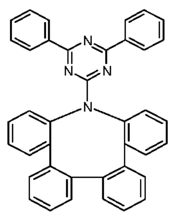 | 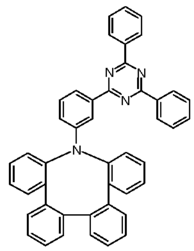 | 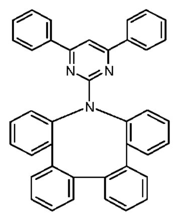 |
| Formel 76 | Formel 77 | Formel 78 |
| 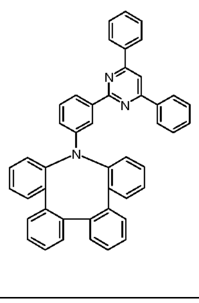 | 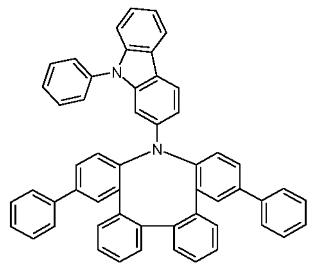 | 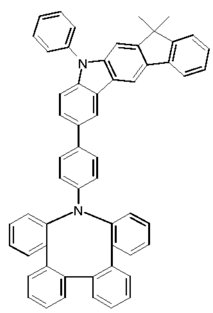 |
| Formel 79 | Formel 80 | Formel 81 |

(fortgesetzt)

| | | |
|---|---|---|
| 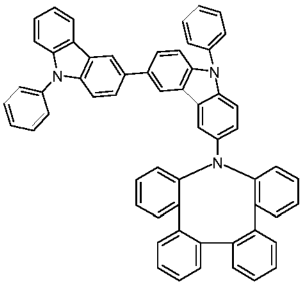 | 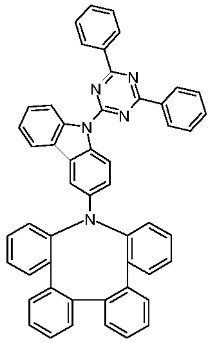 | 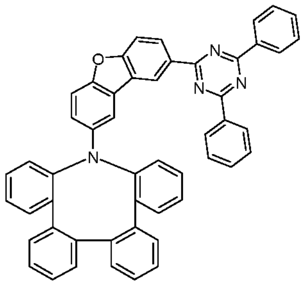 |
| Formel 82 | Formel 83 | Formel 84 |
| 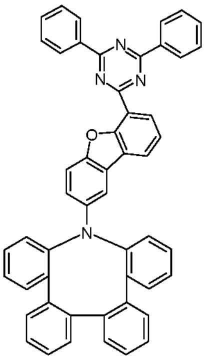 | 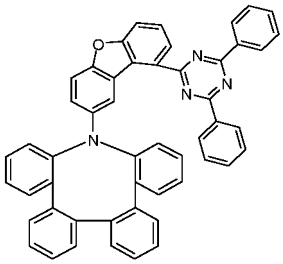 | 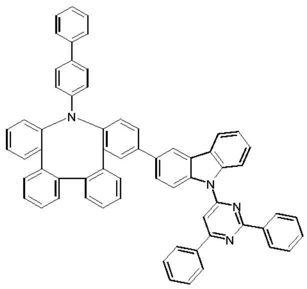 |
| Formel 85 | Formel 86 | Formel 87 |
| 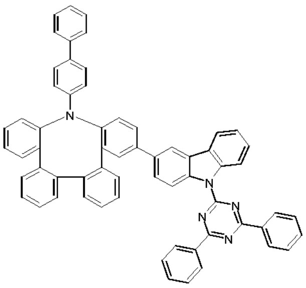 | 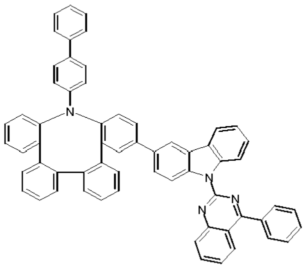 | 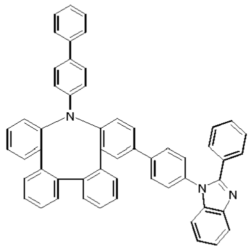 |
| Formel 88 | Formel 89 | Formel 90 |
| 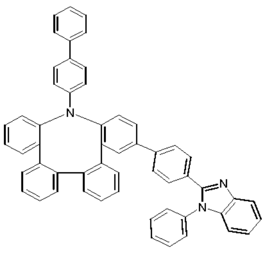 | 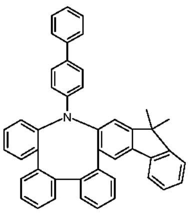 | 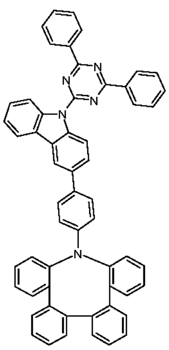 |
| Formel 91 | Formel 92 | Formel 93 |

(fortgesetzt)

| | | |
|---|---|---|
| 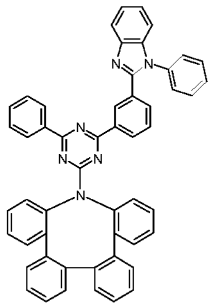 | 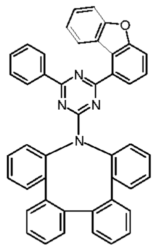 | 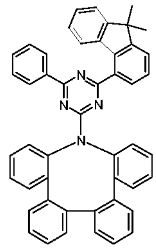 |
| Formel 94 | Formel 95 | Formel 96 |
| 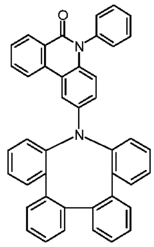 | 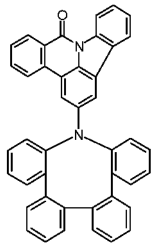 | 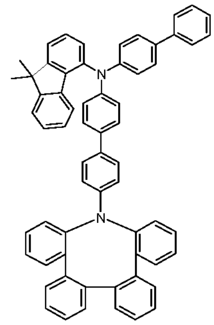 |
| Formel 97 | Formel 98 | Formel 99 |
| 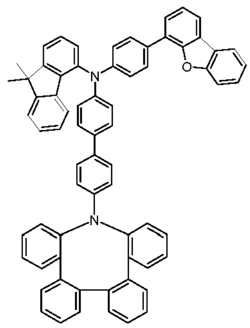 | 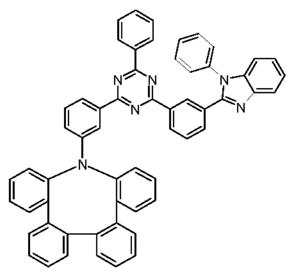 | 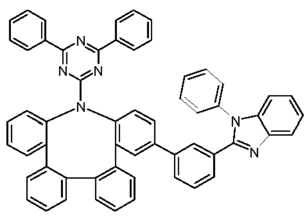 |
| Formel 100 | Formel 101 | Formel 102 |
| 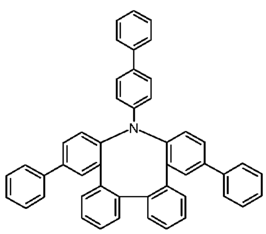 | 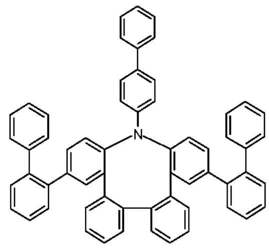 | 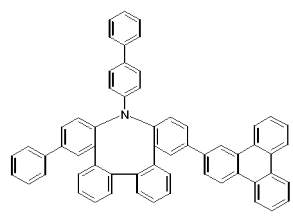 |
| Formel 103 | Formel 104 | Formel 105 |
| 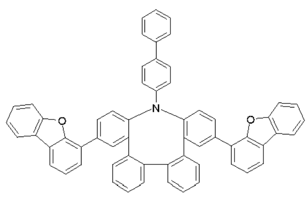 | 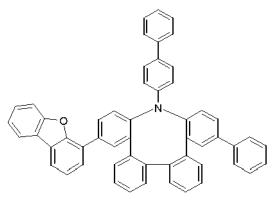 | 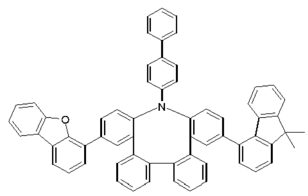 |
| Formel 106 | Formel 107 | Formel 108 |

(fortgesetzt)

| | | |
|---|---|---|
| 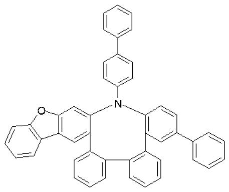 | 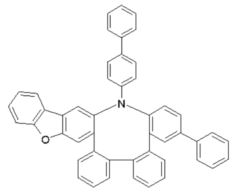 | 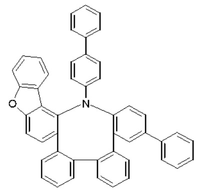 |
| Formel 109 | Formel 110 | Formel 111 |
| 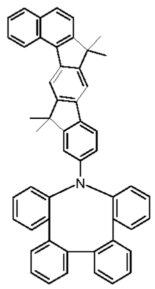 | 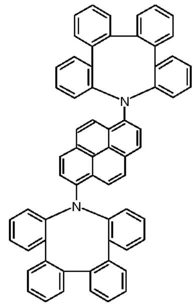 | 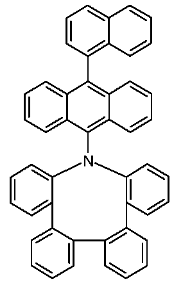 |
| Formel 112 | Formel 113 | Formel 114 |
| 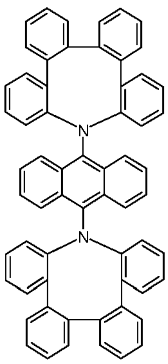 | 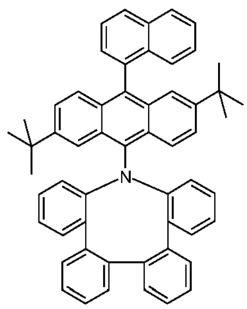 | 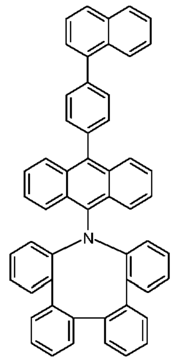 |
| Formel 115 | Formel 116 | Formel 117 |
| 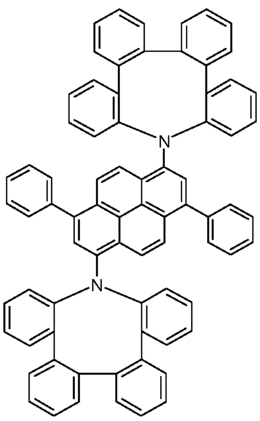 | 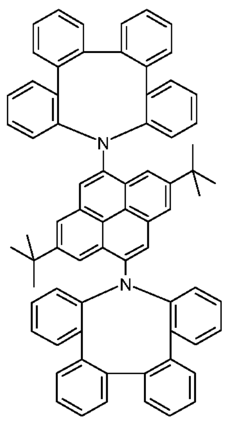 | 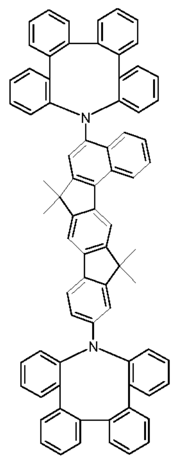 |
| Formel 118 | Formel 119 | Formel 120 |

(fortgesetzt)

| | | |
|---|---|---|
| 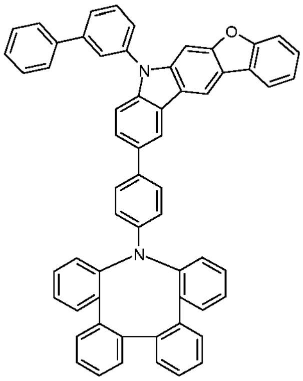 | 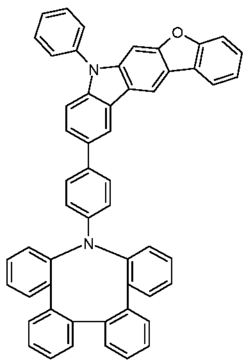 | 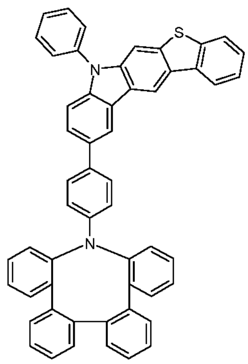 |
| Formel 121 | Formel 122 | Formel 123 |
| 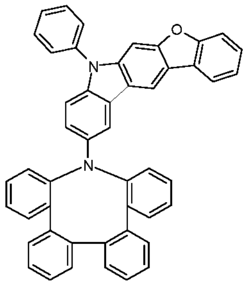 | 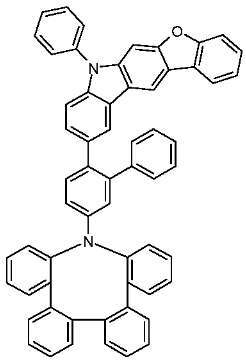 | 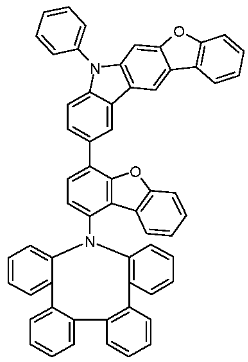 |
| Formel 124 | Formel 125 | Formel 126 |
| 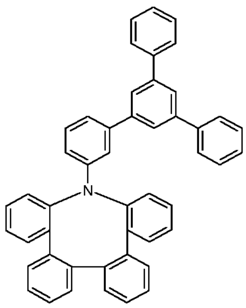 | 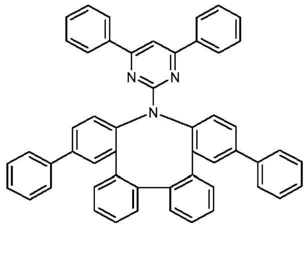 | 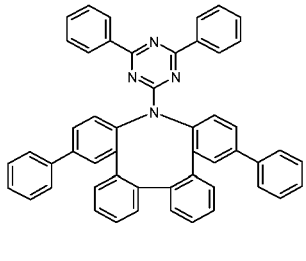 |
| Formel 127 | Formel 128 | Formel 129 |
| 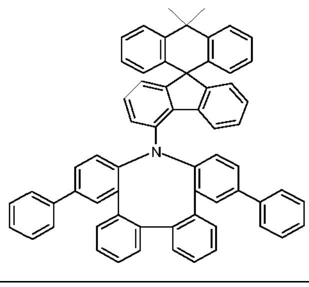 | 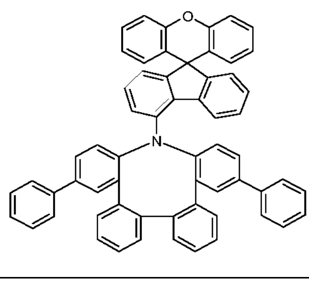 | 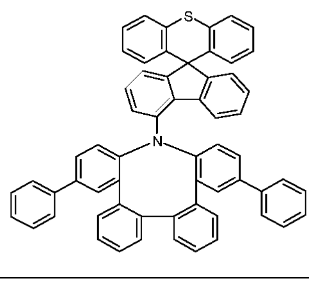 |
| Formel 130 | Formel 131 | Formel 132 |

(fortgesetzt)

| | | |
|---|---|---|
| 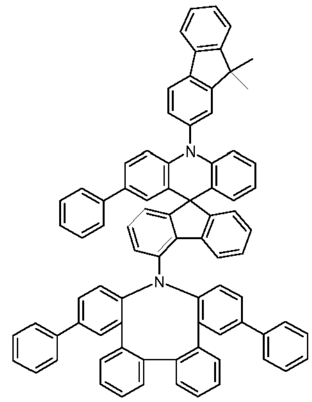 | 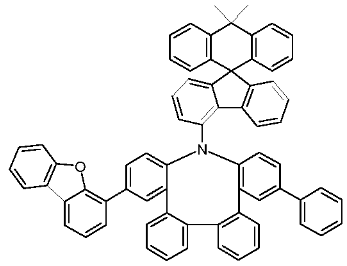 | 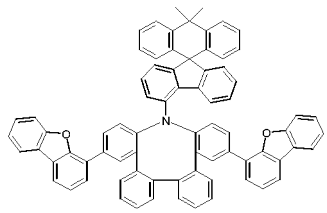 |
| Formel 133 | Formel 134 | Formel 135 |
| 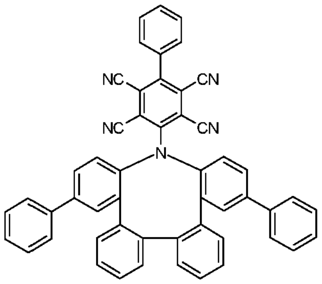 | 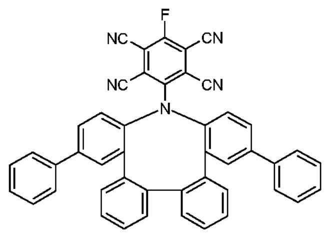 | 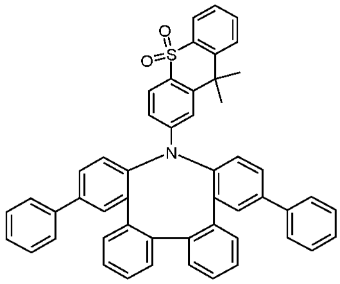 |
| Formel 136 | Formel 137 | Formel 138 |
| 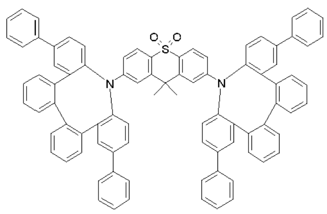 | 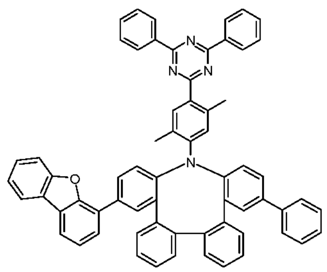 | 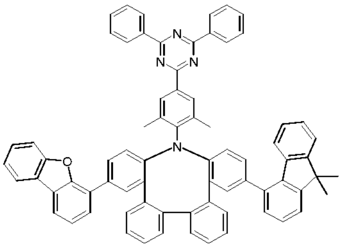 |
| Formel 139 | Formel 140 | Formel 141 |
| 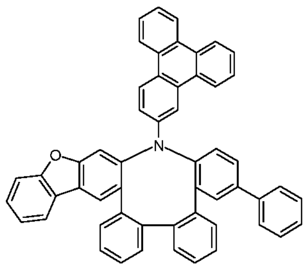 | 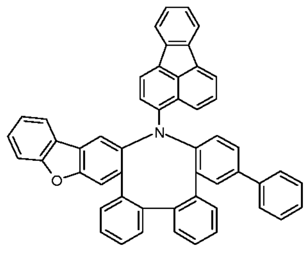 | 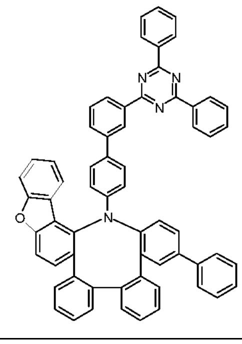 |
| Formel 142 | Formel 143 | Formel 144 |

(fortgesetzt)

| | | |
|---|---|---|
| 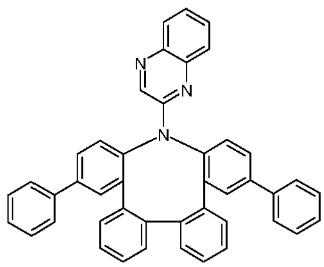 | 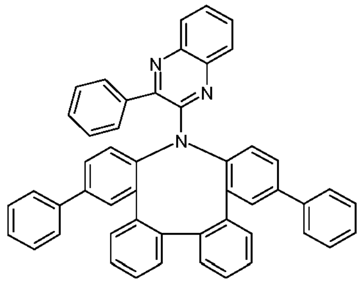 | 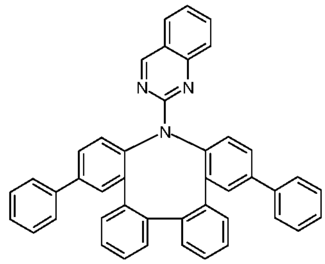 |
| Formel 145 | Formel 146 | Formel 147 |
| 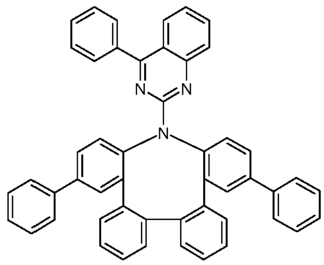 | 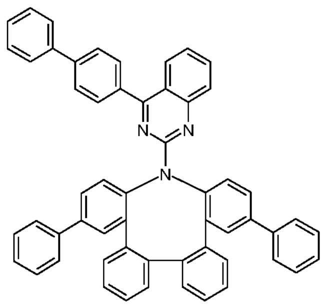 | 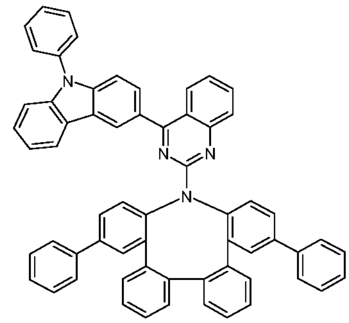 |
| Formel 148 | Formel 149 | Formel 150 |
| 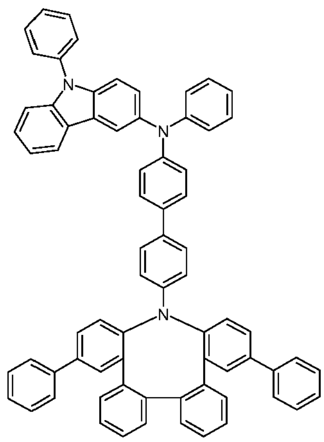 | 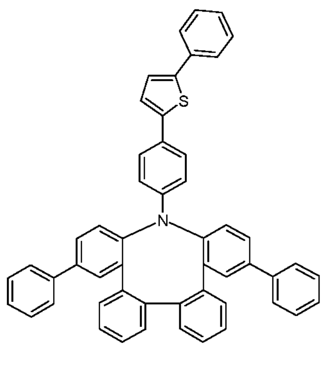 | 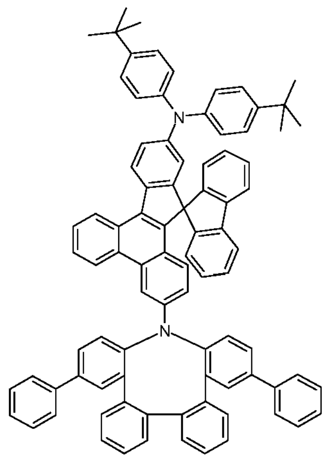 |
| Formel 151 | Formel 152 | Formel 153 |
| 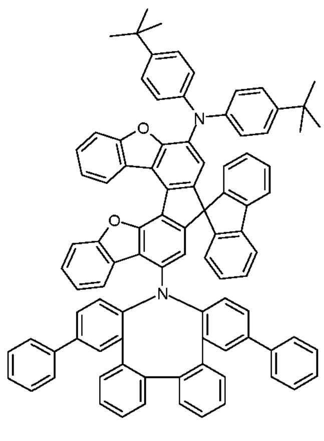 | 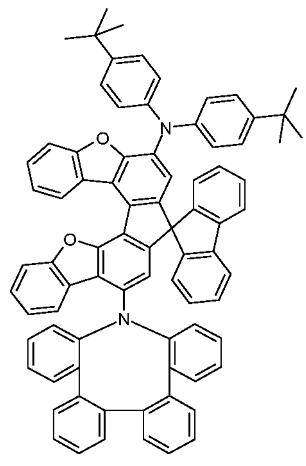 | 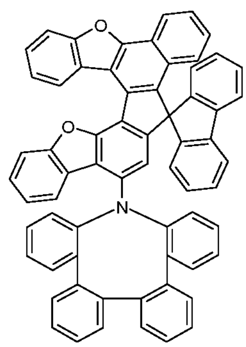 |

(fortgesetzt)

| Formel 154 | Formel 155 | Formel 156 |
|---|---|---|
| 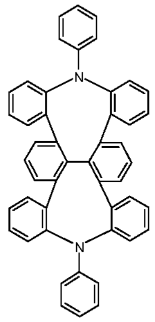 | 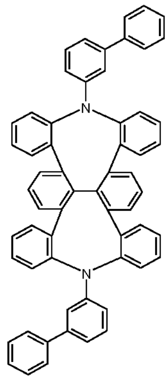 | 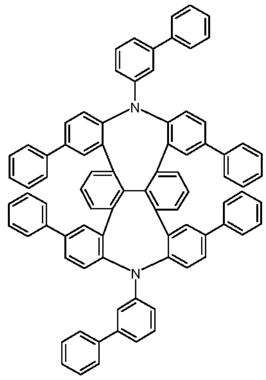 |
| #Formel 157 | #Formel 158 | #Formel 159 |
| 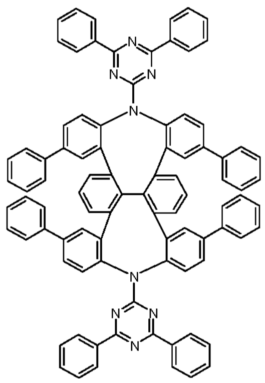 | 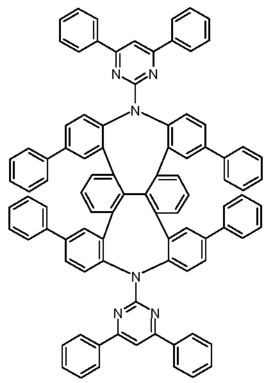 | 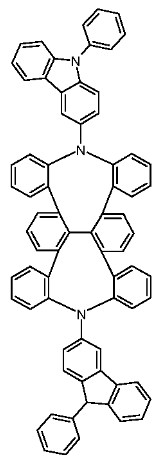 |
| #Formel 160 | #Formel 161 | #Formel 162 |
| 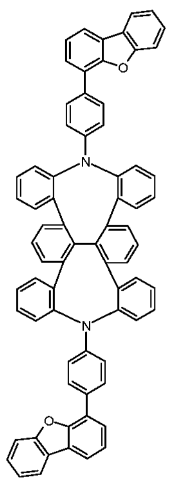 | 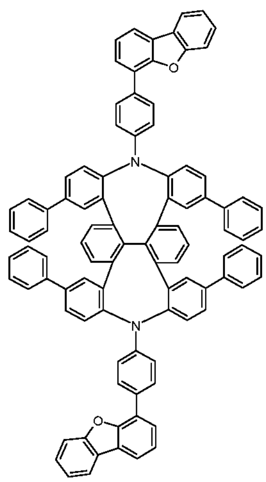 | 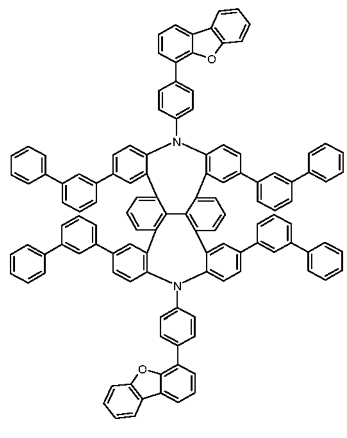 . |
| #Formel 163 | #Formel 164 | #Formel 165 |

(fortgesetzt)

| | | |
|---|---|---|
| 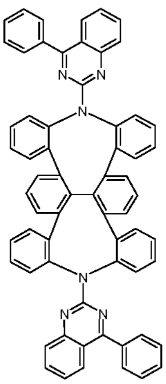 | 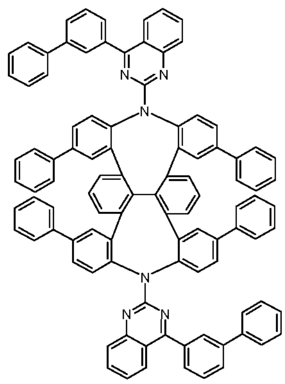 | 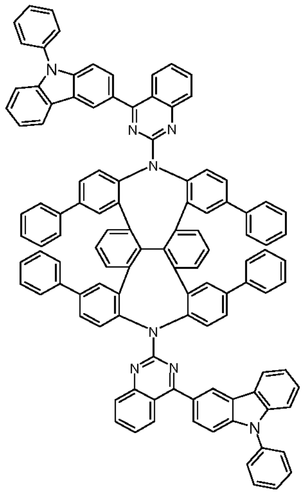 |
| #Formel 166 | #Formel 167 | #Formel 168 |
| 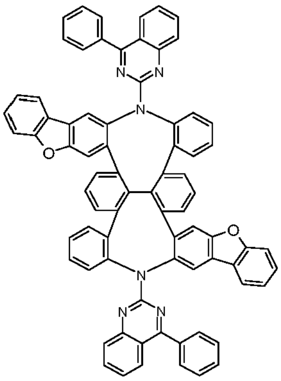 | 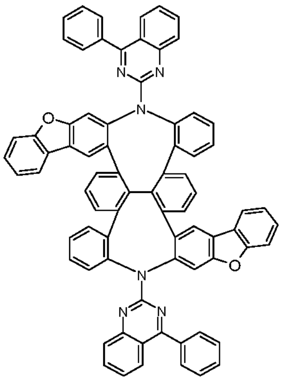 | 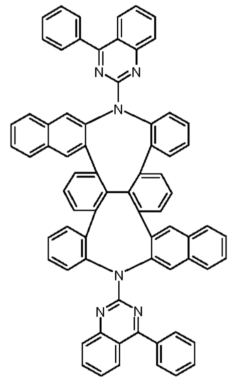 |
| #Formel 169 | #Formel 170 | #Formel 171 |
| 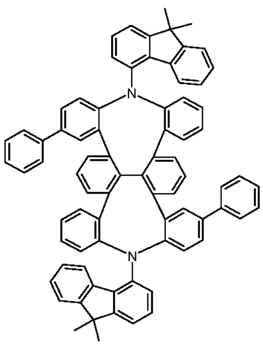 | 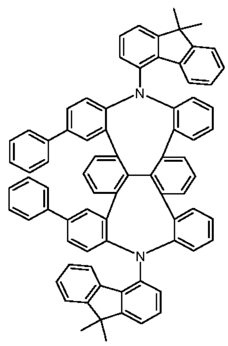 | 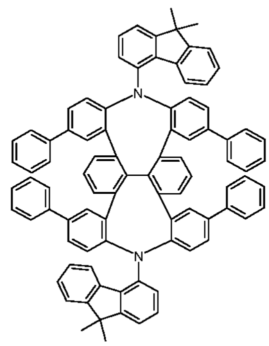 |
| #Formel 172 | #Formel 173 | #Formel 174 |

(fortgesetzt)

| | | |
|---|---|---|
| 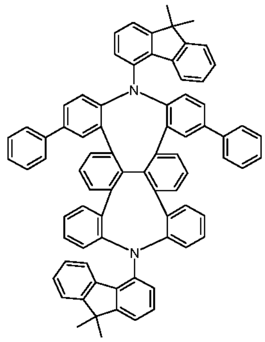 | 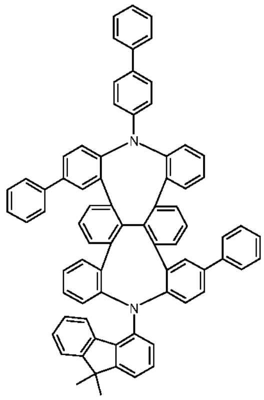 | 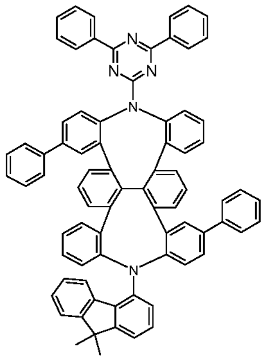 |
| #Formel 175 | #Formel 176 | #Formel 177 |
| 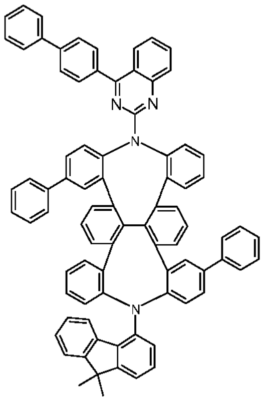 | 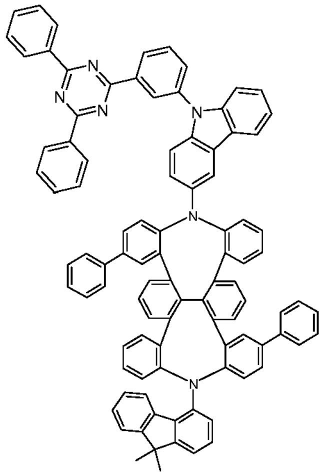 | 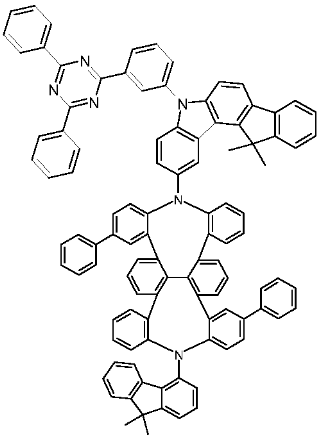 |
| #Formel 178 | #Formel 179 | #Formel 180 |

Bevorzugte Ausführungsformen von erfindungsgemäßen Verbindungen werden in den Beispielen näher ausgeführt, wobei diese Verbindungen allein oder in Kombination mit weiteren für alle erfindungsgemäßen Verwendungszwecke eingesetzt werden können.

Unter der Voraussetzung, dass die in Anspruch 1 genannten Bedingungen eingehalten werden, sind die oben genannten bevorzugten Ausführungsformen beliebig miteinander kombinierbar. In einer besonders bevorzugten Ausführungsform der Erfindung gelten die oben genannten bevorzugten Ausführungsformen gleichzeitig.

Die erfindungsgemäßen Verbindungen sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der Verbindungen, umfassend Strukturen gemäß Formel (I), bei dem in einer Kupplungsreaktion eine Verbindung, umfassend mindestens eine Diarylamin-Gruppe, mit einer Gruppe, umfassend mindestens einen Aryl- oder Heteroarylrest, verbunden wird.

Geeignete Verbindungen mit einer Diarylamin-Gruppe können vielfach kommerziell erhalten werden, wobei die in den Beispielen dargelegten Ausgangsverbindungen durch bekannte Verfahren erhältlich sind, so dass hierauf verwiesen wird.

Beispielsweise können Verbindungen mit einer Diarylamin-Gruppe, die zur Herstellung einer erfindungsgemäßen Verbindung geeignet sind, aus bekannten Verbindungen durch entsprechende Kupplungsreaktionen erhalten werden.

Verbindungen mit einer Diarylamin-Gruppe können durch bekannte Kupplungsreaktionen mit weiteren Aryl- oder Heteroarylverbindungen umgesetzt werden, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Im allen folgenden Syntheseschemata sind die Verbindungen zur Vereinfachung der Strukturen mit einer geringen Anzahl an Substituenten gezeigt gezeigt. Dies schließt das Vorhandensein von beliebigen weiteren Substituenten in den Verfahren nicht aus.

Eine Umsetzung ergibt sich beispielhaft gemäß folgenden Schemata, ohne dass hierdurch eine Beschränkung erfolgen soll. Die Teilschritte der einzelnen Schemata können hierbei beliebig kombiniert werden.

## Schema 1: Aufbau einer geeigneten cyclischen Diarylaminverbindung

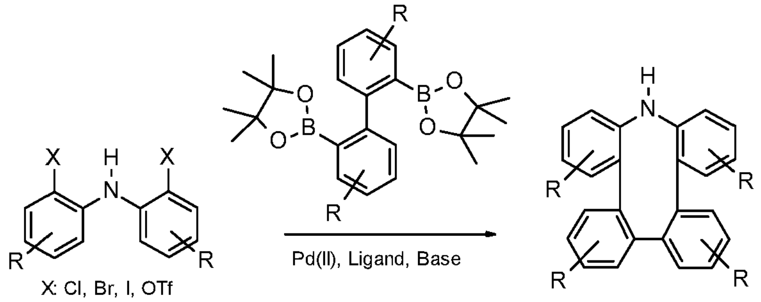


## Schema 2: Buchwald- oder Ullmann-Kupplung zur erfindungsgemäßen Verbindung

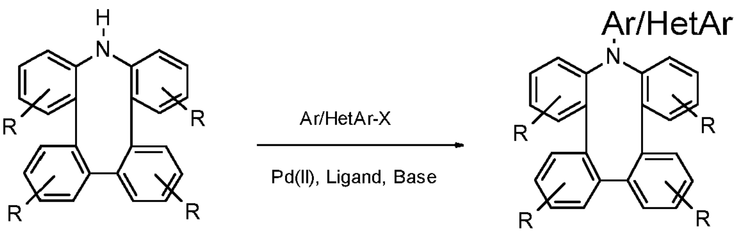


## Schema 3: SNAr-Reaktion zur erfindungsgemäßen Verbindung

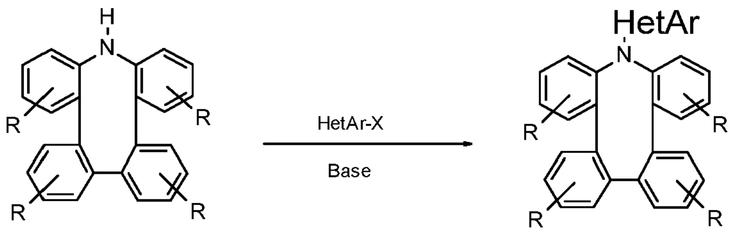


Die Bedeutung der in den Schemata 1 bis 3 verwendeten Symbole entspricht im Wesentlichen denen, die für Formel (I) definiert wurde, wobei aus Gründen der Übersichtlichkeit auf eine Nummerierung verzichtet wurde. Die Abkürzung Ar steht hierbei für einen Arylrest, HetAr für einen Heteroarylrest.

Ausgehend von 2,2'-Dihalogen- oder Triflat-substituierten sec. Arylaminen kann in einem ersten Schritt ein 2,2'-Bispinakolatoboran-substituiertes Biphenyl via Palladium-katalysierter Suzuki-Kupplung dargestellt werden. Für diesen Reaktionstyp können literaturbekannte Katalysatorsysteme, bestehend aus einer Pd-Quelle (z.B. $Pd(ac)_2$, $PdCl_2$, $Pd(dba)_2$, etc. ), einem Phosphin (z.B. Triphenylphosphin, Tri-o-tolyl phosphin, Tricyclohexylphosphin, Bisdiphenyl-phosphinoferrocen, S-Phos, X-Phos, Ru-Phos, etc.) und einer Base (KAc, $K_3PO_4$, $Cs(CO_3)_2$, etc.) verwendet werden.

Das so erhaltene sec. Amin kann durch Umsetzung mit Aryl-/Heteroarylhalogeniden- oder -triflaten in einer Palladium-katalysierten Buchwald- bzw. einer Kupfer-katalysierten Ullmann-Kupplung zu den erfindungsgemäßen tert. Aminen umgesetzt werden.

Die Umsetzung des sec. Amins mit kann alternativ mit elektronenarmen Heterocyclen (Pyridinen, Diazinen, Triazinen) in Gegenwart einer Base zu den erfindungsgemäßen tert. Aminen erfolgen.

Die so erhaltenen tert. Amine können, sofern sie in p-Position zum N-Atom ein Wasserstoffatom aufweisen, nach gängigen Verfahren, z.B. mit Brom oder N-Bromsuccinimid, bromiert werden. So erhaltene Bromide können durch gängige Verfahren (Grignard-Cross-, Heck-, Suzuki-, Negishi-, Sonogashira-, Yamamoto-, Buchwald-, Ullmann-Kupplung, etc.) weiter funktionalisiert bzw. in Oligomere, Dendrimere oder Polymere umgewandelt werden.

Die gezeigten Verfahren zur Synthese der erfindungsgemäßen Verbindungen sind exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Durch diese Verfahren, gegebenenfalls gefolgt von Aufreinigung, wie z. B. Umkristallisation oder Sublimation, lassen sich die erfindungsgemäßen Verbindungen, umfassend Strukturen gemäß Formel (I) in hoher Reinheit, bevorzugt mehr als 99 % (bestimmt mittels $^1$H-NMR und/oder HPLC) erhalten.

Die erfindungsgemäßen Verbindungen können auch geeignete Substituenten aufweisen, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityl- oder verzweigte Terphenyl- oder Quaterphenylgruppen substituiert sein, die eine Löslichkeit in gängigen organischen Lösemitteln bewirken, so dass die Verbindungen beispielsweise in Toluol oder Xylol bei Raumtemperatur in ausreichender Konzentration löslich sind, um die Verbindungen aus Lösung verarbeiten zu können. Diese löslichen Verbindungen eignen sich besonders gut für die Verarbeitung aus Lösung, beispielsweise durch Druckverfahren. Weiterhin ist festzuhalten, dass die erfindungsgemäßen Verbindungen, umfassend mindestens eine Struktur der Formel (I) bereits eine gesteigerte Löslichkeit in diesen Lösungsmitteln besitzen.

Die erfindungsgemäßen Verbindungen können auch mit einem Polymer gemischt werden. Ebenso ist es möglich, diese Verbindungen kovalent in ein Polymer einzubauen. Dies ist insbesondere möglich mit Verbindungen, welche mit reaktiven Abgangsgruppen, wie Brom, Iod, Chlor, Boronsäure oder Boronsäureester, oder mit reaktiven, polymerisierbaren Gruppen, wie Olefinen oder Oxetanen, substituiert sind. Diese können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten Strukturen der Formel (I) oder erfindungsgemäße Verbindungen, wobei ein oder mehrere Bindungen der erfindungsgemäßen Verbindungen oder der Strukturen der Formel (I) zum Polymer, Oligomer oder Dendrimer vorhanden sind. Je nach Verknüpfung der Strukturen der Formel (I) bzw. der Verbindungen bilden diese daher eine Seitenkette des Oligomers oder Polymers oder sind in der Hauptkette verknüpft. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Copolymere, wobei die Einheiten gemäß Formel (I) bzw. die zuvor und nachfolgend ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/022026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten.

Von besonderem Interesse sind des Weiteren erfindungsgemäße Verbindungen, die sich durch eine hohe Glasübergangstemperatur auszeichnen. In diesem Zusammenhang sind insbesondere erfindungsgemäße Verbindungen umfassend Strukturen der allgemeinen Formel (I) bzw. die zuvor und nachfolgend ausgeführten bevorzugten Ausführungsformen bevorzugt, die eine Glasübergangstemperatur von mindestens 70°C, besonders bevorzugt von mindestens 110°C, ganz besonders bevorzugt von mindestens 125°C und insbesondere bevorzugt von mindestens 150°C aufweisen, bestimmt nach DIN 51005 (Version 2005-08).

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise

Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Diese weitere Verbindung kann auch polymer sein.

Nochmals ein weiterer Gegenstand der vorliegenden Erfindung ist daher eine Zusammensetzung enthaltend eine erfindungsgemäße Verbindung und wenigstens ein weiteres organisches funktionelles Material. Funktionelle Materialien sind generell die organischen oder anorganischen Materialien, welche zwischen Anode und Kathode eingebracht sind. Vorzugsweise ist das organisch funktionelle Material ausgewählt aus der Gruppe bestehend aus fluoreszierenden Emittern, phosphoreszierenden Emittern, Emittern, die TADF (thermally activated delayed fluorescence) zeigen, Host-Materialien, Elektronentransportmaterialien, Elektroneninjektionsmaterialien, Lochtransportmaterialien, Lochinjektionsmaterialien, Elektronenblockiermaterialien, Lochblockiermaterialien, Wide-Band-Gap-Materialien und n-Dotanden.

Gemäß einem besonderen Aspekt der vorliegenden Erfindung können die erfindungsgemäßen Verbindungen als Emitter, vorzugsweise als fluoreszierender Emitter eingesetzt werden, wobei Emitter vielfach in Kombination mit geeigneten Matrixmaterialien verwendet werden. Ferner können die erfindungsgemäßen Verbindungen als Matrixmaterial, insbesondere für phosphoreszierende Emitter eingesetzt werden, wobei Matrixmaterialien vielfach in Kombination mit weiteren Matrixmaterialien verwendet werden.

Die vorliegenden Erfindung betrifft daher auch eine Zusammensetzung enthaltend wenigstens eine Verbindung umfassend Strukuren gemäß Formel (I) bzw. die zuvor und nachfolgend ausgeführten bevorzugten Ausführungsformen sowie wenigstens ein weiteres Matrixmaterial. Gemäß einem besonderen Aspekt der vorliegenden Erfindung weist das weitere Matrixmaterial elektronentransportierende Eigenschaften auf.

Ein weiterer Gegenstand der vorliegenden Erfindung stellt eine Zusammensetzung dar, enthaltend wenigstens eine Verbindung, umfassend mindestens eine Struktur gemäß Formel (I) bzw. die zuvor und nachfolgend ausgeführten bevorzugten Ausführungsformen sowie wenigstens ein Wide-Band-Gap-Material, wobei unter Wide-Band-Gap-Material ein Material im Sinne der Offenbarung von US 7,294,849 verstanden wird. Diese Systeme zeigen besondere vorteilhafte Leistungsdaten in elektrolumineszierenden Vorrichtungen.

Vorzugsweise kann die zusätzliche Verbindung eine Bandlücke (band gap) von 2,5 eV oder mehr, bevorzugt 3,0 eV oder mehr, ganz bevorzugt von 3,5 eV oder mehr aufweisen. Die Bandlücke kann unter anderem durch die Energieniveaus des highest occupied molecular orbital (HOMO) und des lowest unoccupied molecular orbital (LUMO) berechnet werden.

Molekülorbitale, insbesondere auch das highest occupied molecular orbital (HOMO) und das lowest unoccupied molecular orbital (LUMO), deren Energieniveaus sowie die Energie des niedrigsten Triplettzustands $T_1$ bzw. des niedrigsten angeregten Singulettzustands $S_1$ der Materialien werden über quantenchemische Rechnungen bestimmt. Zur Berechnung organischer Substanzen wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SCF/DFT/Default Spin/B3PW91" mit dem Basissatz "6-31G(d)" verwendet (Charge 0, Spin Singlet). Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

Die hierin beschriebene Methode ist unabhängig von dem verwendeten Softwarepaket und liefert immer dieselben Ergebnisse. Beispiele oft benutzter Programme für diesen Zweck sind "Gaussian09W" (Gaussian Inc.) und Q-Chem 4.1 (Q-Chem, Inc.).

Die vorliegende Erfindung betrifft auch eine Zusammensetzung umfassend wenigstens eine Verbindung umfassend Strukuren gemäß Formel (I) bzw. die zuvor und nachfolgend ausgeführten bevorzugten Ausführungsformen sowie wenigstens einen phosphoreszierende Emitter, wobei unter dem Begriff phosphoreszierende Emitter auch phosphoreszierende Dotanden verstanden werden.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Bevorzugte phosphoreszierende Dotanden zur Verwendung in Matrix-Systemen, vorzugsweise Mixed-Matrix-Systemen sind die im Folgenden angebenen bevorzugten phosphoreszierenden Dotanden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2016/124304, WO 2016/015815, WO 2016/000803, WO 2015117718, WO 2015104045 und WO 2015036074 . Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

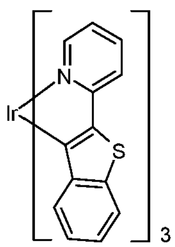

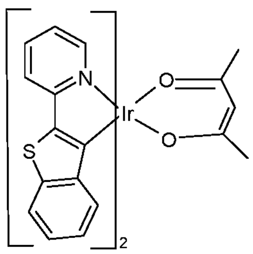

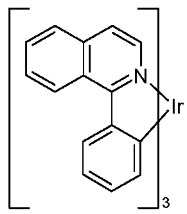

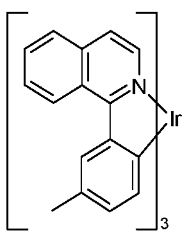

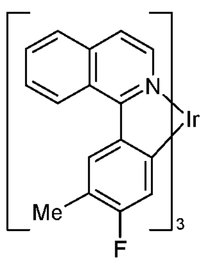

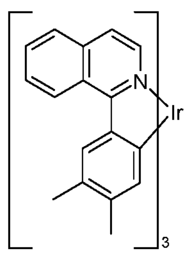

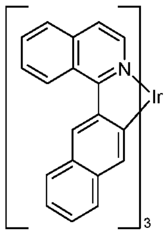
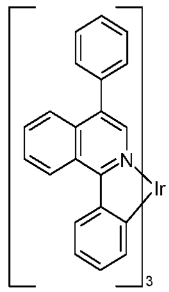
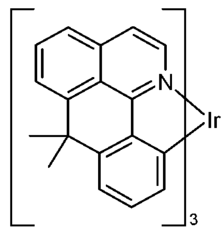

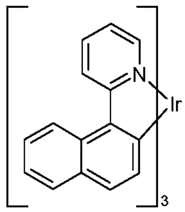
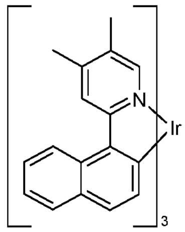
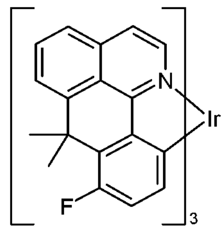

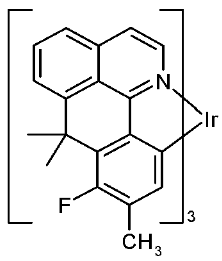
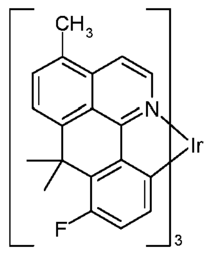
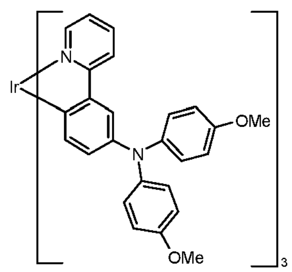

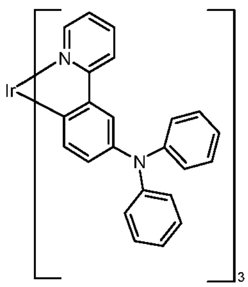
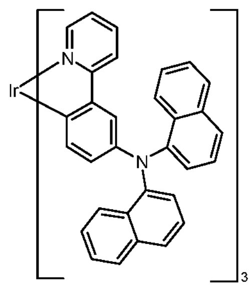
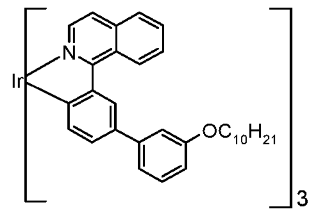

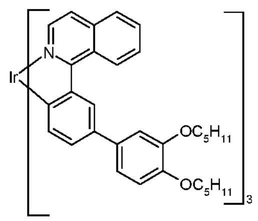
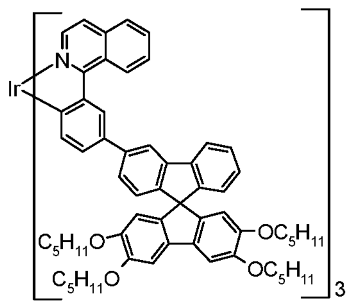
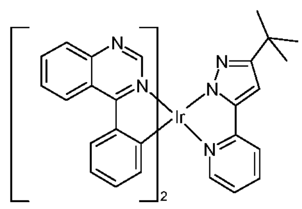

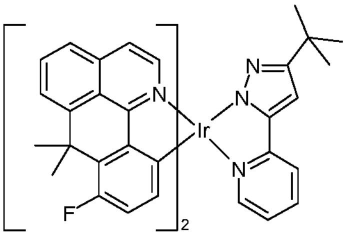
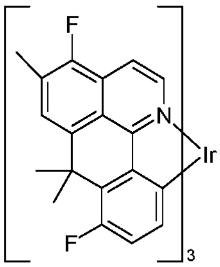
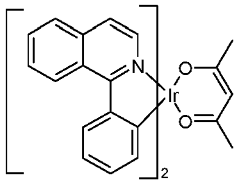

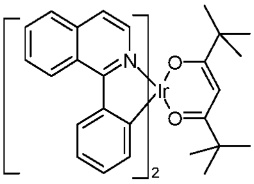
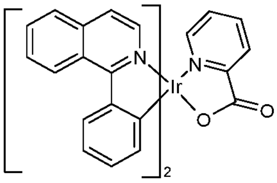
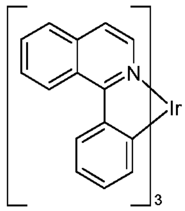

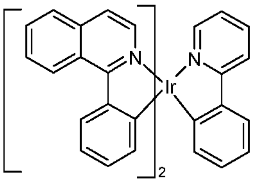
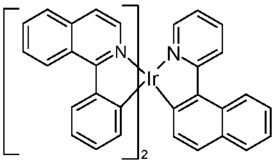
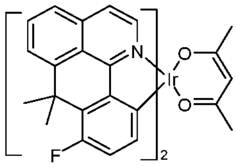

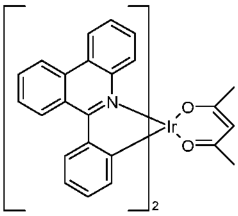
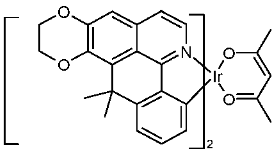
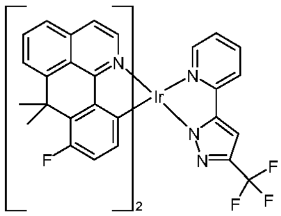

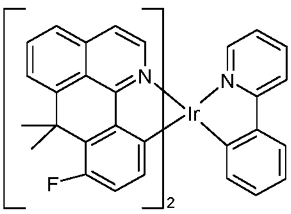
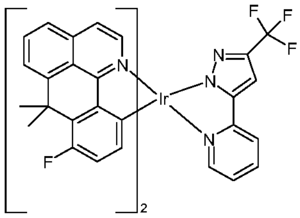
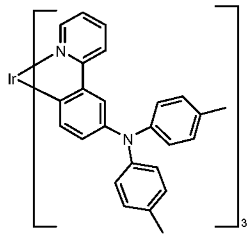

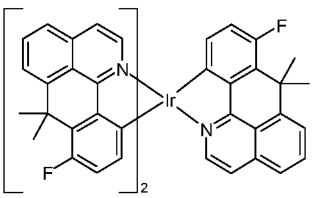
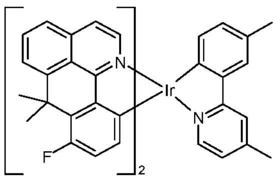
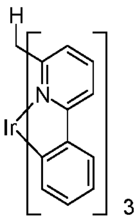

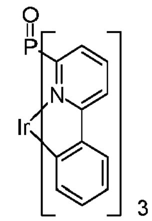
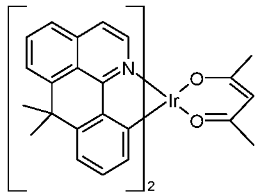
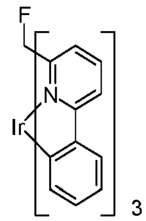

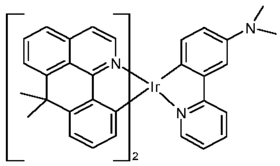
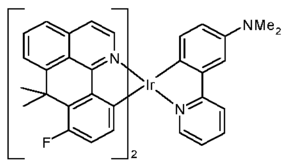
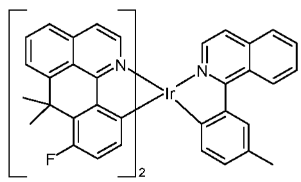

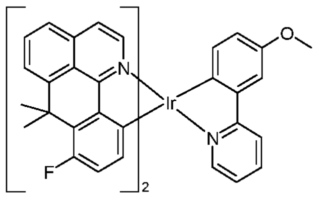
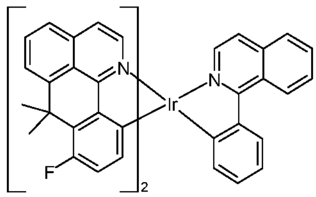
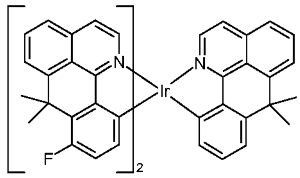

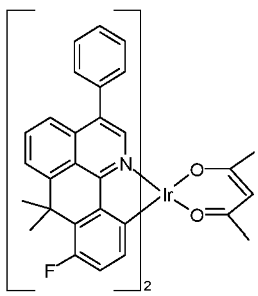
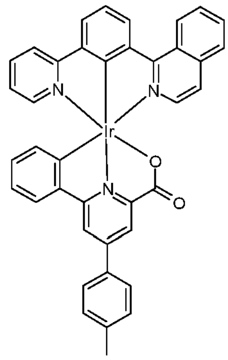
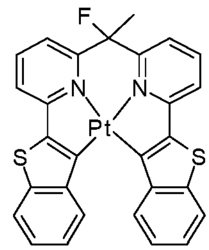

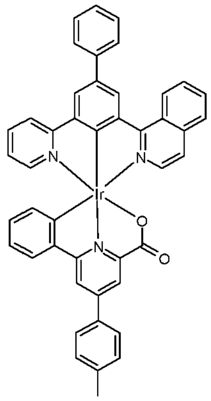
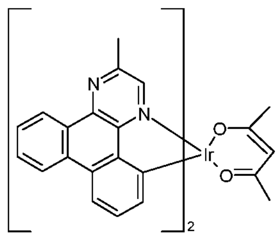
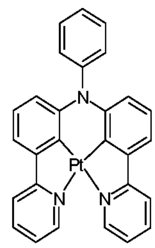

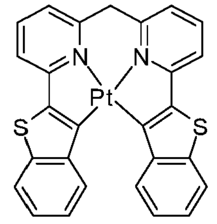
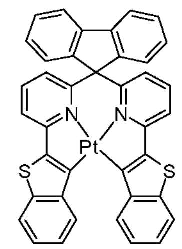
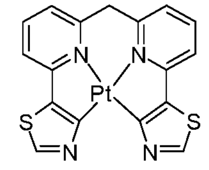

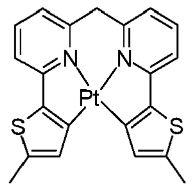
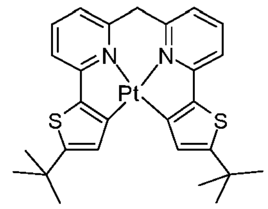
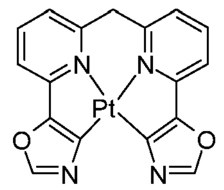

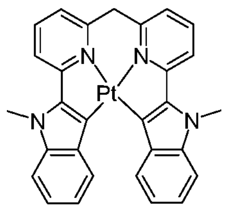
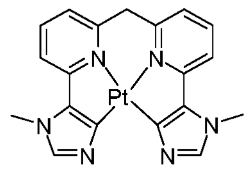
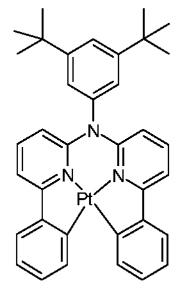

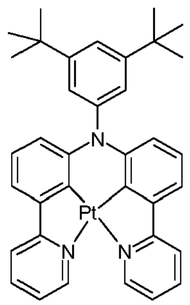
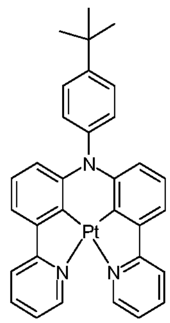
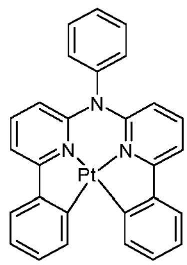

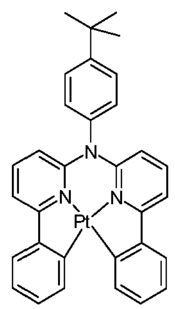
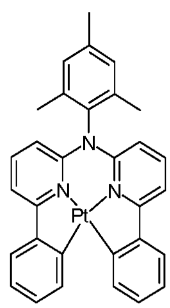
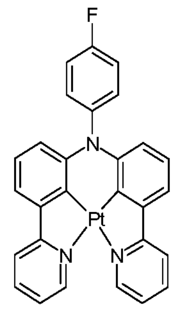

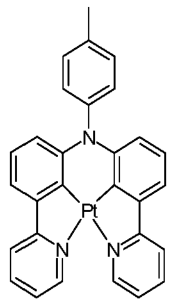
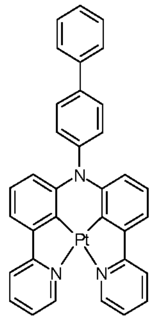
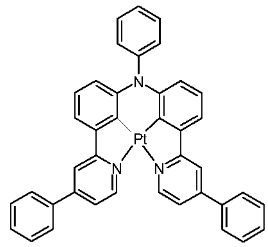

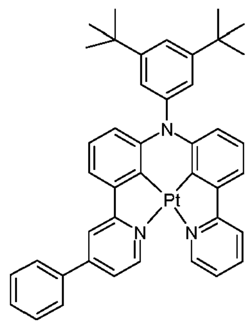
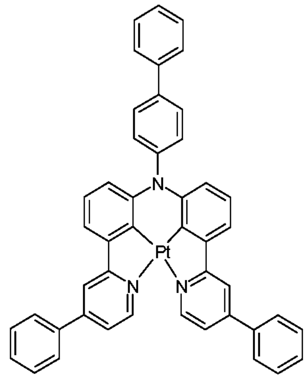
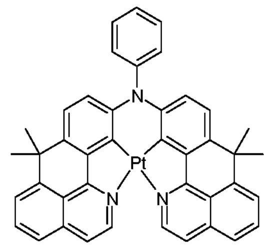

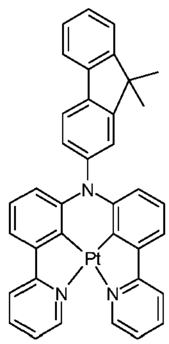
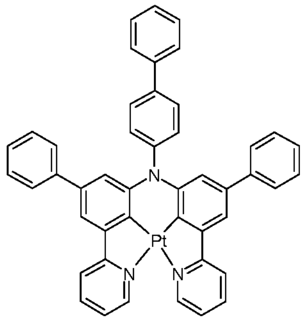
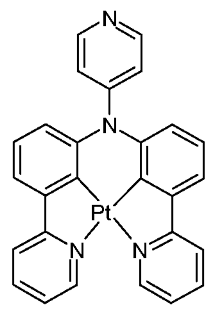

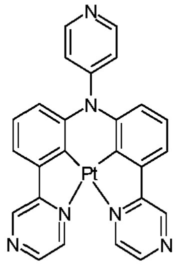
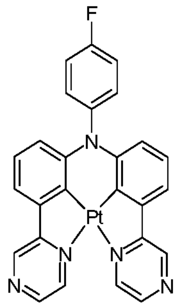
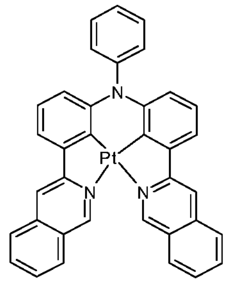

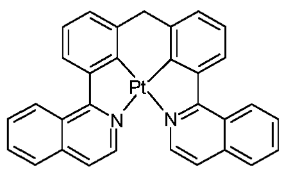
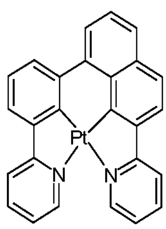
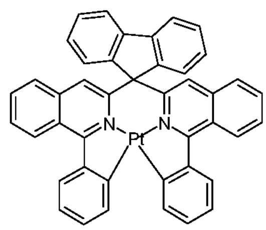

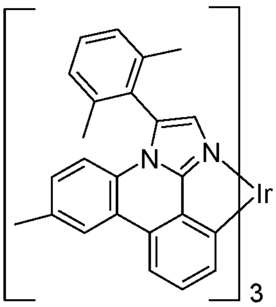
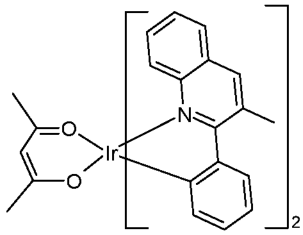
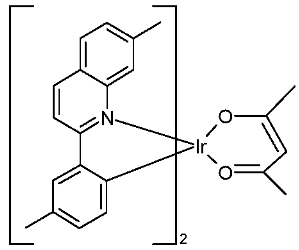

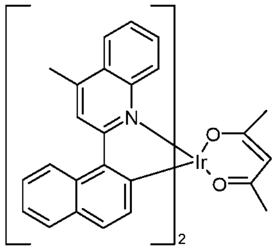
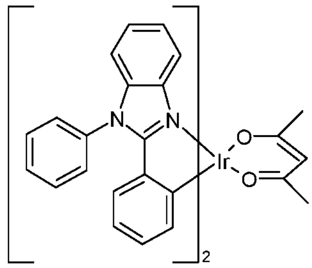
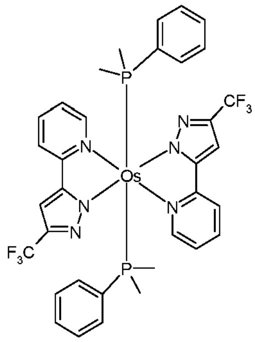

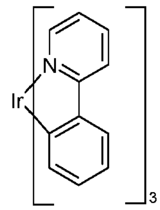
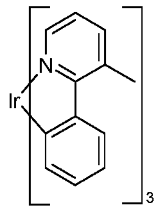
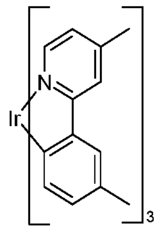

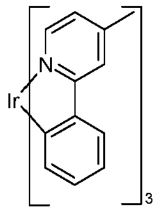
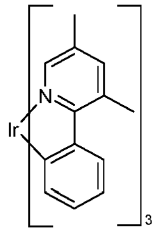
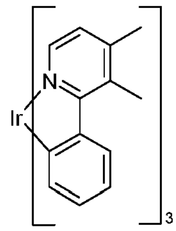

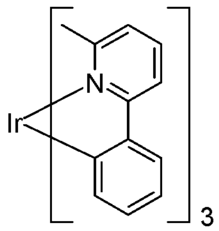
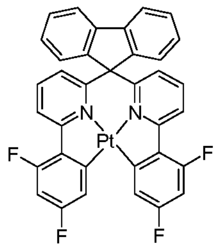
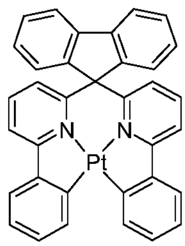

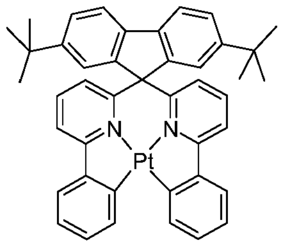

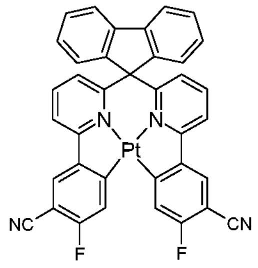

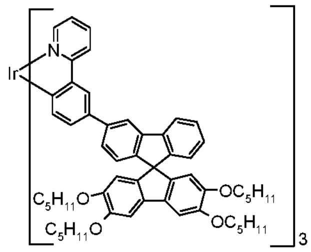

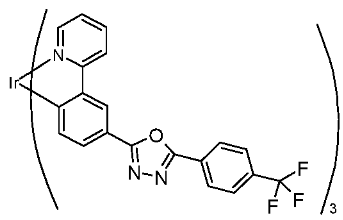

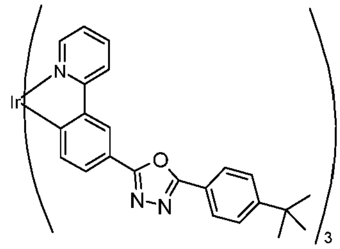

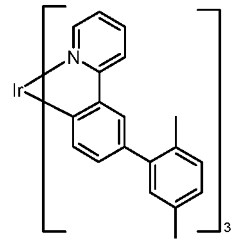

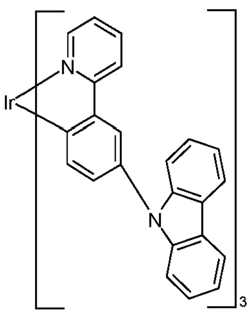

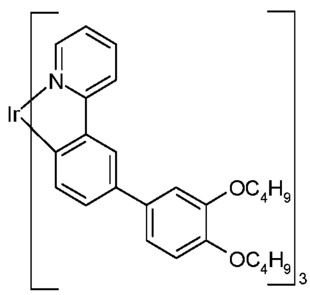

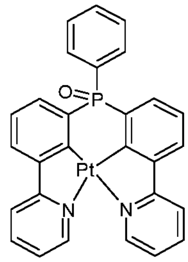

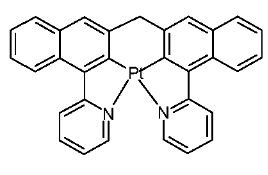

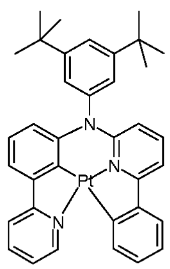

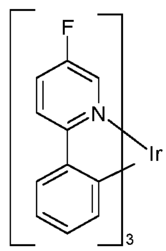

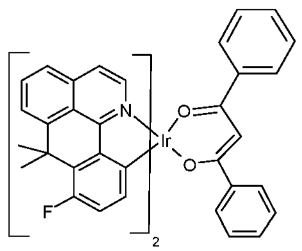

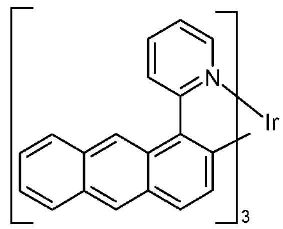

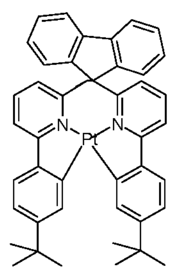

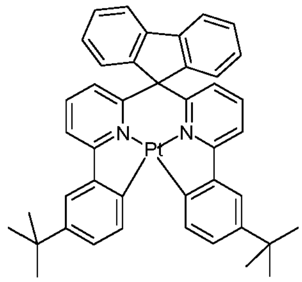
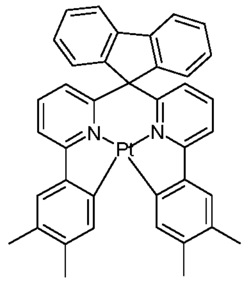
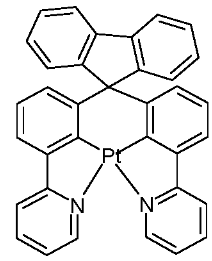

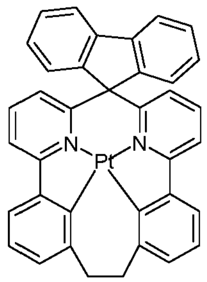
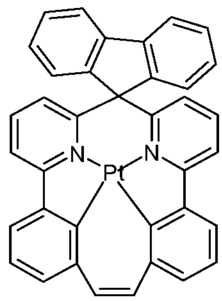
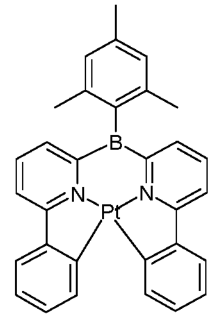

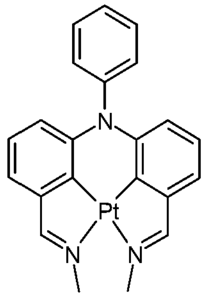
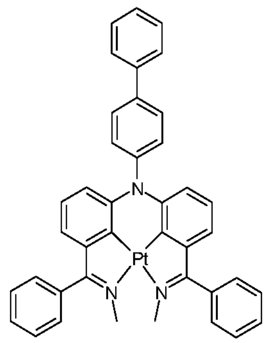
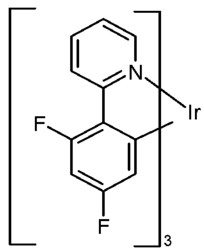

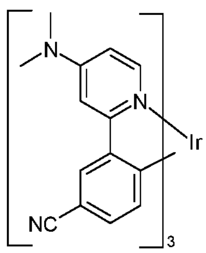
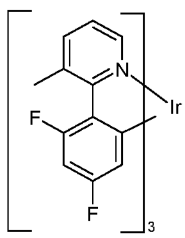
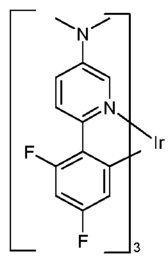

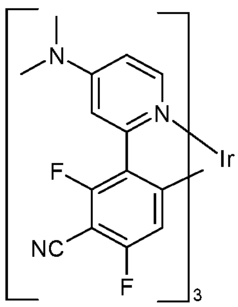
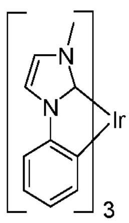
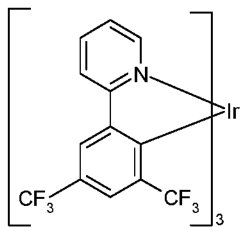

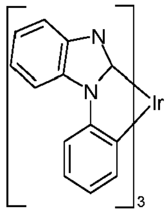
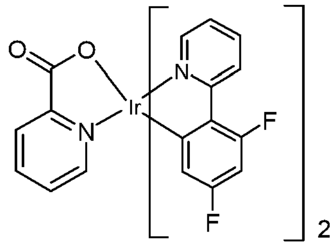
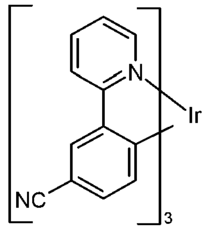

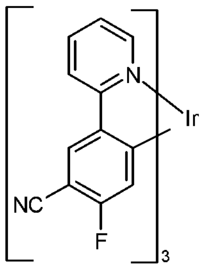
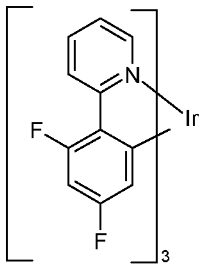
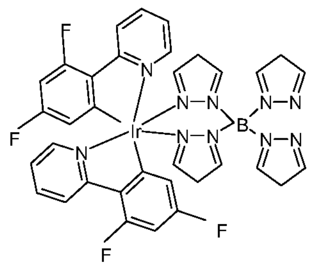

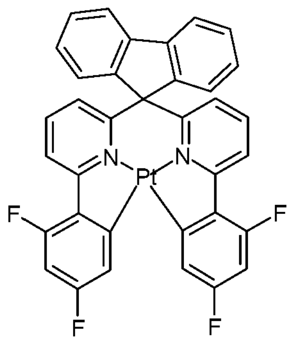
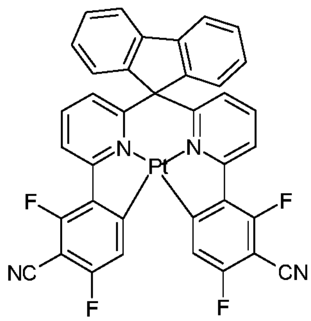
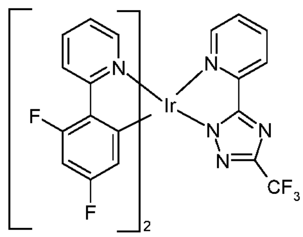

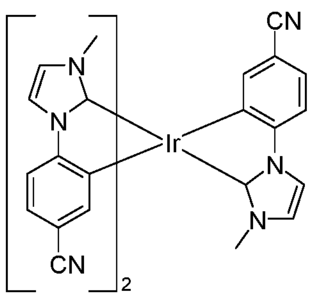
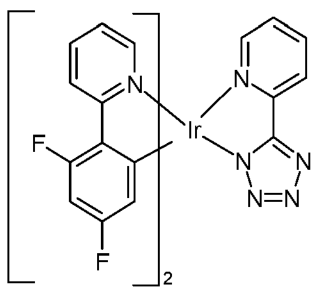
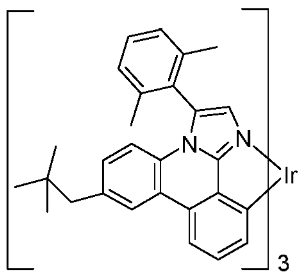

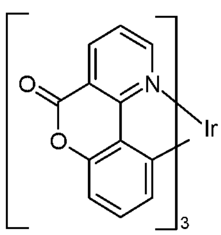
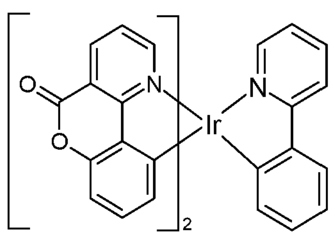
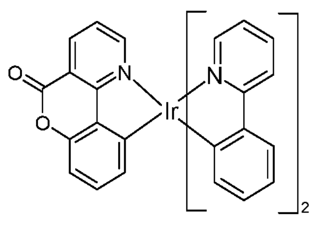

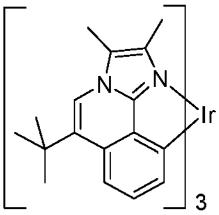
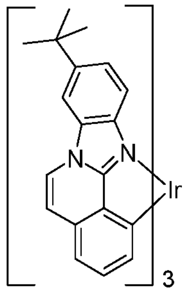
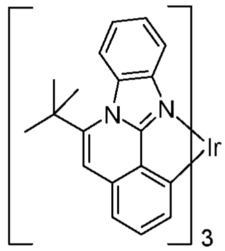

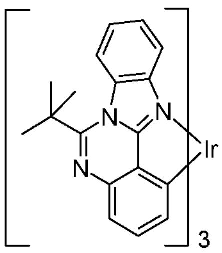
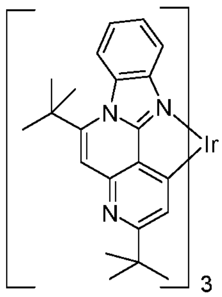
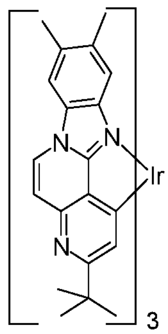

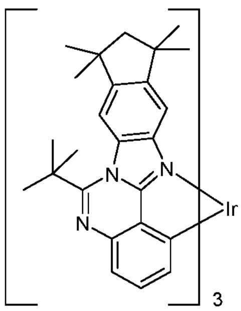
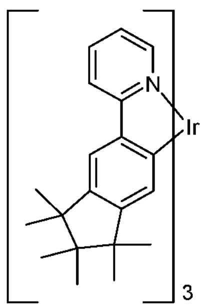
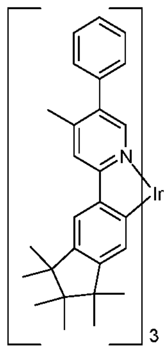

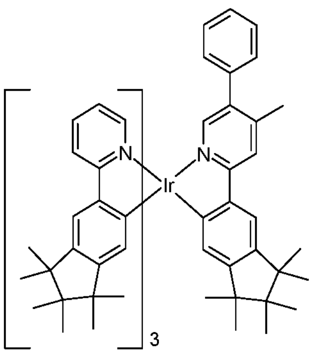
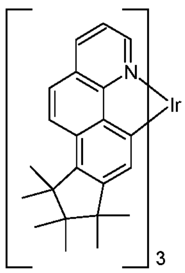
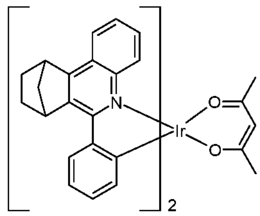

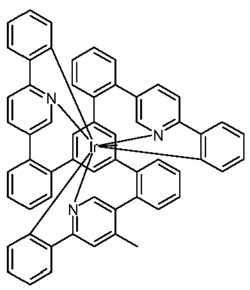
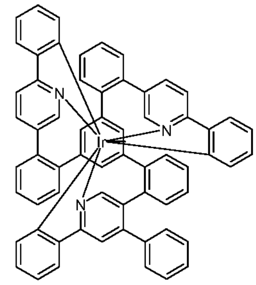
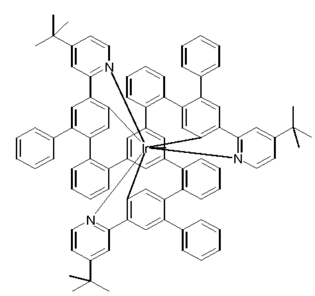

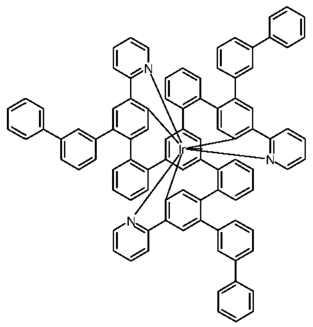

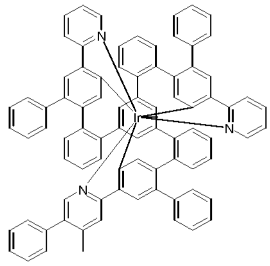

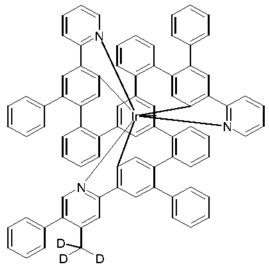

Die oben beschriebenen Verbindungen, umfassend Strukturen der Formel (I) bzw. die oben aufgeführten bevorzugten Ausführungsformen, können in einer elektronischen Vorrichtung bevorzugt als aktive Komponente verwendet werden. Unter einer elektronischen Vorrichtung wird eine Vorrichtung verstanden, welche Anode, Kathode und mindestens eine zwischen Anode und Kathode liegende Schicht enthält, wobei diese Schicht mindestens eine organische bzw. metallorganische Verbindung enthält. Die erfindungsgemäße elektronische Vorrichtung enthält also Anode, Kathode und mindestens eine dazwischen liegende Schicht, welche mindestens eine Verbindung, umfassend Strukturen der Formel (I), enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs,enthaltend in mindestens einer Schicht mindestens eine Verbindung, umfassend Strukturen der Formel (I). Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien.

Eine bevorzugte Ausführungsform der Erfindung sind organische Elektrolumineszenzvorrichtungen. Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoOs oder $WO_3$ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein Hybrid-System handeln, wobei eine oder mehrere Schichten fluoreszieren und eine oder mehrere andere Schichten phosphoreszieren.

In einer bevorzugten Ausführungsform der Erfindung enthält die organische Elektrolumineszenzvorrichtung die erfindungsgemäße Verbindung umfassend Strukturen gemäß Formel (I) bzw. die oben aufgeführten bevorzugten Ausführungsformen als Matrixmaterial, vorzugsweise als lochleitendes Matrixmaterial in einer oder mehreren emittierenden Schichten, bevorzugt in Kombination mit einem weiteren Matrixmaterial, vorzugsweise einem elektronenleitenden Matrixmaterial. In einer weiteren bevorzugten Ausführungsform der Erfindung ist das weitere Matrixmaterial eine lochtransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist. Eine emittierende Schicht umfasst mindestens eine emittierende Verbindung.

Geeignete Matrixmaterialien, welche in Kombination mit den Verbindungen gemäß Formel (I) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, insbesondere Monoamine, z. B. gemäß WO 2014/015935, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivate, z. B. gemäß WO 2012/048781, Lactame, z. B. gemäß WO 2011/116865, WO 2011/137951 oder WO 2013/064206, oder 4-Spirocarbazol-Derivate, z. B. gemäß WO 2014/094963 oder WO 2015/192939. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (TA-1),

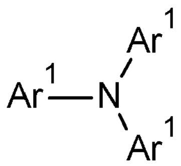

Formel (TA-1)

wobei $Ar^1$ gleich oder verschieden bei jedem Auftreten ein aromatisches oder heteroaromatisches Ringsystem mit 6 bis 40 C-Atomen, das jeweils mit einem oder mehreren Resten $R^2$ substituiert sein kann, eine Aryloxygruppe mit 5 bis 60 aromatischen Ringatomen, die mit einem oder mehreren Resten $R^2$ substituiert sein kann, oder einer Aralkylgruppe mit 5 bis 60 aromatischen Ringatomen, die jeweils mit einem oder mehreren Resten $R^2$ substituiert sein kann, darstellt, wobei optional zwei oder mehr benachbarte Substituenten $R^2$ ein mono- oder polycyclisches, aliphatisches, heteroaliphatisches, aromatisches oder heteroaromatisches Ringsystem, vorzugsweise ein mono- oder polycyclisches, aliphatisches Ringsystem, bilden können, das mit einem oder mehreren Resten $R^3$ substituiert sein kann, wobei das Symbol $R^2$ die zuvor, insbesondere für Formel (I) genannte Bedeutung aufweist. Vorzugsweise stellt $Ar^1$ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten $R^2$ substituiert sein kann, vorzugsweise jedoch unsubstituiert ist.

Beispiele für geeignete Gruppen $Ar^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind.

Bevorzugt sind die Gruppen $Ar^1$ gleich oder verschieden bei jedem Auftreten ausgewählt aus den oben genannten Gruppen $R^1$-1 bis $R^1$-95, besonders bevorzugt $R^1$-1 bis $R^1$-54.

In einer bevorzugten Ausführungsform der Verbindungen der Formel (TA-1) ist mindestens eine Gruppe $Ar^1$ ausgewählt aus einer Biphenylgruppe, wobei es sich um eine ortho-, meta- oder para-Biphenylgruppe handeln kann. In einer weiteren bevorzugten Ausführungsform der Verbindungen der Formel (TA-1) ist mindestens eine Gruppe $Ar^1$ ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (TA-1) ist mindestens eine Gruppe $Ar^1$ ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (TA-1) ist eine Gruppe $Ar^1$ ausgewählt aus einer Fluoren- oder Spirobifluorengruppe, insbesondere einer 4-Fluoren- bzw. 4-Spirobifluorengruppe, und eine Gruppe $Ar^1$ ist ausgewählt aus einer Biphenylgruppe, insbesondere einer para-Biphenylgruppe, oder einer Fluorengruppe, insbesondere einer 2-Fluorengruppe, und die dritte Gruppe $Ar^1$ ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazolderivate, die als Co-Host-Materialien zusammen mit den erfindungsgemäßen Verbindungen eingesetzt werden, sind ausgewählt aus den Verbindungen der folgenden Formel (TA-2),

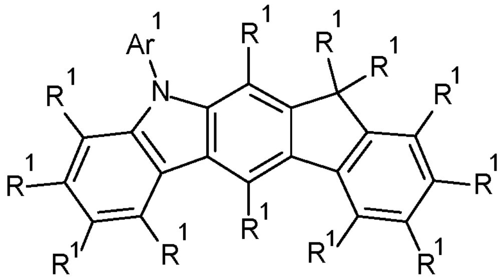

Formel (TA-2)

wobei $Ar^1$ und $R^1$ die oben insbesondere für Formeln (I) und/oder (TA-1) aufgeführten Bedeutungen aufweisen. Dabei sind bevorzugte Ausführungsformen der Gruppe $Ar^1$ die oben aufgeführten Strukturen $R^1$-1 bis $R^1$-95, besonders bevorzugt $R^1$-1 bis $R^1$-54.

Eine bevorzugte Ausführungsform der Verbindungen der Formel (TA-2) sind die Verbindungen der folgenden Formel (TA-2a),

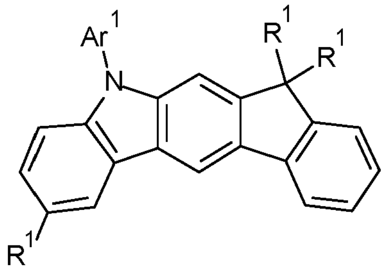

Formel (TA-2a)

wobei $Ar^1$ und $R^1$ die oben, insbesondere für Formeln (I) und/oder (TA-1) aufgeführten Bedeutungen aufweisen. Dabei stehen die beiden Gruppen $R^1$, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen $R^1$, die an das Indenokohlenstoffatom gebunden sind, für Methylgruppen. Weiterhin bevorzugt steht der Substituent $R^1$, der in Formel (TA-2a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (TA-3),

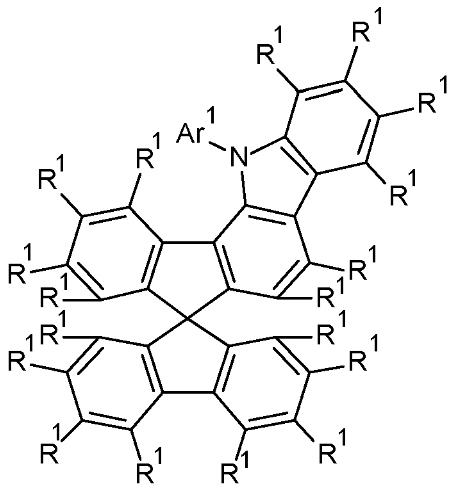

Formel (TA-3)

wobei $Ar^1$ und $R^1$ die oben, insbesondere für Formel (I) aufgeführten Bedeutungen aufweisen. Dabei sind bevorzugte Ausführungsformen der Gruppe $Ar^1$ die oben aufgeführten Strukturen $R^1$-1 bis $R^1$-95, besonders bevorzugt $R^1$-1 bis $R^1$-54.

Eine bevorzugte Ausführungsform der Verbindungen der Formel (TA-3) sind die Verbindungen der folgenden Formel (TA-3a),

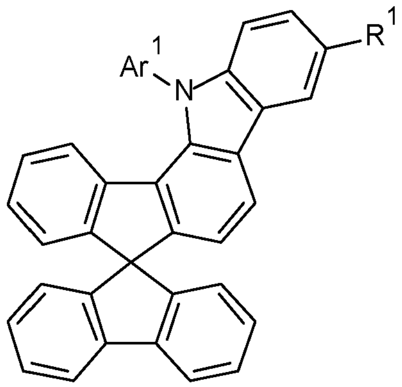

Formel (TA-3a)

wobei $Ar^1$ und $R^1$ die oben, insbesondere für Formel (I) aufgeführten Bedeutungen aufweisen. Dabei sind bevorzugte Ausführungsformen der Gruppe $Ar^1$ die oben aufgeführten Strukturen $R^1$-1 bis $R^1$-95, besonders bevorzugt $R^1$-1 bis $R^1$-54.

Bevorzugte Lactame, die als Co-Host-Materialien zusammen mit den erfindungsgemäßen Verbindungen eingesetzt werden, sind ausgewählt aus den Verbindungen der folgenden Formel (LAC-1),

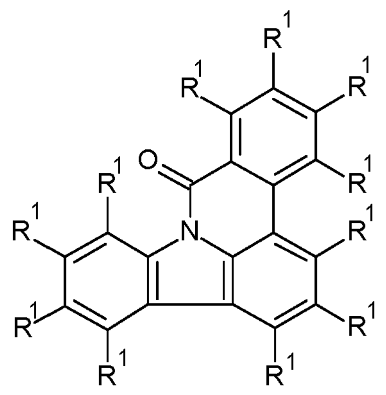

Formel (LAC-1)

wobei $R^1$ die oben, insbesondere für Formel (I) aufgeführte Bedeutung aufweist.

Eine bevorzugte Ausführungsform der Verbindungen der Formel (LAC-1) sind die Verbindungen der folgenden Formel (LAC-1a),

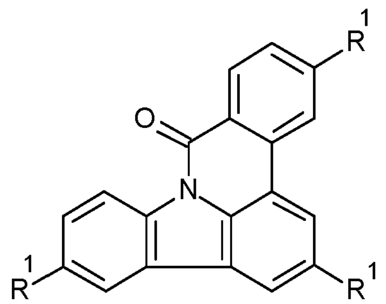

Formel (LAC-1a)

wobei $R^1$ die oben, insbesondere für Formel (I) genannte Bedeutung aufweist. Dabei steht $R^1$ bevorzugt gleich oder verschieden bei jedem Auftreten für H oder ein aromatisches oder heteroaromatisches Ringsystem mit 5 bis 40 aromatischen Ringatomen, das mit einem oder mehreren Resten $R^2$ substituiert sein kann, wobei $R^2$ die zuvor, insbesondere für Formel (I) genannte Bedeutung aufweisen kann. Ganz besonders bevorzugt sind die Substituenten $R^1$ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten $R^2$ substituiert sein kann, bevorzugt aber unsubstituiert ist. Beispiele für geeignete Substituenten $R^1$ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste $R^2$ substituiert sein können, bevorzugt aber unsubstituiert sind. Dabei sind geeignete Strukturen $R^1$ die gleichen Strukturen, wie sie zuvor für R-1 bis R-95 abgebildet sind, besonders bevorzugt $R^1$-1 bis $R^1$-54.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Ebenso bevorzugt ist die Verwendung einer Mischung aus einem ladungstransportierenden Matrixmaterial und einem elektronisch inerten Matrixmaterial, welches nicht bzw. nicht in wesentlichem Maße am Ladungstransport beteiligt ist, wie z. B. in WO 2010/108579 beschrieben.

Weiterhin bevorzugt ist es, eine Mischung aus zwei oder mehr Triplett-Emittern zusammen mit einer Matrix einzusetzen. Dabei dient der Triplett-Emitter mit dem kürzerwelligen Emissionsspektrum als Co-Matrix für den Triplett-Emitter mit dem längerwelligen Emissionsspektrum.

Besonders bevorzugt kann eine erfindungsgemäße Verbindung umfassend Strukuren gemäß Formel (I) in einer bevorzugten Ausführungsform als Matrixmaterial in einer Emissionsschicht einer organischen elektronischen Vorrichtung, insbesondere in einer organischen elektrolumineszierenden Vorrichtung, beispielsweise in einer OLED oder OLEC, eingesetzt werden. Dabei ist das Matrixmaterial enthaltend eine Verbindung umfassend Strukuren gemäß Formel

(I) bzw. die zuvor und nachfolgend ausgeführten bevorzugten Ausführungsformen in der elektronischen Vorrichtung in Kombination mit einem oder mehreren Dotanden, vorzugsweise phosphoreszierenden Dotanden, vorhanden.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindung umfassend Strukuren gemäß Formel (I) bzw. die zuvor und nachfolgend ausgeführten bevorzugten Ausführungsformen als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Ferner ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in einer oder mehreren lochleitenden Schichten umfasst, als lochleitende Verbindung.

Weiterhin ist eine elektronische Vorrichtung, vorzugsweise eine organische Elektrolumineszenzvorrichtung Gegenstand der vorliegenden Erfindung, die eine oder mehrere erfindungsgemäße Verbindungen und/oder mindestens ein erfindungsgemäßes Oligomer, Polymer oder Dendrimer in emittierenden Schichten umfasst, als emittierende Verbindung, vorzugsweise als fluoreszierender Emitter, oder als Matrixmaterial, vorzugsweise in Kombination mit einem phosphoreszierenden Emitter.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Ebenso kommen hierfür organische Alkalimetallkomplexe in Frage, z. B. Liq (Lithiumchinolinat). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, z. B. PEDOT, PANI oder Derivate dieser Polymere. Bevorzugt ist weiterhin, wenn auf die Anode ein p-dotiertes Lochtransportmaterial als Lochinjektionsschicht aufgebracht wird, wobei sich als p-Dotanden Metalloxide, beispielsweise MoOs oder $WO_3$, oder (per)fluorierte elektronenarme Aromaten eignen. Weitere geeignete p-Dotanden sind HAT-CN (Hexacyano-hexaazatriphenylen) oder die Verbindung NPD9 von Novaled. Eine solche Schicht vereinfacht die Lochinjektion in Materialien mit einem tiefen HOMO, also einem betragsmäßig großen HOMO.

In den weiteren Schichten können generell alle Materialien verwendet werden, wie sie gemäß dem Stand der

Technik für die Schichten verwendet werden, und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine elektronischen Vorrichtung, insbesondere eine organische Elektrolumineszenzvorrichtung, welche dadurch gekennzeichnet ist, dass eine oder mehrere Schichten mit einem Sublimationsverfahren beschichtet werden. Dabei werden die Materialien in Vakuum-Sublimationsanlagen bei einem Anfangsdruck von üblicherweise kleiner $10^{-5}$ mbar, bevorzugt kleiner $10^{-6}$ mbar aufgedampft. Es ist auch möglich, dass der Anfangsdruck noch geringer oder noch höher ist, beispielsweise kleiner $10^{-7}$ mbar.

Bevorzugt ist ebenfalls eine elektronischen Vorrichtung, insbesondere eine organische Elektrolumineszenzvorrichtung, welche dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronischen Vorrichtung, insbesondere eine organische Elektrolumineszenzvorrichtung, welche dadurch gekennzeichnet ist, dass eine oder mehrere Schichten aus Lösung, wie z. B. durch Spincoating, oder mit einem beliebigen Druckverfahren, wie z. B. Siebdruck, Flexodruck, Offsetdruck oder Nozzle-Printing, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig, welche beispielsweise durch geeignete Substitution erhalten werden.

Die elektronische Vorrichtung, insbesondere die organische Elektrolumineszenzvorrichtung, kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine efindungsgemäße Verbindung umfassend Strukturen gemäß Formel (I) und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf elektronischen Vorrichtungen, insbesondere organische Elektrolumineszenzvorrichtungen enthaltend efindungsgemäße Verbindungen umfassend Strukturen gemäß Formel (I) bzw. die oben aufgeführten bevorzugten Ausführungsformen angewandt werden.

Die erfindungsgemäßen elektronischen Vorrichtungen, insbesondere organische Elektrolumineszenzvorrichtungen, zeichnen sich durch einen oder mehrere der folgenden überraschenden Vorteile gegenüber dem Stand der Technik aus:

1. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen, insbesondere als fluoreszierende Emitter, als Hostmaterial oder als elektronenleitende und/oder lochleitende Materialien, weisen eine sehr gute Lebensdauer auf. Hierbei bewirken diese Verbindungen insbesondere einen geringen Roll-off, d.h. einen geringen Abfall der Leistungseffizienz der Vorrichtung bei hohen Leuchtdichten.

2. Elektronische Vorrichtungen, insbesondere organische Elektrolumineszenzvorrichtungen enthaltend Verbindungen, Oligomere, Polymere oder Dendrimere mit Strukturen gemäß Formel (I) bzw. die zuvor und nachfolgend ausgeführten bevorzugten Ausführungsformen als fluoreszierende Emitter oder als elektronenleitende Materialien, lochleitende Materialien und/oder Hostmaterialien weisen eine hervorragende Effizienz auf. Hierbei bewirken erfindungsgemäße Verbindungen, Oligomere, Polymere oder Dendrimere mit Strukturen gemäß Formel (I) bzw. die zuvor und nachfolgend ausgeführten bevorzugten Ausführungsformen eine geringe Betriebsspannung bei Verwendung in elektronischen Vorrichtungen.

3. Die erfindungsgemäßen Verbindungen, Oligomere, Polymere oder Dendrimere mit Strukturen gemäß Formel (I) bzw. die zuvor und nachfolgend ausgeführten bevorzugten Ausführungsformen zeigen eine sehr hohe Stabilität und Lebensdauer.

4. Mit Verbindungen, Oligomere, Polymere oder Dendrimeren mit Strukturen gemäß Formel (I) bzw. den zuvor und nachfolgend ausgeführten bevorzugten Ausführungsformen kann in elektronischen Vorrichtungen, insbesondere organischen Elektrolumineszenzvorrichtungen, die Bildung von optischen Verlustkanälen vermieden werden. Hierdurch zeichnen sich diese Vorrichtungen durch eine hohe PL- und damit hohe EL-Effizienz von Emittern bzw. eine

ausgezeichnete Energieübertragung der Matrices auf Dotanden aus.

5. Die Verwendung von Verbindungen, Oligomere, Polymere oder Dendrimere mit Strukturen gemäß Formel (I) bzw. die zuvor und nachfolgend ausgeführten bevorzugten Ausführungsformen in Schichten elektronischer Vorrichtungen, insbesondere organischer Elektrolumineszenzvorrichtungen führt zu einer hohen Mobilität der Elektron-Leiterstrukturen.

6. Verbindungen, Oligomere, Polymere oder Dendrimere mit Strukturen gemäß Formel (I) bzw. die zuvor und nachfolgend ausgeführten bevozugten Ausführungsformen zeichnen sich durch eine ausgezeichnete thermische Stabilität aus, wobei Verbindungen mit einer Molmasse von weniger als ca. 1200 g/mol gut sublimierbar sind.

7. Verbindungen, Oligomere, Polymere oder Dendrimere mit Strukturen gemäß Formel (I) bzw. die zuvor und nachfolgend ausgeführten bevorzugten Ausführungsformen weisen eine ausgezeichnete Glasfilmbildung auf.

8. Verbindungen, Oligomere, Polymere oder Dendrimere mit Strukturen gemäß Formel (I) bzw. die zuvor und nachfolgend ausgeführten bevorzugten Ausführungsformen bilden aus Lösungen sehr gute Filme.

9. Die Verbindungen, Oligomere, Polymere oder Dendrimere umfassend Strukturen gemäß Formel (I) bzw. die zuvor und nachfolgend ausgeführten bevorzugten Ausführungsformen weisen ein überraschend hohes Triplett-Niveau $T_1$ auf.

Diese oben genannten Vorteile gehen nicht mit einer Verschlechterung der weiteren elektronischen Eigenschaften einher.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Ein nochmals weiterer Gegenstand der vorliegenden Erfindung ist die Verwendung einer erfindungsgemäßen Verbindung und/oder eines erfindungsgemäßen Oligomers, Polymers oder Dendrimers in einer elektronischen Vorrichtung als fluoreszierender Emitter, Hostmaterial für phosphoreszierende Emitter, Elektronentransportmaterial und/oder Lochtransportmaterial, vorzugsweise als Hostmaterial für phosphoreszierende Emitter oder als Lochtransportmaterial.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen. Besonders bevorzugt ist elektronische Vorrichtung ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), organischen elektrischen Sensoren, lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (I) bzw. gemäß den bevorzugten Ausführungsformen einsetzen.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

**Beispiele**

Die nachfolgenden Synthesen werden, sofern nicht anders angegeben, unter einer Schutzgasatmosphäre in getrockneten Lösungsmitteln durchgeführt. Die Edukte können von ALDRICH bezogen werden. Die Nummern bei den literaturbekannten Edukten, die teilweise in eckigen Klammern angegeben sind, sind die entsprechenden CAS-Nummern.

**Synthesebeispiele**

**Stufe 1:**

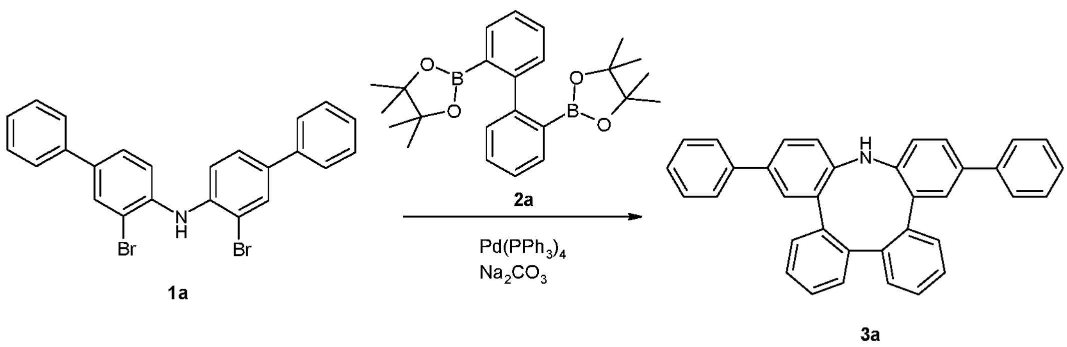


**Variante A:** Es werden 18.0g (37.6mmol, 1.0eq) 3-Bromo-*N*-(3-bromo[1,1'-biphenyl]-4-yl)-[1,1'-biphenyl]-4-amin [1388152-32-6] **1a,** zusammen mit 15.3g (37.6mmol, 1.0eq) 2,2'-[1,1'-biphenyl]-2,2'-diylbis[4,4,5,5-tetramethyl-1,3,2-dioxaborolane [398128-09-1] **2a** in jeweils 200ml Toluol und 1,4-Dioxan gelöst und mit 100ml Wasser versetzt. Der Ansatz wird für 30 Minuten mit Argon entgast. Anschließend werden 12.0g (106mmol, 3.0eq) Natriumcarbonat und 2.17g (1.88mmol, 0.05eq) Tetrakis(triphenylphosphin) zugegeben und der Ansatz für zwei Tage bei 100°C gerührt. Nach beendeter Reaktion wird die wässrige Phase abgetrennt, zweimal mit Toluol extrahiert und die vereinten organischen Phasen noch einmal mit Wasser gewaschen. Anschließend wird über Natriumsulfat getrocknet und die Lösungsmittel am Rotationsverdampfer entfernt. Der Rückstand wird aus Toluol/Heptan umkristallisiert. Es werden 7.27g (15.4mmol, 41%) des gewünschten Produktes **3a** erhalten.

**Variante B:** 0.02eq Palladium(II)-acetat, 0.05eq Tri-*o*-Tolylphosphin, 2.25eq Kaliumphosphat; in Toluol/Dioxan/Wasser 2:2:1 als Lösungsmittel

**Variante C:** 0.03eq Palladium(II)-acetat, 0.05eq Tri-*t*-Butylphosphin, 3.0eq Kaliumfluorid anhydr.; in THF als Lösungsmittel

Analog werden herestellt:

| Eintrag | Edukt 1 | Edukt 2 | Produkt 3 | Ausbeute | Variante |
|---|---|---|---|---|---|
| 3b | 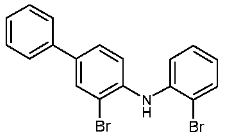 [101875-58-5] | 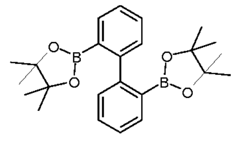 [398128-09-1] | 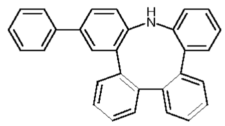 | 34% | C |
| 3c | 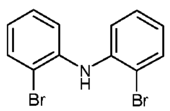 [67242-17-5] | 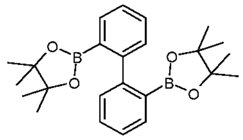 [398128-09-1] | 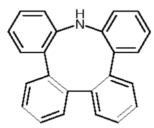 | 55% | B |
| 3d | 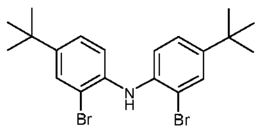 [1015228-11-1] | 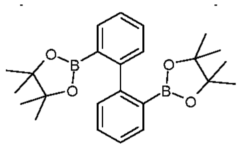 [398128-09-1] | 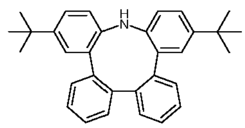 | 28% | C |

**Stufe 2:**

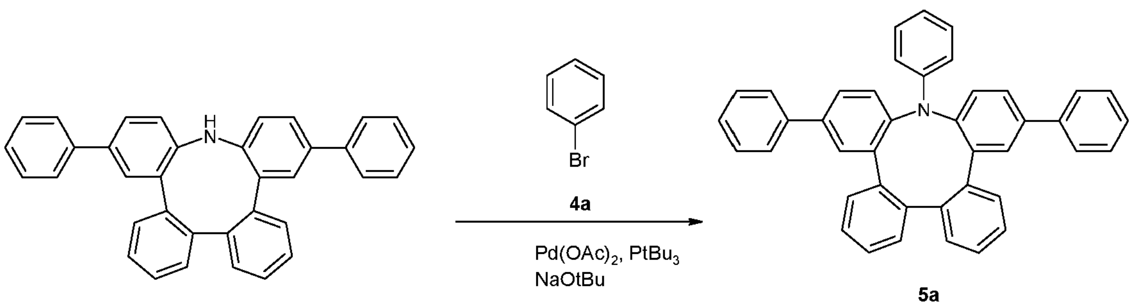


3a

5a

**Variante A:** Es werden 10.0g (21.2mmol, 1.0eq) der hergestellten Verbindung **3a** zusammen mit 4.00g (25.4mmol, 1.2eq) Brombenzol [108-86-1] **4a** in 200ml Toluol gelöst und für 30 Minuten mit Argon entgast. Anschließend werden 4.07g (42.4mmol, 2.0eq) Natrium-t-butoxid, 238mg (1.06mmol, 0.05eq) Palladium(II)-acetat und 2.1ml (2.12mmol, 0.10eq) Tri-t-butylphosphin (1.0M in Toluol) zugegeben und unter Rückfluss über Nacht gerührt. Nach beendeter Reaktion wird der Ansatz mit 200ml Wasser versetzt, die organische Phase abgetrennt und zweimal mit Wasser extrahiert. Die organische Phase wird über Natriumsulfat getrocknet und am Rotationsverdampfer auf ca. 80ml eingeengt. Der ausgefallene Feststoff wird abgesaugt und mittels Heißextraktion in Toluol aufgereinigt. Das Produkt wird dreimal mit Toluol/Heptan umkristallisiert und abschließend sublimiert. Es werden 6.59g (12.0mmol, 57%) der gewünschten Zielverbindung **5a** mit einer HPLC-Reinheit >99.9% erhalten.

**Variante B:** 0.05eq Tris(dibenzylideneaceton)dipalladium(0), 0.10eq Dicyclohexyl-(2',6'-dimethoxy-biphenyl-2-yl)-phosphan / S-Phos, 2.0eq Natrium-t-butoxid; in Toluol als Lösungsmittel

**Variante C:** 0.05eq 1,1'Bis(diphenylphosphino)ferrocendichlorpalladium(II) Komplex mit Dichlor-methan, 2.0eq Natrium-*t*-butoxid; in Toluol als Lösungsmittel

Analog werden hergestellt:

| Eintrag | Edukt 3 | Edukt 4 | Produkt 5 | Ausbeute [%] | Vari ante |
|---|---|---|---|---|---|
| 5b | 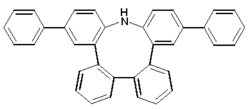 | 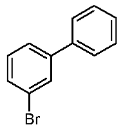 | 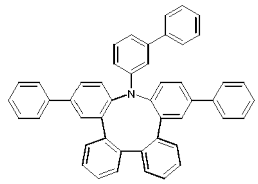 | 31 | A |
| 5c | 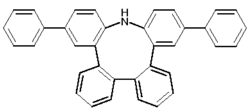 | 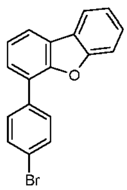 | 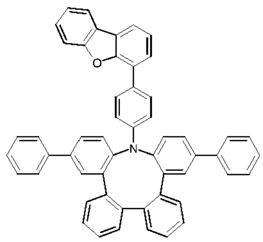 | 56 | C |
| 5d | 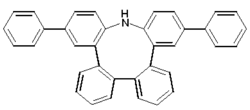 | 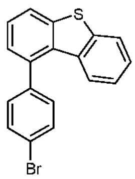 | 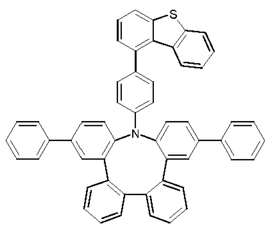 | 45 | A |
| 5e | 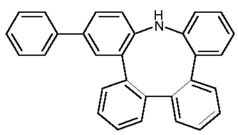 | 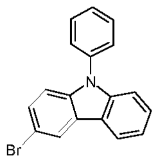 | 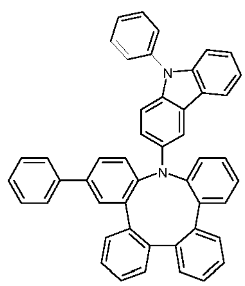 | 41 | B |
| 5f | 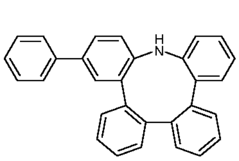 | 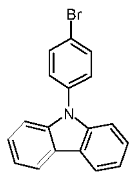 | 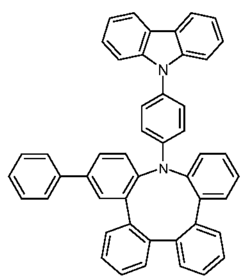 | 66 | B |

(fortgesetzt)

| Eintrag | Edukt 3 | Edukt 4 | Produkt 5 | Ausbeute [%] | Vari ante |
|---|---|---|---|---|---|
| 5g | 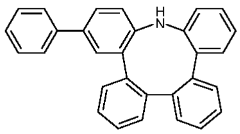 | 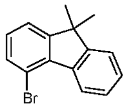 | 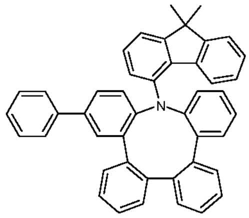 | 38 | B |
| 5h | 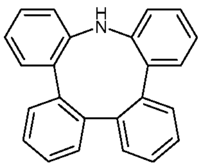 | 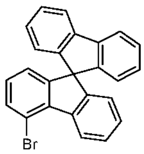 | 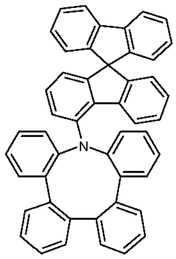 | 46 | B |
| 5i | 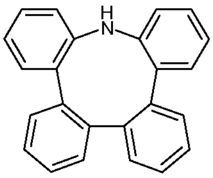 | 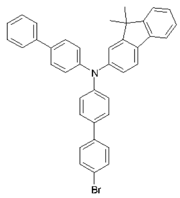 | 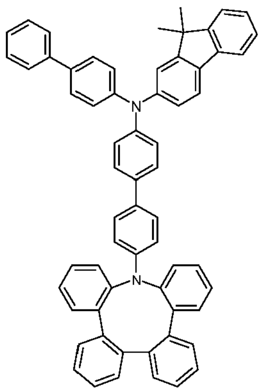 | 71 | A |
| 5j | 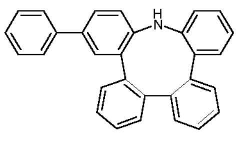 | 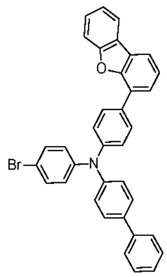 | 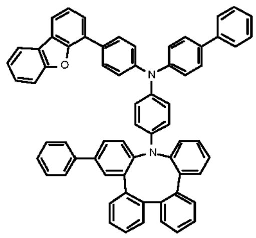 | 54 | B |
| 5k | 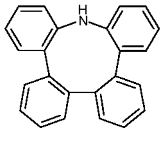 | 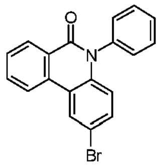 | 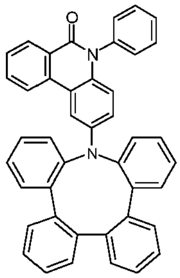 | 33 | B |

(fortgesetzt)

| Eintrag | Edukt 3 | Edukt 4 | Produkt 5 | Ausbeute [%] | Vari ante |
|---|---|---|---|---|---|
| 5l | 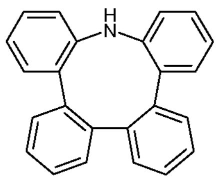 | 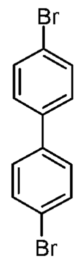 | 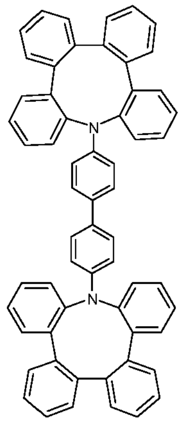 | 41 | A |
| 5m | 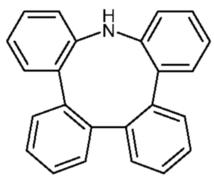 | 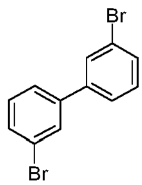 | 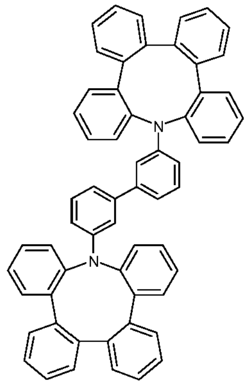 | 21 | B |
| 5n | 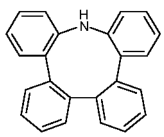 | 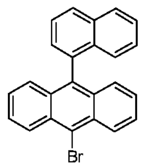 | 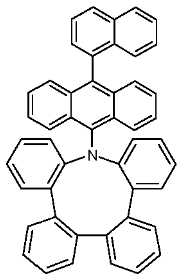 | 52 | C |
| 5o | 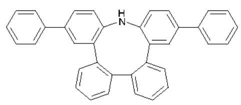 | 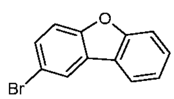 | 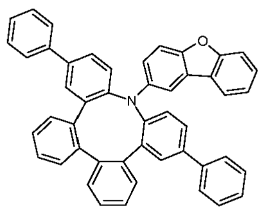 | 39 | A |
| 5p | 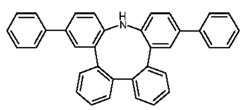 | 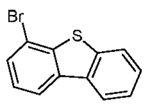 | 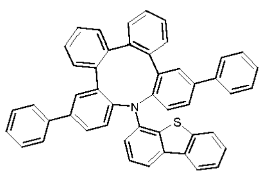 | 11 | C |

(fortgesetzt)

| Eintrag | Edukt 3 | Edukt 4 | Produkt 5 | Ausbeute [%] | Vari ante |
|---|---|---|---|---|---|
| 5q | 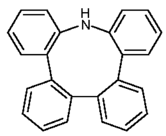 | 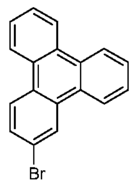 | 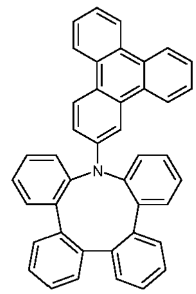 | 53 | B |
| 5r | 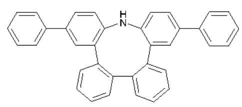 | 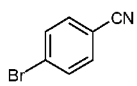 | 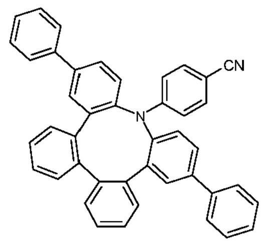 | 31 | B |
| 5s | 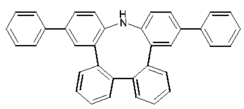 | 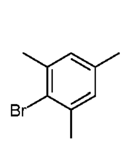 | 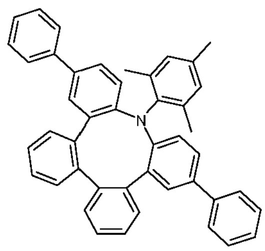 | 13 | B |

**Stufe 3:**

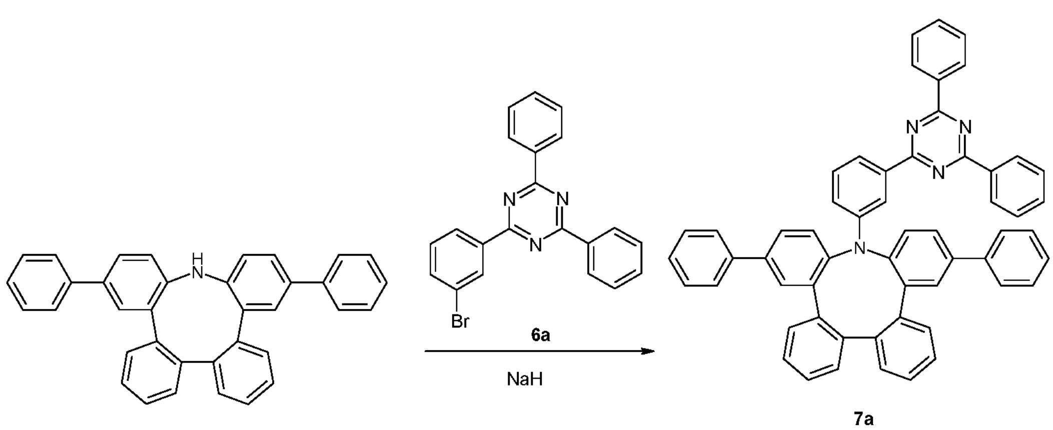


3a 7a

**Variante A:** Eine Lösung aus 8.0g (17mmol, 1.0eq) **3a** in 75ml getrocknetem DFM wird langsam zu einer Suspension aus 820mg (20.4mmol, 1.2eq) Natriumhydrid (60%ige Suspension in Mineralöl) in 50ml getrocknetem DMF zugetropft und für zwei Stunden gerührt. Anschließend werden 7.9g (20mmol, 1.2eq) 2-(3-Bromo-phenyl)-4,6-diphenyl-[1,3,5]triazin **6a** in 50ml getrocknetem THF zugetropft und das Gemisch über Nacht bei Raumtemperatur gerührt. Nach beendeter Reaktion wird der Ansatz auf 300ml einer 1:1 Wasser/Eis-Mischung gegeben und der entstandene Feststoff abgesaugt. Das Rohprodukt wird mittels Heißextraktion aus Toluol und dreifach Umkristallisation aus Heptan/Toluol aufgereinigt und zweimal sublimiert. 8.2g (10.5mmol, 62%) des gewünschten Produkts **7a** werden

mit einer HPLC-Reinheit von >99.9% erhalten.

**Variante B:** 5.0eq Cäsiumcarbonat in Dimethylacetamid

Analog werden hergestellt:

| Eintrag | Edukt 3 | Edukt 6 | Produkt 7 | Ausbeute [%] | Vari ante |
|---|---|---|---|---|---|
| 7b | 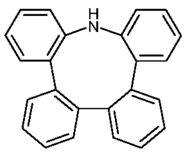 | 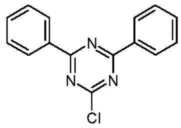 | 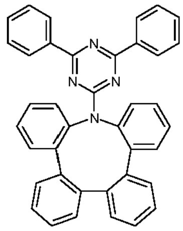 | 73 | A |
| 7c | 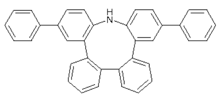 | 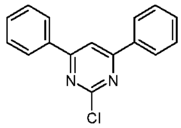 | 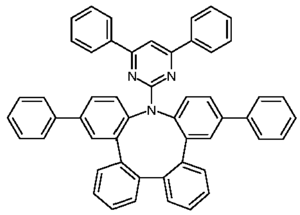 | 65 | A |
| 7d | 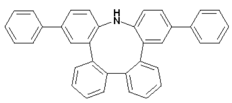 | 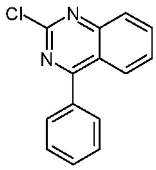 | 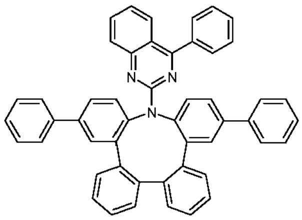 | 69 | A |
| 7e | 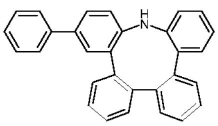 | 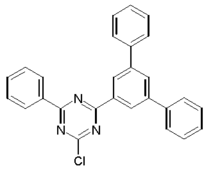 | 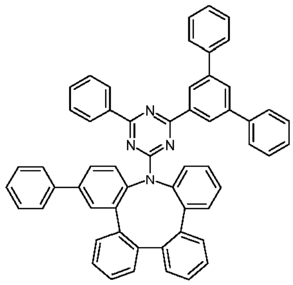 | 44 | B |
| 7f | 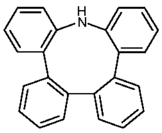 | 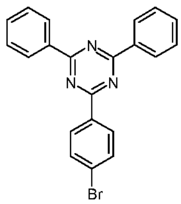 | 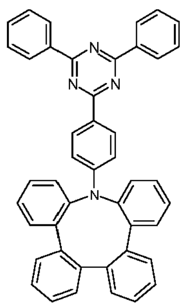 | 52 | B |

(fortgesetzt)

| Eintrag | Edukt 3 | Edukt 6 | Produkt 7 | Ausbeute [%] | Vari ante |
|---|---|---|---|---|---|
| 7g | 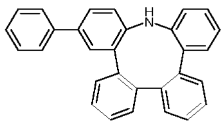 | 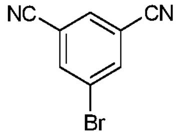 | 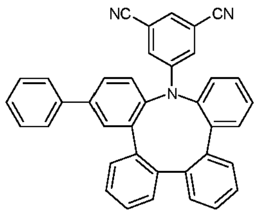 | 38 | A |

**Herstellung der OLEDs**

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, verwendete Materialien) angepasst wird.

In den folgenden Beispielen werden die Ergebnisse verschiedener OLEDs vorgestellt. Glasplättchen, mit strukturiertem ITO (50 nm, Indium-Zinn-Oxid) bilden die Substrate, auf welche die OLEDs aufgebracht werden. Die OLEDs haben prinzipiell folgenden Schichtaufbau: Substrat / Lochtransportschicht 1 (HTL1) bestehend aus HTM dotiert mit 5 % NDP-9 (kommerziell erhältlich von der Fa. Novaled), 20 nm / Lochtransportschicht 2 (HTL2) / optionale Elektronenblockierschicht (EBL) / Emissionsschicht (EML) / optionale Lochblockierschicht (HBL) / Elektronentransportschicht (ETL) und abschließend eine Kathode. Die Kathode wird durch eine 100 nm dicke Aluminiumschicht gebildet.

Zunächst werden vakuumprozessierte OLEDs beschrieben. Hierfür werden alle Materialien in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M3-M2:Ir(L1)$_3$ (55%:35%:10%) bedeutet hierbei, dass das Material M3 in einem Volumenanteil von 55%, M2 in einem Anteil von 35% und Ir(L1)$_3$ in einem Anteil von 10% in der Schicht vorliegt. Analog kann auch die Elektronentransportschicht aus einer Mischung zweier Materialien bestehen. Der genaue Aufbau der OLEDs ist Tabelle 1 zu entnehmen. Die zur Herstellung der OLEDs verwendeten Materialien sind in Tabelle 3 gezeigt.

Die OLEDs werden standardmäßig charakterisiert. Als Lebensdauer wird die Zeit definiert, nach der die Leuchtdichte von einer bestimmten Startleuchtdichte aus auf einen gewissen Anteil abgesunken ist. Die Angabe LD50 bedeutet, dass es sich bei der genannten Lebensdauer um die Zeit handelt, bei der die Leuchtdichte auf 50% der Startleuchtdichte abgefallen ist, also von z.B. 1000 cd/m$^2$ auf 500 cd/m$^2$. Je nach Emissionsfarbe werden unterschiedliche Starthelligkeiten gewählt. Die Werte für die Lebensdauer können mit Hilfe dem Fachmann bekannten Umrechnungsformeln auf eine Angabe für andere Startleuchtdichten umgerechnet werden. Hierbei ist die Lebensdauer für eine Startleuchtdichte von 1000 cd/m$^2$ eine übliche Angabe.

Die erfindungsgemäßen Verbindungen lassen sich unter anderem als Lochleiter (HTL) und als lochleitendes Matrixmaterial (h-TMM) in der Emissionsschicht in OLEDs einsetzen (s. Tabelle 1). Die Ergebnisse der OLEDs sind in Tabelle 2 zusammengefasst.

Tabelle 1: Aufbau der OLEDs

| Bsp. | HTL2 Dicke | EBL Dicke | EML Dicke | HBL Dicke | ETL Dicke |
|---|---|---|---|---|---|
| Rote OLEDs | | | | | |
| D-R1 | 5b 280 nm | --- | M5:M6: Ir-R1 (65%:30%:5%) 35 nm | --- | ETM1:ETM2 (50%:50%) 40 nm |
| D-R2 | HTM 280 nm | --- | M5:5c:Ir-R1 (60%:35%:5%) 35 nm | --- | ETM1:ETM2 (50%:50%) 40 nm |
| D-R3 | 5b 280 nm | --- | M5:5c:Ir-R1 (60%:35%:5%) 35 nm | --- | ETM1:ETM2 (50%:50%) 40 nm |
| D-R4 | 5l 280 nm | --- | 7d:Ir-R1 (60%:35%:5%) 35 nm | --- | ETM1:ETM2 (50%:50%) 40 nm |

(fortgesetzt)

| | | | | | |
|---|---|---|---|---|---|
| **Gelbe OLEDs** | | | | | |
| D-Y1 | 5b 250 nm | --- | M5:M6: Ir-Y1 (62%:30%:8%) 30 nm | --- | ETM1:ETM2 (50%:50%) 45 nm |
| D-Y2 | 5c 250 nm | --- | M5:5c:Ir-Y1 (60%:30%:10%) 30 nm | --- | ETM1:ETM2 (50%:50%) 45 nm |
| **Grüne OLEDs** | | | | | |
| D-G1 | HTM 220 nm | 5c 10 nm | M5:5c:Ir-G1 (60%:30%:10%) 30 nm | HBM2 10 nm | ETM1:ETM2 (50%:50%) 35 nm |
| D-G2 | 5c 220 nm | 5c 10 nm | M5:5c:Ir-G1 (60%:30%:10%) 30 nm | HBM2 10 nm | ETM1:ETM2 (50%:50%) 35 nm |
| D-G3 | HTM 220 nm | 5a 10 nm | M5:M6:Ir-G1 (45%:40%:15%) 30 nm | HBM2 10 nm | ETM1:ETM2 (50%:50%) 35 nm |
| D-G4 | HTM 220 nm | 5b 10 nm | M5:M6:Ir-G1 (45%:40%:15%) 30 nm | HBM2 10 nm | ETM1:ETM2 (50%:50%) 35 nm |
| D-G5 | HTM 220 nm | 5c 10 nm | M5:5e:Ir-G1 (55%:30%:15%) 30 nm | HBM2 10 nm | ETM1:ETM2 (50%:50%) 35 nm |
| D-G6 | HTM 220 nm | 5g 10 nm | M5:5f: Ir-G1 (65%:20%:15%) 30 nm | HBM2 10 nm | ETM1:ETM2 (50%:50%) 35 nm |
| D-G7 | HTM 220 nm | 5g 10 nm | M5:5k:Ir-G1 (45%:40%:15%) 30 nm | HBM2 10 nm | ETM1:ETM2 (50%:50%) 35 nm |
| D-G8 | 5i 220 nm | 5c 10 nm | M5:M6:Ir-G1 (55%:30%:15%) 30 nm | HBM2 10 nm | ETM1:ETM2 (50%:50%) 35 nm |
| D-G9 | HTM 220 nm | 5c 10 nm | M5:5q:Ir-G1 (55%:30%:15%) 30 nm | HBM2 10 nm | ETM1:ETM2 (50%:50%) 35 nm |
| D-G10 | HTM 220 nm | 5c 10 nm | 7f:Ir-G1 (80%:20%) 30 nm | HBM2 10 nm | ETM1:ETM2 (50%:50%) 35 nm |
| **Blaue OLEDs** | | | | | |
| D-B1 | 5b 190 nm | EBM 10 nm | M1:M4:Ir-B1 (60%:35%:5%) 25 nm | HBM1 10 nm | ETM1:ETM2 (50%:50%) 15 nm |
| D-B2 | 5c 190 nm | EBM 10 nm | M2:M4: Ir-B1 (60%:35%:5%) 25 nm | HBM1 10 nm | ETM1:ETM2 (50%:50%) 15 nm |
| D-B3 | 5m 190 nm | EBM 10 nm | M2:M4: Ir-B1 (60%:35%:5%) 25 nm | HBM1 10 nm | ETM1:ETM2 (50%:50%) 15 nm |
| D-B4 | 5l 190 nm | EBM 10 nm | M2:M4: Ir-B1 (60%:30%:10%) 25 nm | HBM1 10 nm | ETM1:ETM2 (50%:50%) 15 nm |

Tabelle 2: Erebnisse der Vakuum-prozessierten OLEDs

| Bsp. | EQE (%) 1000 cd/m$^2$ | Spannung (V) 1000 cd/m$^2$ | CIE x/y 1000 cd/m$^2$ | LD80 (h) 1000 cd/m$^2$ |
|---|---|---|---|---|
| **Rote OLEDs** | | | | |
| D-R1 | 15.3 | 3.0 | 0.67/0.33 | 15000 |
| D-R2 | 16.2 | 3.1 | 0.67/0.33 | 16000 |
| D-R3 | 15.9 | 3.0 | 0.67/0.33 | 14500 |
| D-R4 | 15.9 | 2.9 | 0.67/0.33 | 18000 |

(fortgesetzt)

| | | | | |
|---|---|---|---|---|
| **Gelbe OLEDs** | | | | |
| D-Y1 | 22.7 | 3.1 | 0.44/0.55 | 73000 |
| D-Y2 | 23.1 | 3.2 | 0.48/0.51 | 62000 |
| **Grüne OLEDs** | | | | |
| D-G1 | 20.6 | 3.3 | 0.32/0.64 | 33000 |
| D-G2 | 19.0 | 3.2 | 0.33/0.63 | 35000 |
| D-G3 | 19.5 | 3.2 | 0.32/0.64 | 41000 |
| D-G4 | 19.8 | 3.1 | 0.32/0.64 | 38000 |
| D-G5 | 20.2 | 3.3 | 0.33/0.63 | 35000 |
| D-G6 | 19.2 | 3.2 | 0.33/0.63 | 26000 |
| D-G7 | 19.6 | 3.2 | 0.33/0.63 | 36000 |
| D-G8 | 19.4 | 3.3 | 0.32/0.64 | 38000 |
| D-G9 | 20.0 | 3.2 | 0.32/0.64 | 40000 |
| D-G10 | 19.8 | 3.1 | 0.32/0.64 | 32000 |
| **Blaue OLEDs** | | | | |
| | | | | **LD50 (h) 1000 cd/m$^2$** |
| D-B1 | 22.3 | 4.4 | 0.16/0.33 | 900 |
| D-B2 | 17.8 | 4.5 | 0.15/0.34 | 1100 |
| D-B3 | 18.8 | 4.8 | 0.15/0.33 | 1000 |
| D-B4 | 19.4 | 4.4 | 0.15/0.33 | 1100 |

Tabelle 3: Strukturformeln der verwendeten Materialien

| | |
|---|---|
| 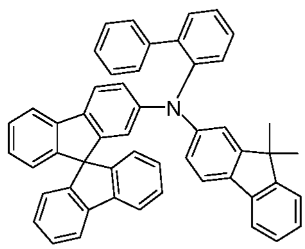 | 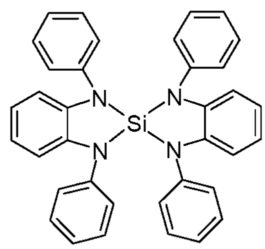 |
| HTM | EBM |
| 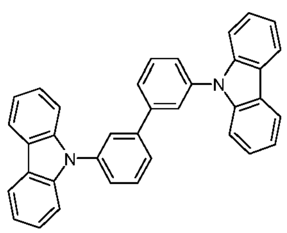 | 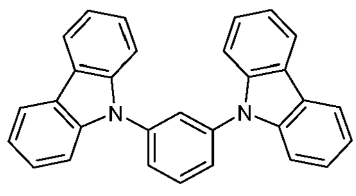 |
| M1 | M2 |

(fortgesetzt)

| | |
|---|---|
| 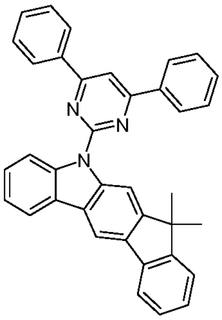 | 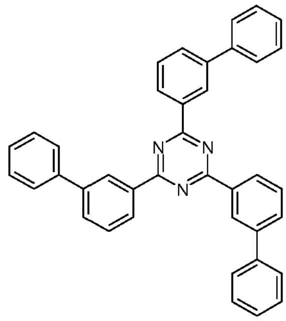 |
| HBM2 | M4 = HBM1 |
| 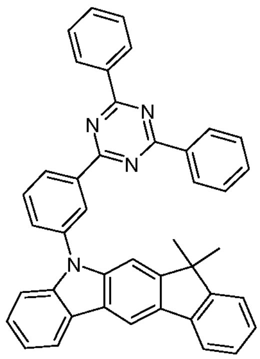 | 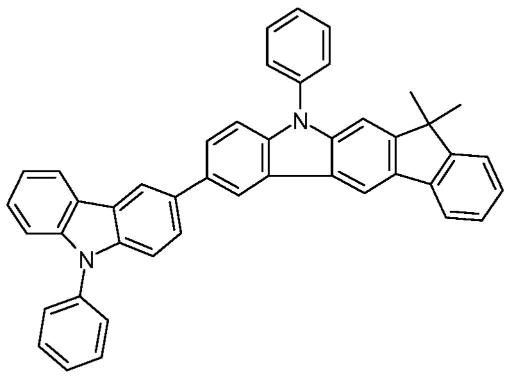 |
| M5 | M6 |
| 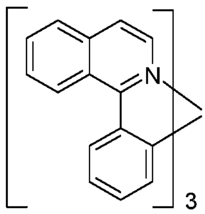 435293-93-9 | 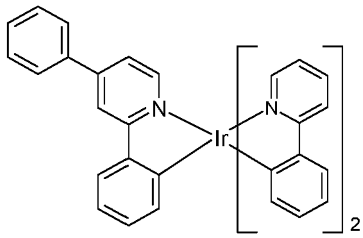 1215281-24-5 |
| Ir-R1 | Ir-Y1 |
| 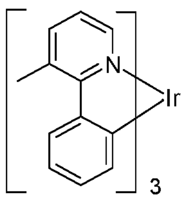 359014-71-4 | 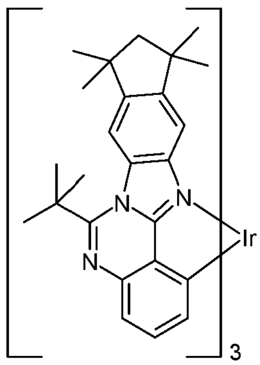 1541114-98-0 |
| Ir-G1 | Ir-B1 |

(fortgesetzt)

| | |
|---|---|
| 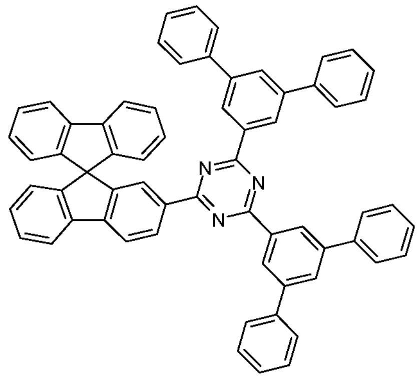 | 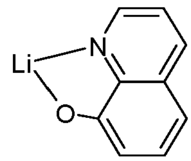 |
| ETM1 | ETM2 |

**Patentansprüche**

**1.** Verbindung, umfassend mindestens eine Struktur der Formel (I):

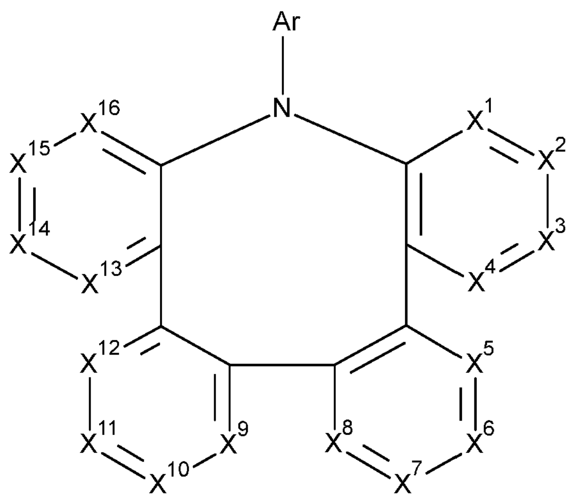

Formel (I)

wobei für die verwendeten Symbole gilt:

$X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ ist $CR^1$;
Ar ist bei jedem Auftreten gleich oder verschieden gewählt aus einer der Formeln ($R^1$-1) bis ($R^1$-95)

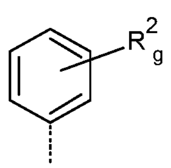

Formel ($R^1$-1)

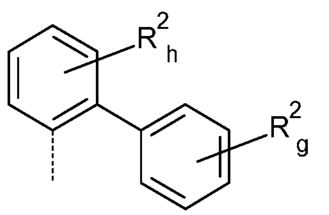

Formel ($R^1$-2)

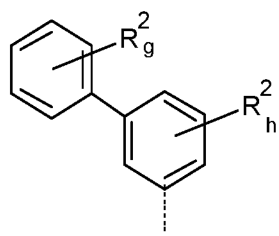

Formel ($R^1$-3)

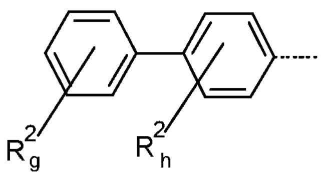

Formel ($R^1$-4)

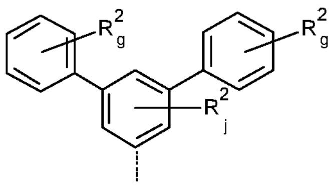

Formel ($R^1$-5)

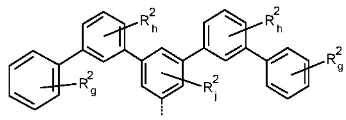

Formel ($R^1$-6)

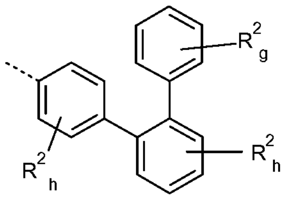

Formel ($R^1$-7)

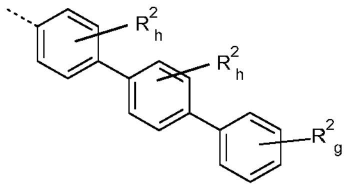

Formel ($R^1$-8)

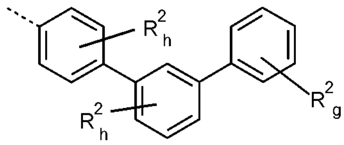

Formel ($R^1$-9)

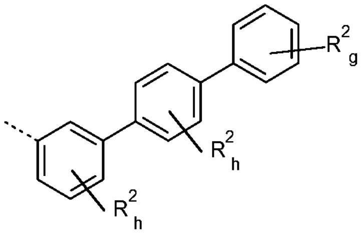

Formel ($R^1$-10)

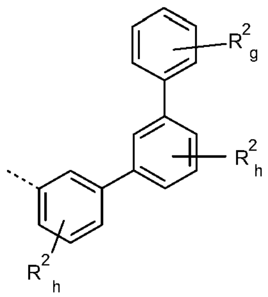

Formel ($R^1$-11)

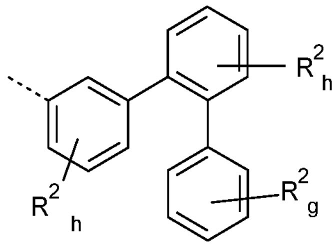

Formel ($R^1$-12)

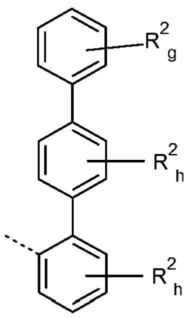

Formel ($R^1$-13)

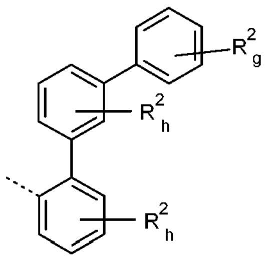

Formel ($R^1$-14)

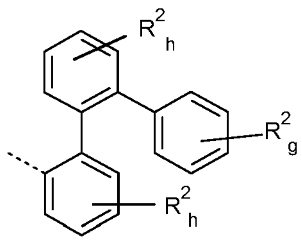

Formel ($R^1$-15)

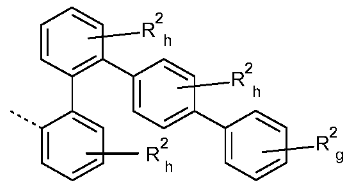

Formel ($R^1$-16)

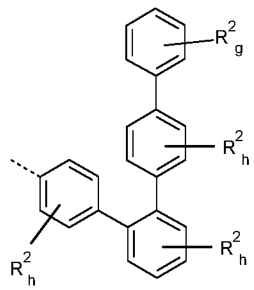

Formel ($R^1$-17)

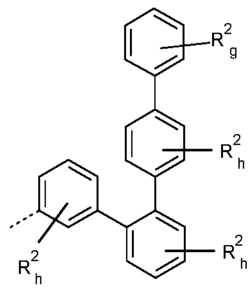

Formel ($R^1$-18)

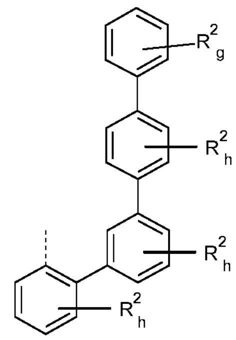

Formel ($R^1$-19)

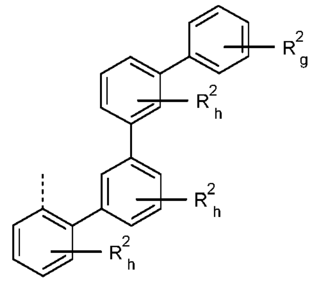

Formel ($R^1$-20)

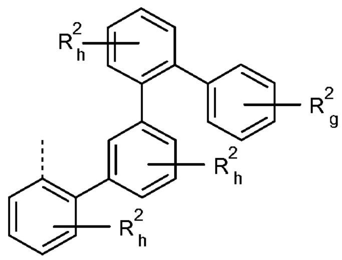

Formel ($R^1$-21)

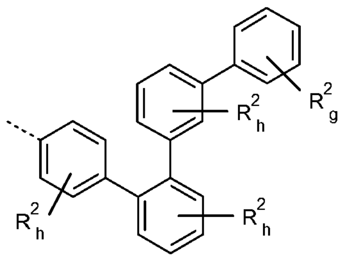

Formel ($R^1$-22)

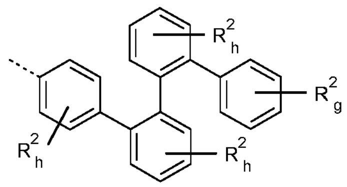

Formel ($R^1$-23)

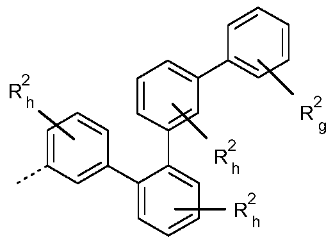

Formel ($R^1$-24)

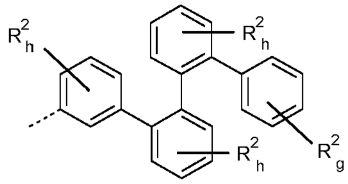

Formel ($R^1$-25)

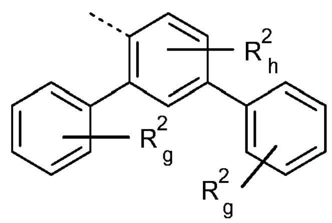

Formel ($R^1$-26)

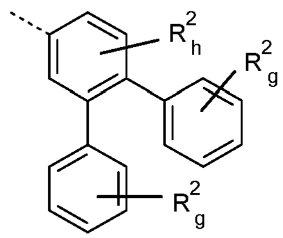

Formel ($R^1$-27)

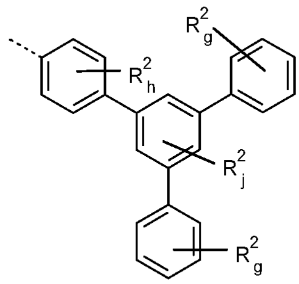

Formel ($R^1$-28)

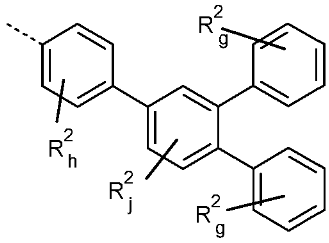

Formel ($R^1$-29)

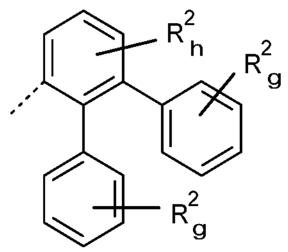

Formel ($R^1$-30)

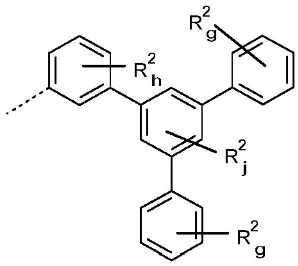

Formel ($R^1$-31)

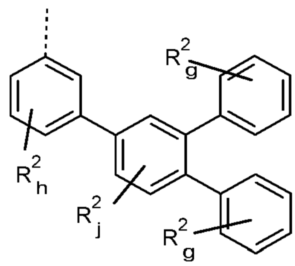

Formel ($R^1$-32)

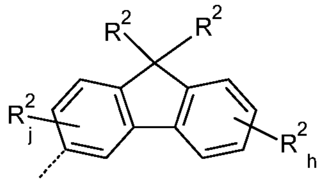

Formel ($R^1$-33)

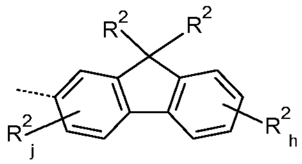

Formel ($R^1$-34)

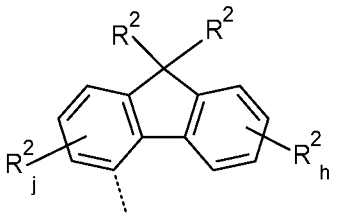

Formel ($R^1$-35)

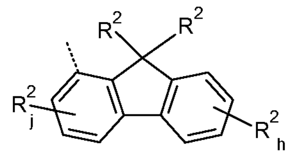

Formel ($R^1$-36)

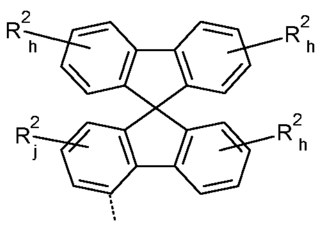

Formel ($R^1$-37)

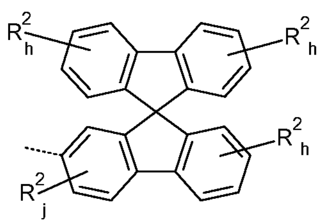

Formel ($R^1$-38)

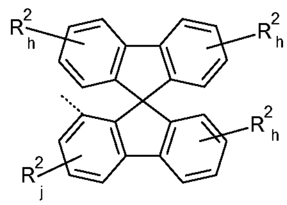

Formel ($R^1$-39)

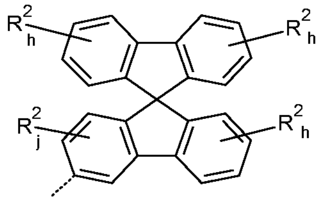

Formel ($R^1$-40)

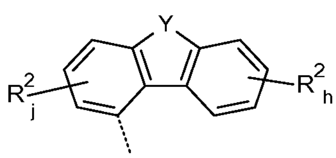

Formel ($R^1$-41)

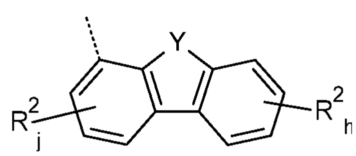

Formel ($R^1$-42)

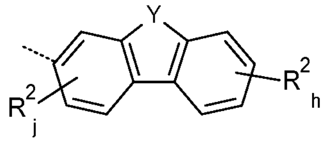
Formel ($R^1$-43)

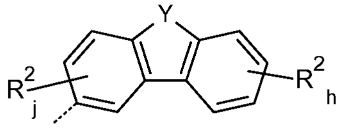
Formel ($R^1$-44)

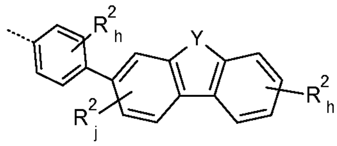
Formel ($R^1$-45)

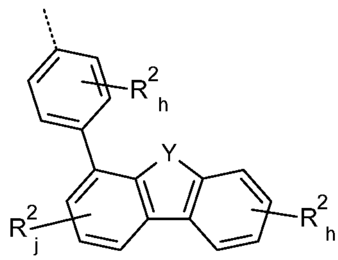
Formel ($R^1$-46)

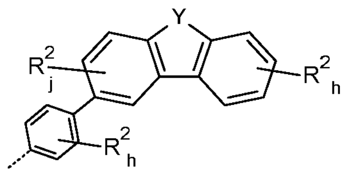
Formel ($R^1$-47)

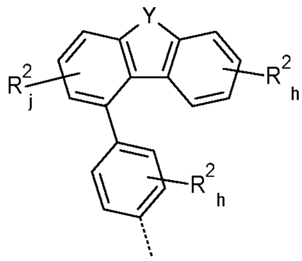
Formel ($R^1$-48)

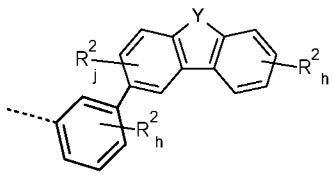
Formel ($R^1$-49)

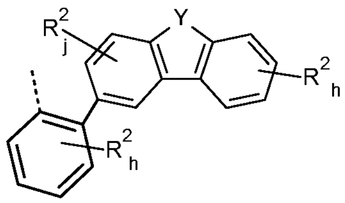
Formel ($R^1$-50)

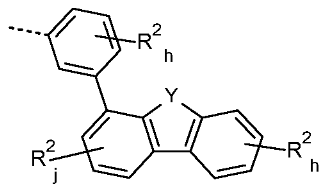
Formel ($R^1$-51)

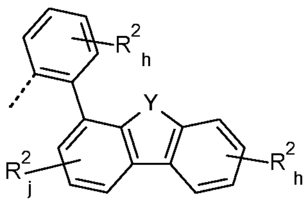
Formel ($R^1$-52)

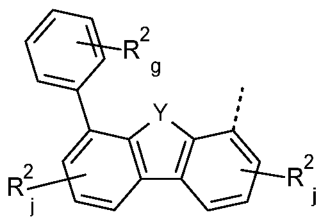
Formel ($R^1$-53)

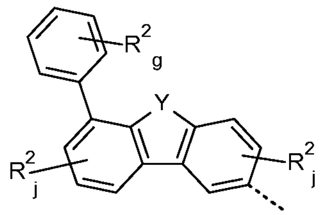
Formel ($R^1$-54)

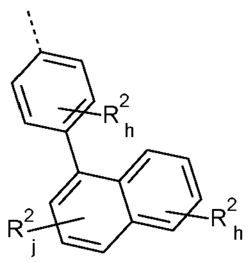
Formel ($R^1$-55)

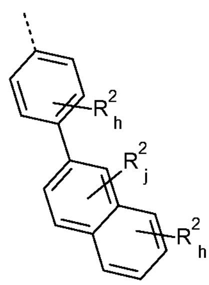
Formel ($R^1$-56)

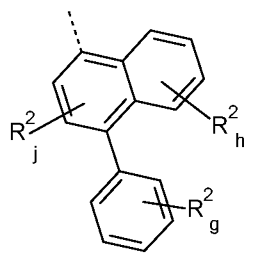
Formel ($R^1$-57)

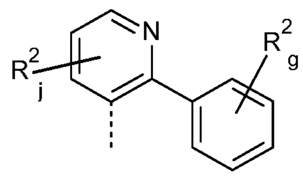

Formel ($R^1$-58)

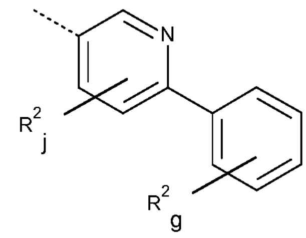

Formel ($R^1$-59)

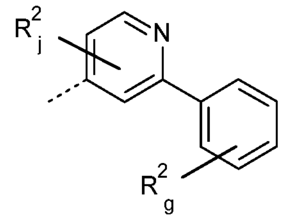

Formel ($R^1$-60)

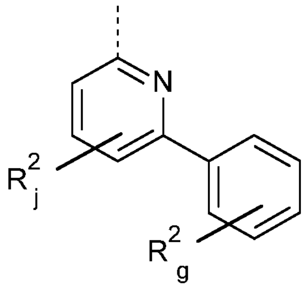

Formel ($R^1$-61)

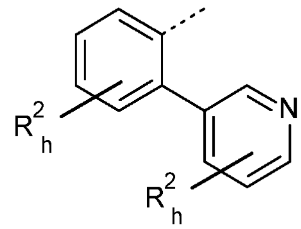

Formel ($R^1$-62)

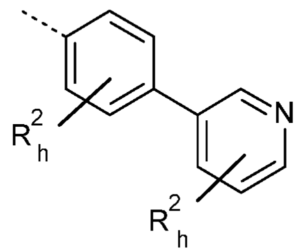

Formel ($R^1$-63)

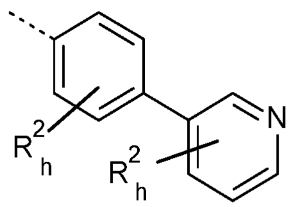

Formel ($R^1$-64)

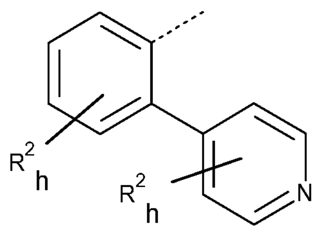

Formel ($R^1$-65)

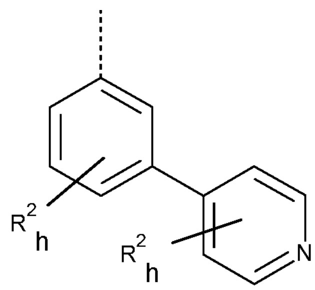

Formel ($R^1$-66)

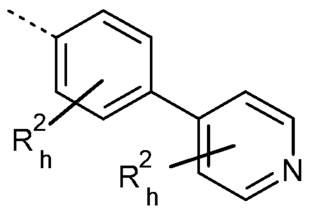

Formel ($R^1$-67)

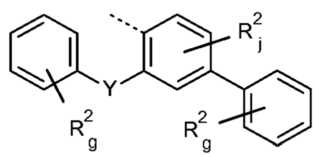

Formel ($R^1$-68)

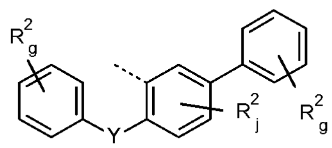

Formel ($R^1$-69)

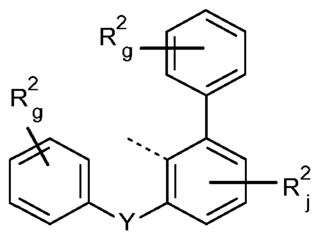

Formel ($R^1$-70)

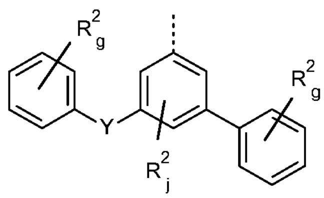

Formel ($R^1$-71)

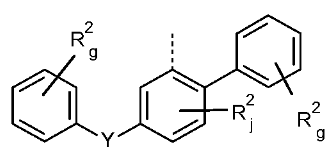

Formel ($R^1$-72)

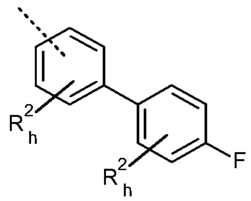
Formel ($R^1$-73)

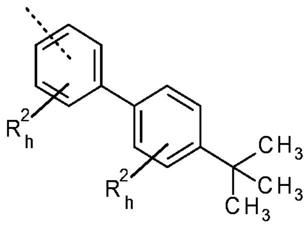
Formel ($R^1$-74)

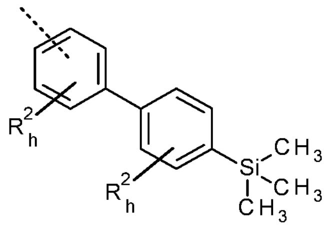
Formel ($R^1$-75)

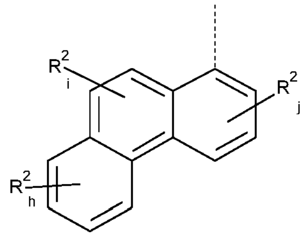
Formel ($R^1$-76)

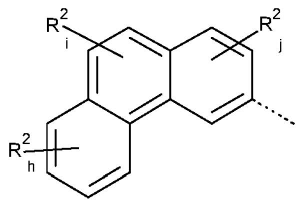
Formel ($R^1$-77)

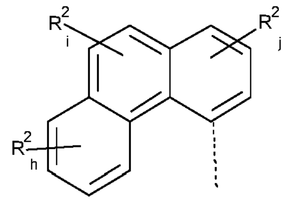
Formel ($R^1$-78)

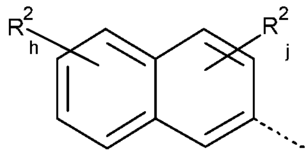
Formel ($R^1$-79)

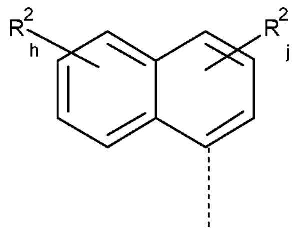
Formel ($R^1$-80)

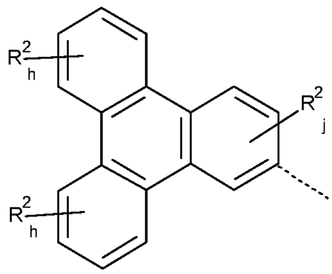
Formel ($R^1$-81)

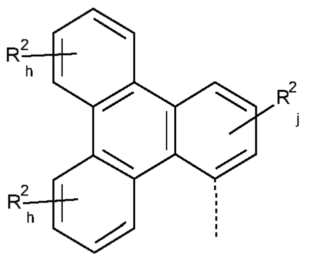
Formel ($R^1$-82)

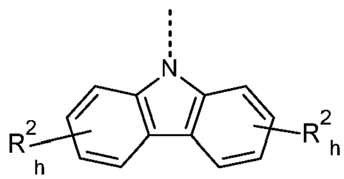
Formel ($R^1$-83)

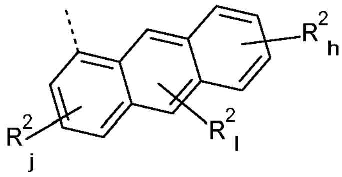
Formel ($R^1$-84)

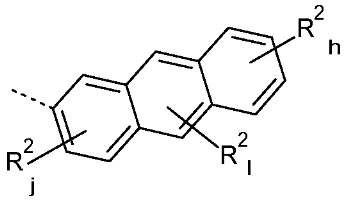
Formel ($R^1$-85)

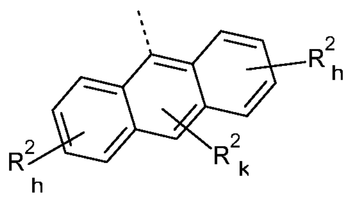
Formel ($R^1$-86)

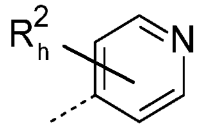
Formel ($R^1$-87)

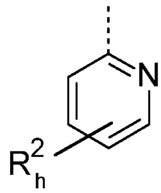
Formel ($R^1$-88)

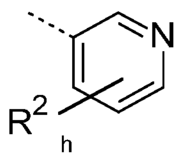
Formel ($R^1$-89)

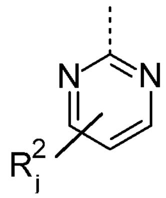
Formel ($R^1$-90)

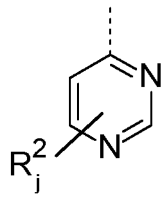
Formel ($R^1$-91)

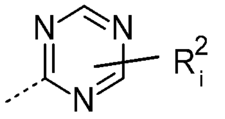
Formel ($R^1$-92)

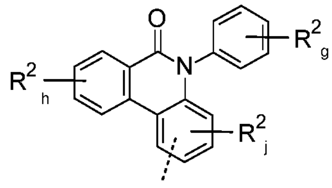
Formel ($R^1$-93)

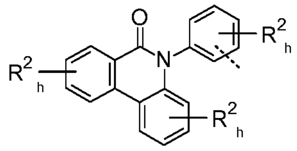
Formel ($R^1$-94)

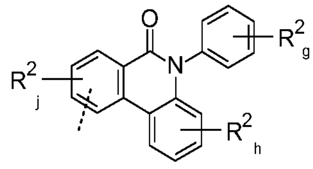
Formel ($R^1$-95)

wobei für die verwendeten Symbole gilt:

Y ist O, S oder $NR^2$, vorzugsweise O oder S;
i ist bei jedem Auftreten unabhängig 0, 1 oder 2;
j ist bei jedem Auftreten unabhängig 0, 1, 2 oder 3;
h ist bei jedem Auftreten unabhängig 0, 1, 2, 3 oder 4;
g ist bei jedem Auftreten unabhängig 0, 1, 2, 3, 4 oder 5; und

die gestrichelte Bindung markiert die Anbindungsposition;

$R^1$ ist bei jedem Auftreten gleich oder verschieden ausgewählt aus der Gruppe bestehend aus H, D, F, CN, $N(Ar^1)_2$, einer geradkettigen Alkylgruppe mit 1 bis 8 C-Atomen, oder einer verzweigten oder cyclischen Alkylgruppe mit 3 bis 8 C-Atomen, oder einer Alkenylgruppe mit 2 bis 8 C-Atomen, die jeweils mit einem oder mehreren Resten $R^2$ substituiert sein kann, oder einem aromatischen oder heteroaromatischen Ringsystem mit 6 bis 24 aromatischen Ringatomen, das jeweils mit einem oder mehreren nicht-aromatischen Resten $R^1$ substituiert sein kann;
$Ar^1$ ist gleich oder verschieden bei jedem Auftreten ein aromatisches oder heteroaromatisches Ringsystem mit 5 bis 40 aromatischen Ringatomen, das jeweils mit einem oder mehreren Resten $R^2$ substituiert sein kann;
$R^2$ ist bei jedem Auftreten gleich oder verschieden ausgewählt aus der Gruppe bestehend aus H, D, einem aliphatischen Kohlenwasserstoffrest mit 1 bis 10 C-Atomen, oder einem aromatischen oder heteroaromatischen Ringsystem mit 5 bis 30 aromatischen Ringatomen, das durch ein oder mehrere Alkylgruppen mit jeweils 1 bis 4 Kohlenstoffatomen substituiert sein kann;

wobei in Formel (I) höchstens 2 der Symbole $X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ ungleich CH oder CD sind.

**2.** Oligomer, Polymer oder Dendrimer enthaltend eine oder mehrere Verbindungen nach Anspruch 1, wobei statt eines Wasserstoffatoms oder eines Substituenten ein oder mehrere Bindungen der Verbindungen zum Polymer, Oligomer oder Dendrimer vorhanden sind.

3. Zusammensetzung enthaltend wenigstens eine Verbindung nach Anspruch 1 oder ein Oligomer, Polymer oder Dendrimer nach Anspruch 2 und wenigstens eine weitere Verbindung ausgewählt aus der Gruppe bestehend aus fluoreszierenden Emittern, phosphoreszierenden Emittern, Host-Materialien, Matrix-Materialien, Elektronentransportmaterialien, Elektroneninjektionsmaterialien, Lochleitermaterialien, Lochinjektionsmaterialien, Elektronenblockiermaterialien und Lochblockiermaterialien.

4. Formulierung, enthaltend mindestens eine Verbindung nach Anspruch 1 oder ein Oligomer, Polymer oder Dendrimer nach Anspruch 2 oder eine Zusammensetzung nach Anspruch 3 und mindestens ein Lösemittel.

5. Verwendung einer Verbindung nach Anspruch 1, eines Oligomers, Polymers oder Dendrimers nach Anspruch 2 oder einer Zusammensetzung nach Anspruch 3 in einer elektronischen Vorrichtung als fluoreszierender Emitter, Hostmaterial für phosphoreszierende Emitter, Elektronentransportmaterial oder Lochtransportmaterial, bevorzugt als Hostmaterial für phosphoreszierende Emitter oder als Lochtransportmaterial.

6. Elektronische Vorrichtung, enthaltend mindestens eine Verbindung Anspruch 1, ein Oligomer, Polymer oder Dendrimer nach Anspruch 2 oder eine Zusammensetzung gemäß Anspruch 3, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, lichtemittierenden elektrochemischen Zellen oder organischen Laserdioden.

## Claims

1. Compound comprising at least one structure of the formula (I):

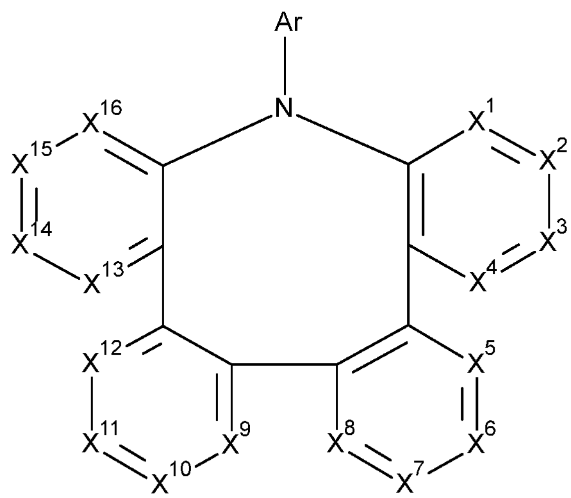

formula (I)

where the following applies to the symbols used:

$X^1$, $X^2$, $X^3$, $X^4$, $X^5$, $X^6$, $X^7$, $X^8$, $X^9$ , $X^{10}$, $X^{11}$, $X^{12}$, $X^{13}$, $X^{14}$, $X^{15}$, $X^{16}$ are $CR^1$;
Ar is selected on each occurrence, identically or differently, from one of the formulae ($R^1$-1) to ($R^1$-95)

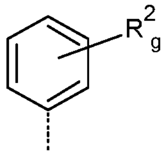

formula ($R^1$-1)

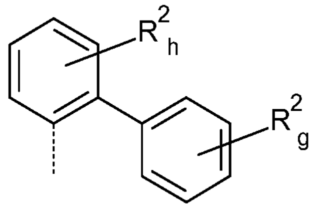

formula ($R^1$-2)

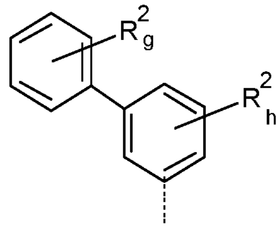

formula ($R^1$-3)

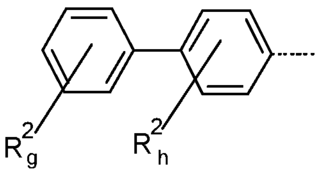

formula ($R^1$-4)

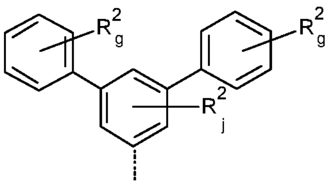

formula ($R^1$-5)

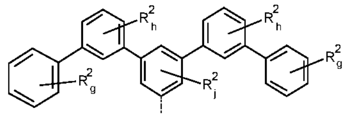

formula ($R^1$-6)

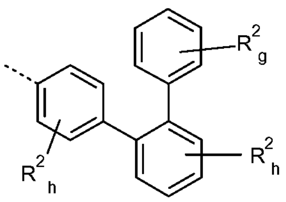

formula ($R^1$-7)

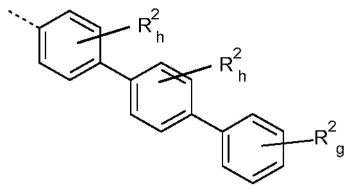

formula ($R^1$-8)

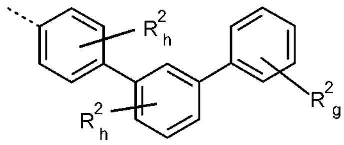

formula ($R^1$-9)

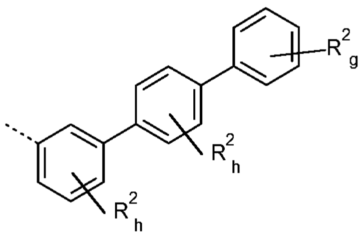

formula ($R^1$-10)

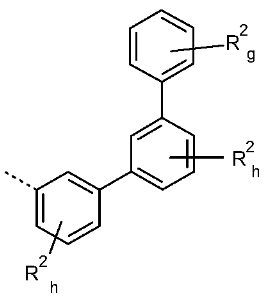

formula ($R^1$-11)

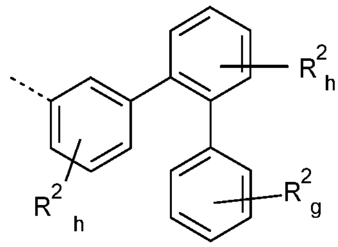

formula ($R^1$-12)

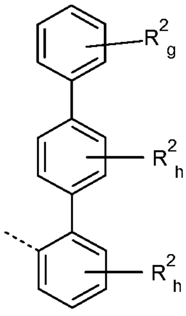

formula ($R^1$-13)

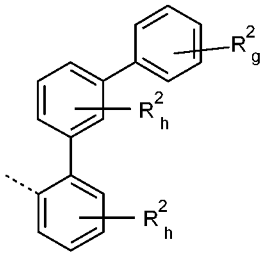

formula ($R^1$-14)

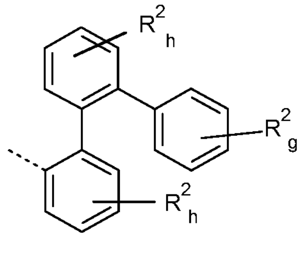

formula ($R^1$-15)

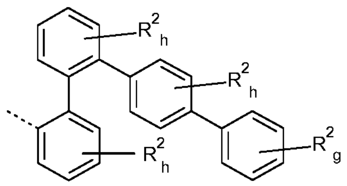

formula ($R^1$-16)

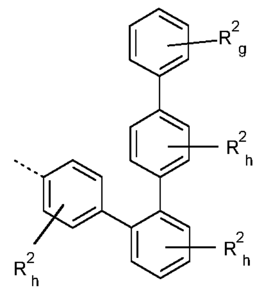

formula ($R^1$-17)

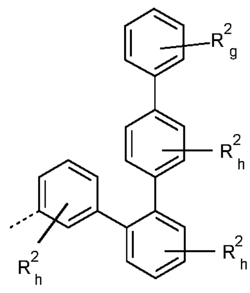

formula ($R^1$-18)

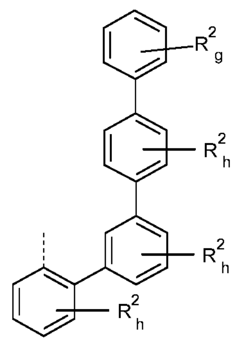

formula ($R^1$-19)

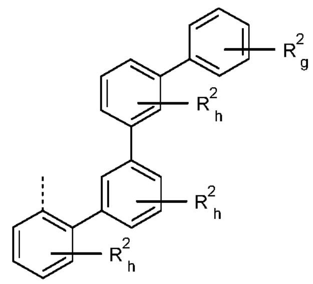

formula ($R^1$-20)

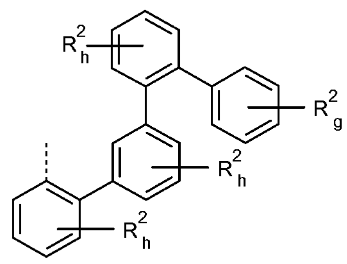

formula ($R^1$-21)

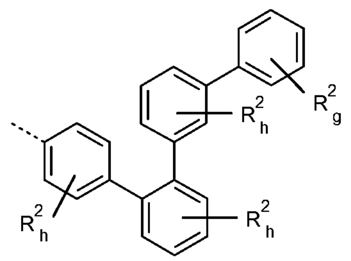

formula ($R^1$-22)

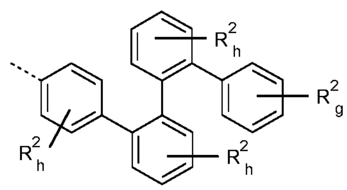

formula ($R^1$-23)

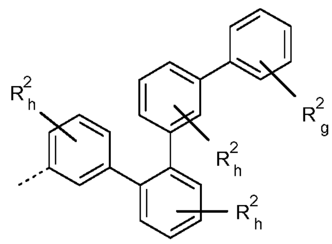

formula ($R^1$-24)

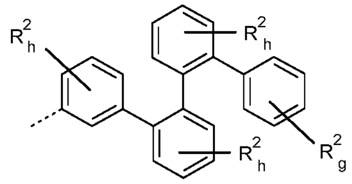

formula ($R^1$-25)

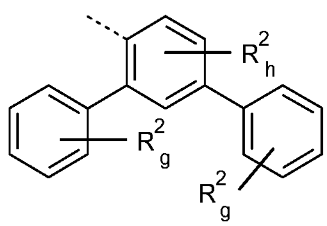

formula ($R^1$-26)

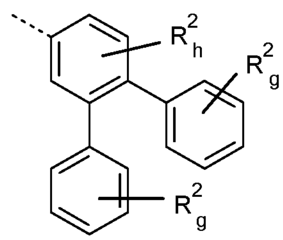

formula ($R^1$-27)

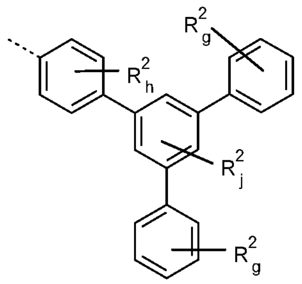

formula ($R^1$-28)

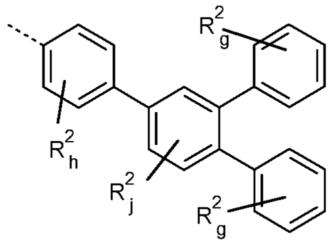

formula ($R^1$-29)

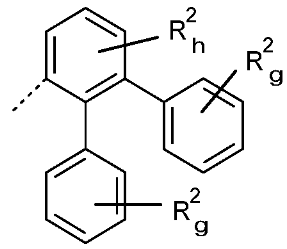

formula ($R^1$-30)

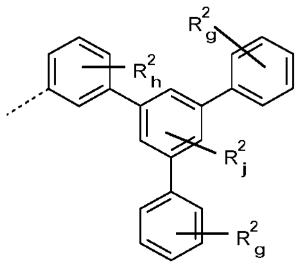

formula ($R^1$-31)

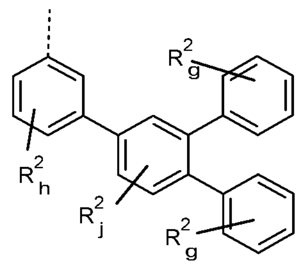

formula ($R^1$-32)

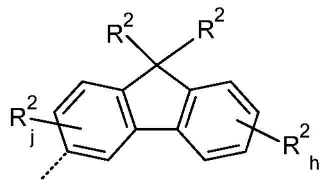

formula ($R^1$-33)

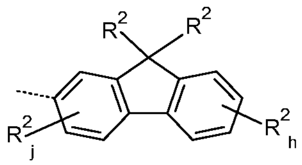

formula ($R^1$-34)

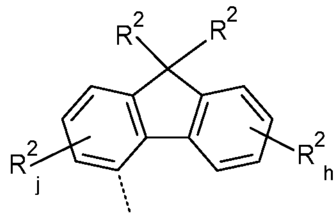

formula ($R^1$-35)

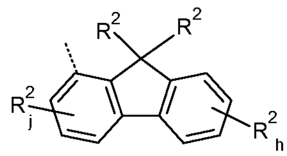

formula ($R^1$-36)

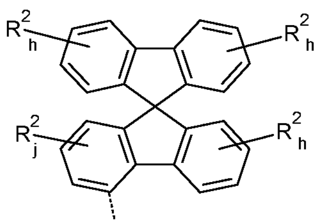

formula ($R^1$-37)

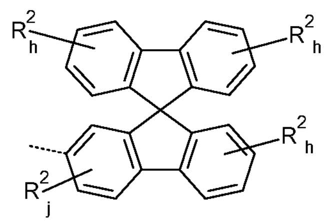

formula ($R^1$-38)

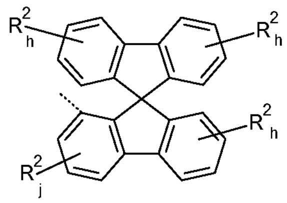

formula ($R^1$-39)

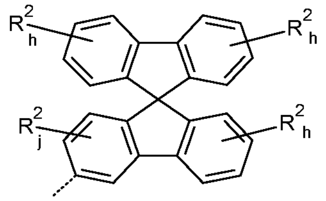

formula ($R^1$-40)

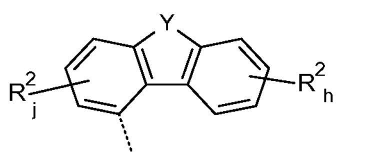

formula ($R^1$-41)

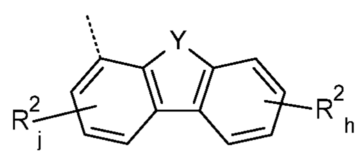

formula ($R^1$-42)

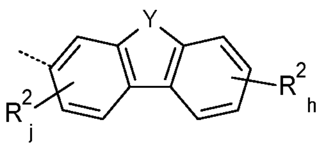

formula ($R^1$-43)

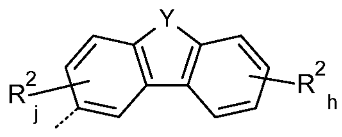

formula ($R^1$-44)

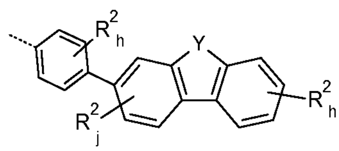

formula ($R^1$-45)

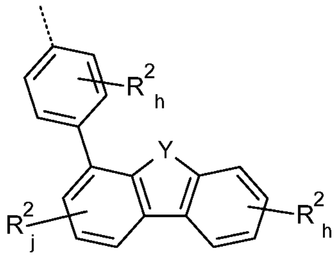

formula ($R^1$-46)

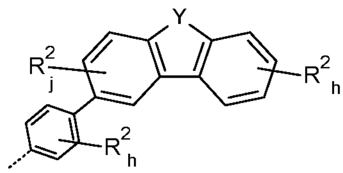

formula ($R^1$-47)

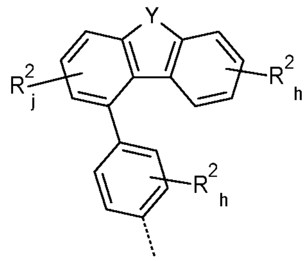

formula ($R^1$-48)

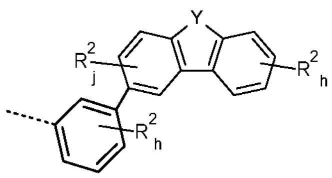

formula ($R^1$-49)

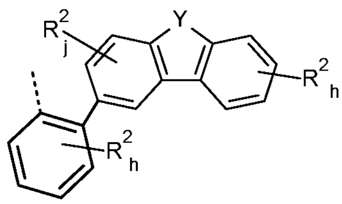

formula ($R^1$-50)

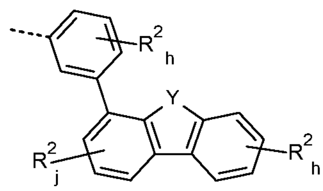

formula ($R^1$-51)

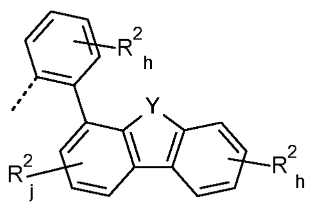

formula ($R^1$-52)

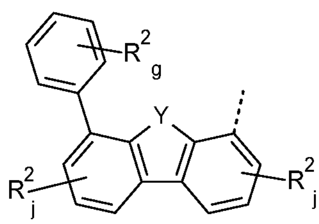

formula ($R^1$-53)

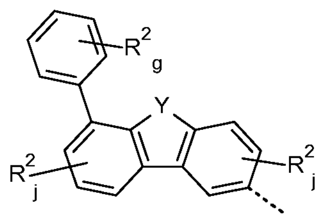

formula ($R^1$-54)

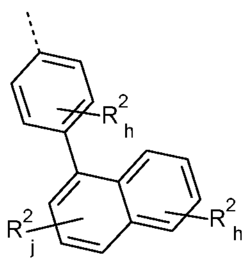

formula ($R^1$-55)

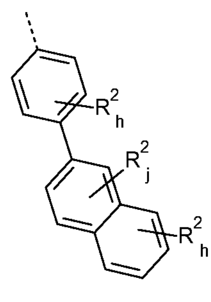

formula ($R^1$-56)

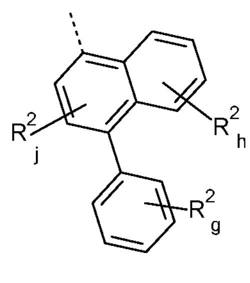

formula ($R^1$-57)

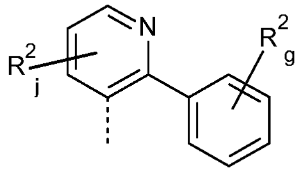

formula ($R^1$-58)

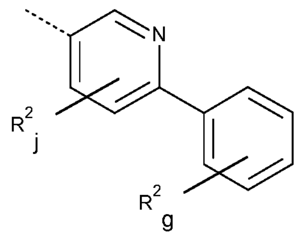

formula ($R^1$-59)

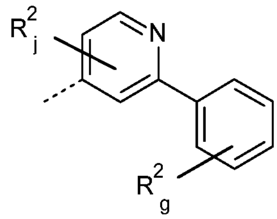

formula ($R^1$-60)

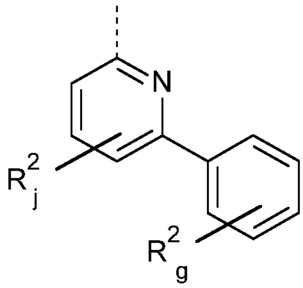

formula ($R^1$-61)

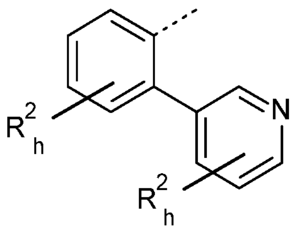

formula ($R^1$-62)

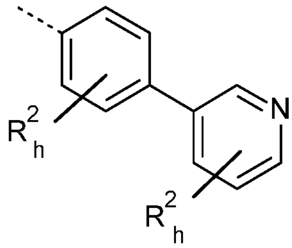

formula ($R^1$-63)

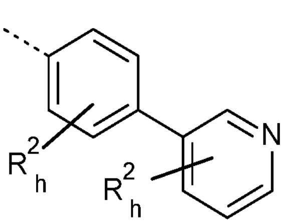

formula ($R^1$-64)

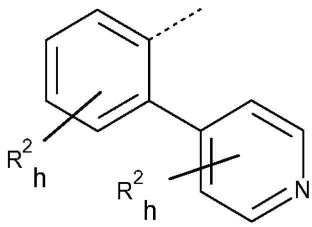

formula ($R^1$-65)

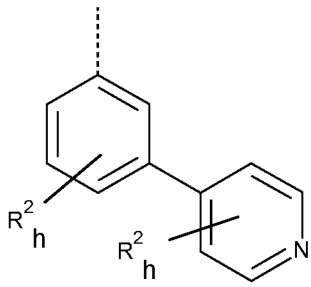

formula ($R^1$-66)

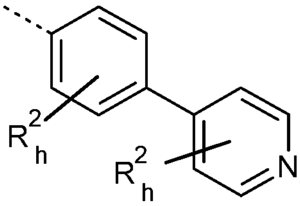

formula ($R^1$-67)

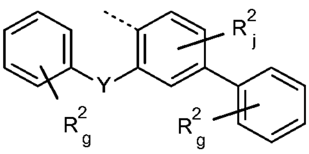

formula ($R^1$-68)

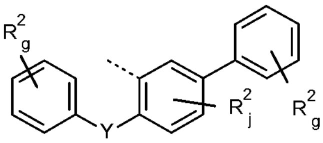

formula ($R^1$-69)

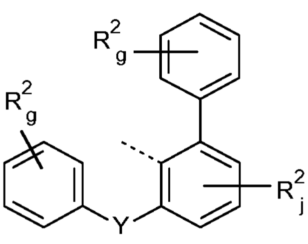

formula ($R^1$-70)

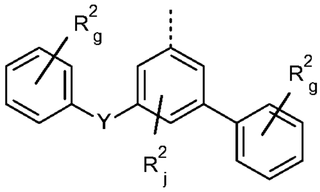

formula ($R^1$-71)

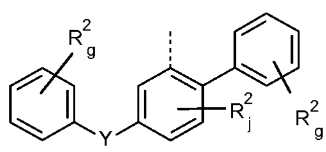

formula ($R^1$-72)

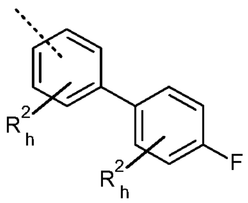

formula ($R^1$-73)

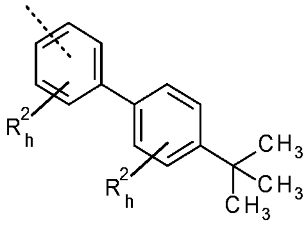

formula ($R^1$-74)

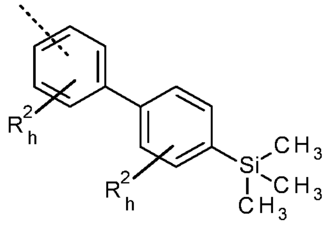

formula ($R^1$-75)

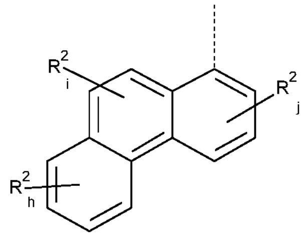

formula ($R^1$-76)

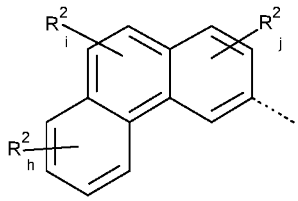

formula ($R^1$-77)

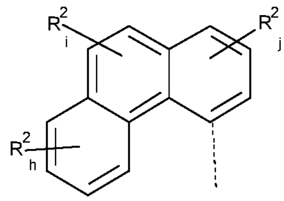

formula ($R^1$-78)

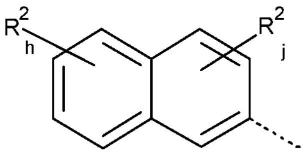

formula ($R^1$-79)

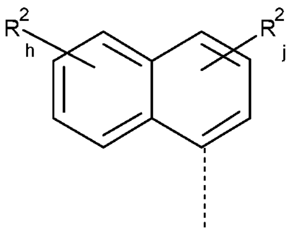

formula ($R^1$-80)

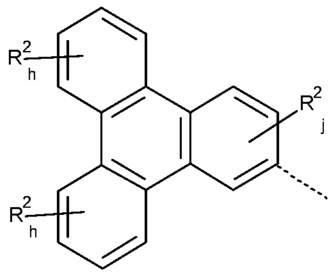

formula ($R^1$-81)

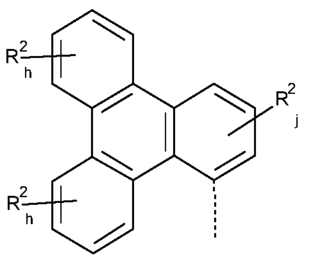

formula ($R^1$-82)

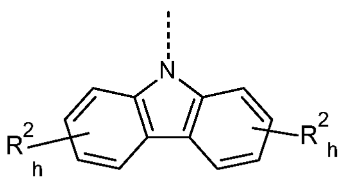

formula ($R^1$-83)

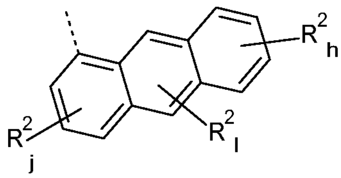

formula ($R^1$-84)

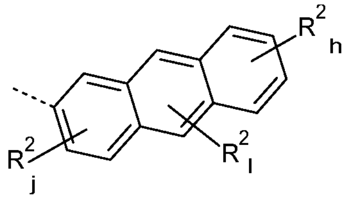

formula ($R^1$-85)

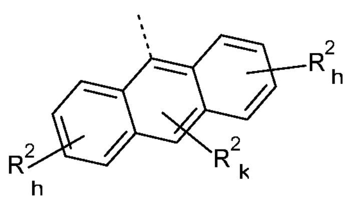

formula ($R^1$-86)

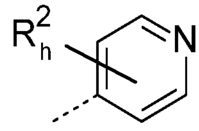

formula ($R^1$-87)

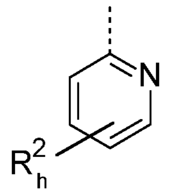

formula ($R^1$-88)

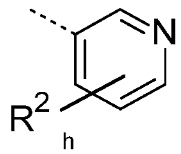

formula ($R^1$-89)

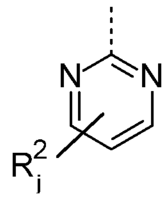

formula ($R^1$-90)

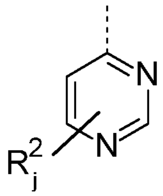

formula ($R^1$-91)

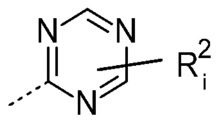

formula ($R^1$-92)

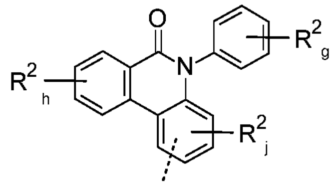

formula ($R^1$-93)

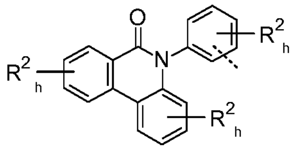

formula ($R^1$-94)

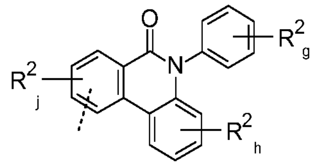

formula ($R^1$-95)

where the following applies to the symbols used:

Y is O, S or $NR^2$, preferably O or S;
i is on each occurrence, independently, 0, 1 or 2;
j is on each occurrence, independently, 0, 1, 2 or 3;
h is on each occurrence, independently, 0, 1, 2, 3 or 4;
g is on each occurrence, independently, 0, 1, 2, 3, 4 or 5; and

the dashed bond marks the bonding position;

$R^1$ is selected on each occurrence, identically or differently, from the group consisting of H, D, F, CN, $N(Ar^1)_2$, a straight-chain alkyl group having 1 to 8 C atoms, or a branched or cyclic alkyl group having 3 to 8 C atoms, or an alkenyl group having 2 to 8 C atoms, which may in each case be substituted by one or more radicals $R^2$, or an aromatic or heteroaromatic ring system having 6 to 24 aromatic ring atoms, which may in each case be substituted by one or more non-aromatic radicals $R^1$;
$Ar^1$ is, identically or differently on each occurrence, an aromatic or heteroaromatic ring system having 5 to 40 aromatic ring atoms, which may in each case be substituted by one or more radicals $R^2$;
$R^2$ is selected on each occurrence, identically or differently, from the group consisting of H, D, an aliphatic hydrocarbon radical having 1 to 10 C atoms, or an aromatic or heteroaromatic ring system having 5 to 30 aromatic ring atoms, which may be substituted by one or more alkyl groups having in each case 1 to 4 carbon atoms;

where in formula (I) at most 2 of the symbols $X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ are not equal to CH or CD.

**2.** Oligomer, polymer or dendrimer containing one or more compounds according to Claim 1, in which, instead of a hydrogen atom or a substituent, one or more bonds are present from the compounds to the polymer, oligomer or dendrimer.

**3.** Composition comprising at least one compound according to Claim 1 or an oligomer, polymer or dendrimer according

to Claim 2 and at least one further compound selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

**4.** Formulation comprising at least one compound according to Claim 1 or an oligomer, polymer or dendrimer according to Claim 2 or a composition according to Claim 3 and at least one solvent.

**5.** Use of a compound according to Claim 1, an oligomer, polymer or dendrimer according to Claim 2 or a composition according to Claim 3 in an electronic device as fluorescent emitter, host material for phosphorescent emitters, electron-transport material or hole-transport material, preferably as host material for phosphorescent emitters or as hole-transport material.

**6.** Electronic device comprising at least one compound according to Claim 1, an oligomer, polymer or dendrimer according to Claim 2 or a composition according to Claim 3, where the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field-quench devices, light-emitting electrochemical cells or organic laser diodes.

## Revendications

**1.** Composé comprenant au moins une structure de formule (I) :

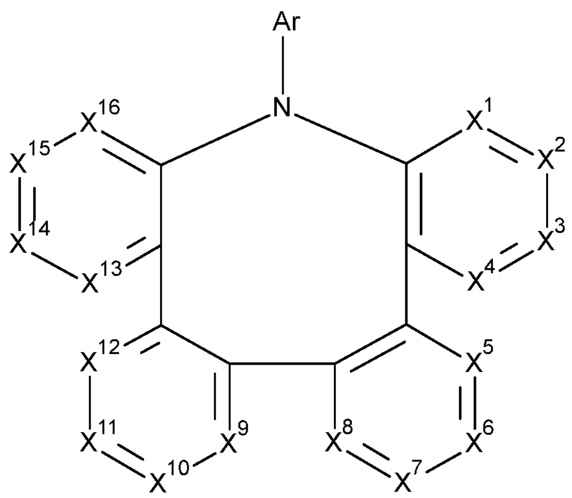

formule (I)

dans laquelle ce qui suit s'applique aux symboles utilisés :

$X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ sont $CR^1$ ;
Ar est choisi à chaque occurrence, de manière identique ou différente, parmi l'une des formules ($R^1$-1) à ($R^1$-95)

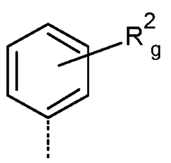

formule ($R^1$-1)

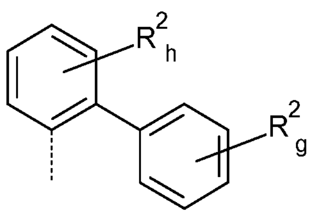

formule ($R^1$-2)

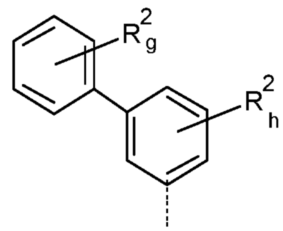

formule ($R^1$-3)

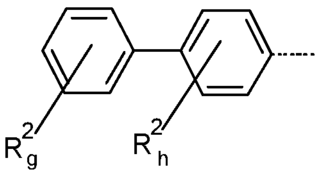

formule ($R^1$-4)

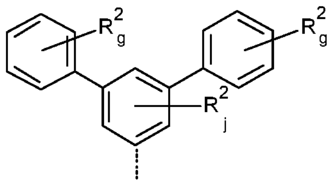

formule ($R^1$-5)

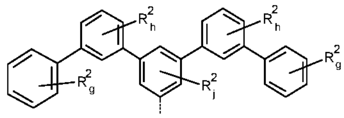

formule ($R^1$-6)

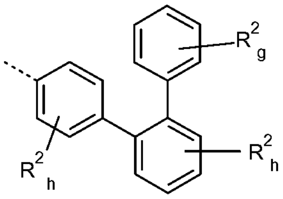

formule ($R^1$-7)

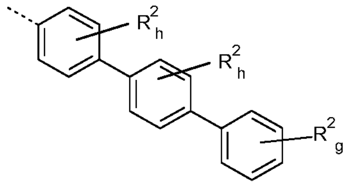

formule ($R^1$-8)

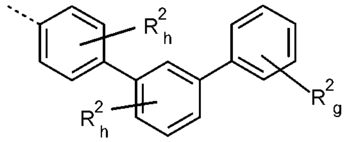

formule ($R^1$-9)

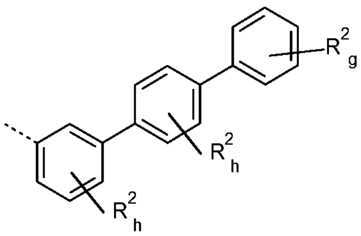

formule ($R^1$-10)

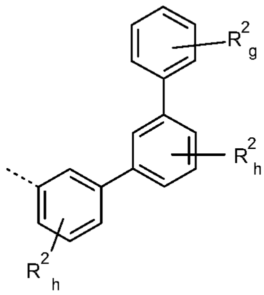

formule ($R^1$-11)

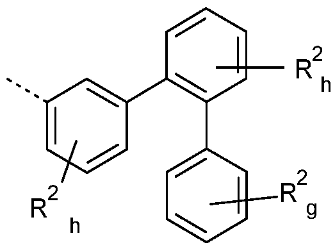

formule ($R^1$-12)

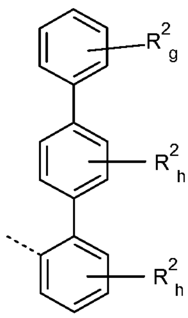

formule ($R^1$-13)

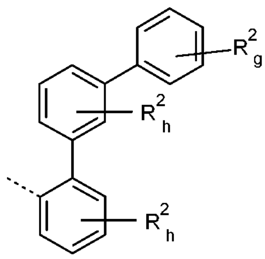

formule ($R^1$-14)

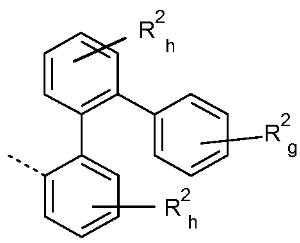

formule ($R^1$-15)

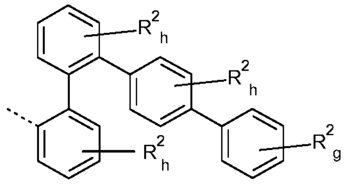

formule ($R^1$-16)

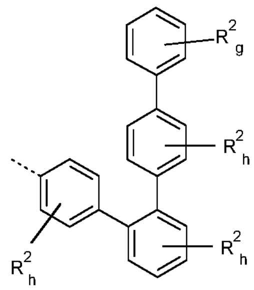

formule ($R^1$-17)

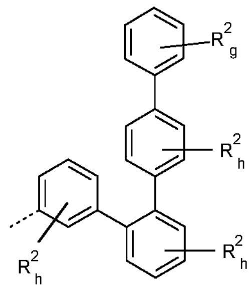

formule ($R^1$-18)

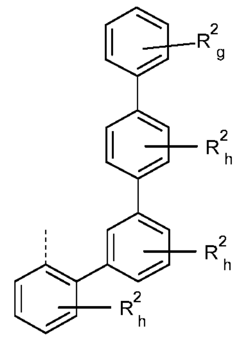

formule ($R^1$-19)

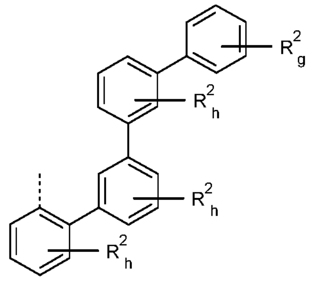

formule ($R^1$-20)

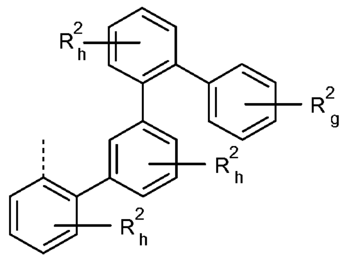

formule ($R^1$-21)

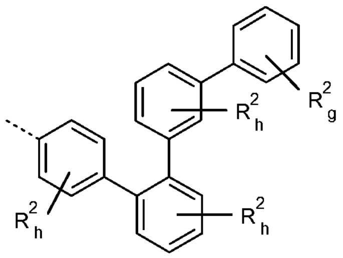

formule ($R^1$-22)

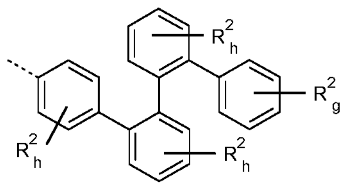

formule ($R^1$-23)

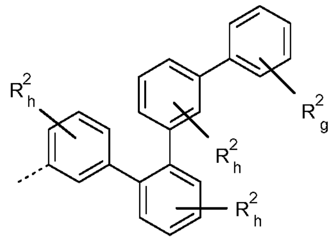

formule ($R^1$-24)

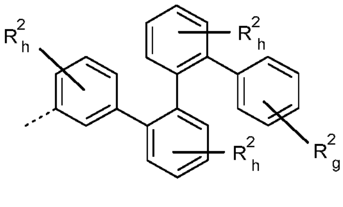

formule ($R^1$-25)

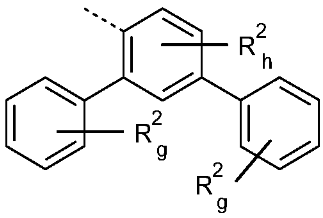

formule ($R^1$-26)

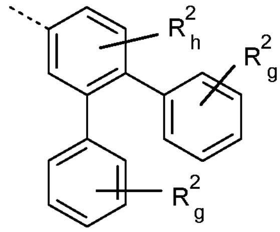

formule ($R^1$-27)

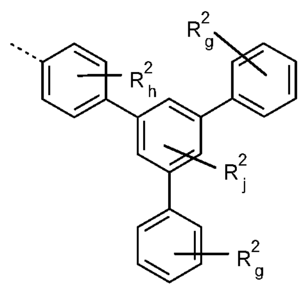

formule ($R^1$-28)

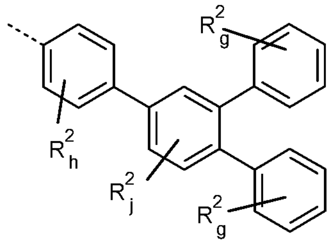

formule ($R^1$-29)

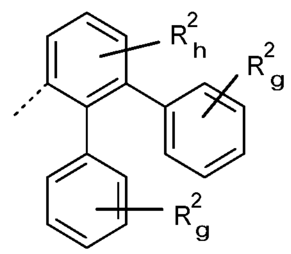

formule ($R^1$-30)

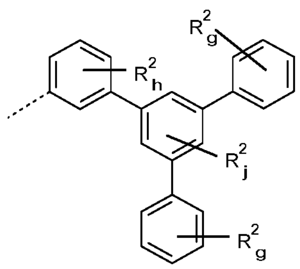

formule ($R^1$-31)

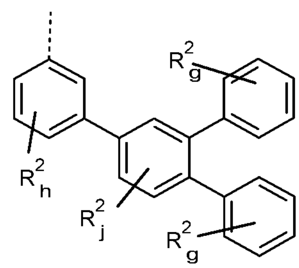

formule ($R^1$-32)

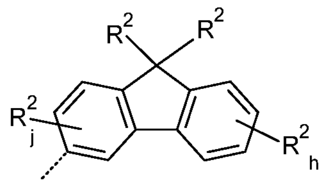

formule ($R^1$-33)

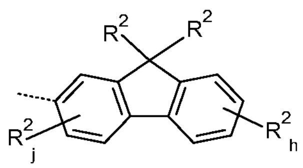

formule ($R^1$-34)

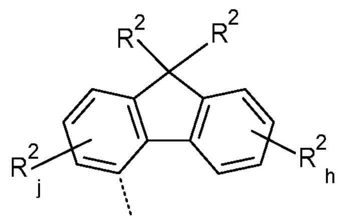

formule ($R^1$-35)

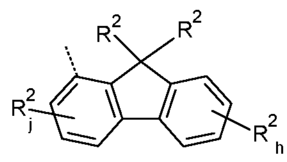

formule ($R^1$-36)

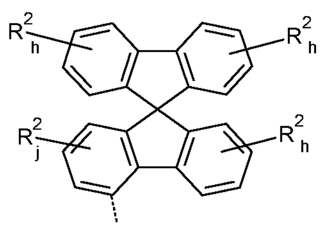

formule ($R^1$-37)

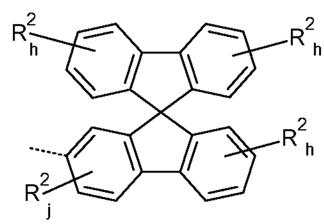

formule ($R^1$-38)

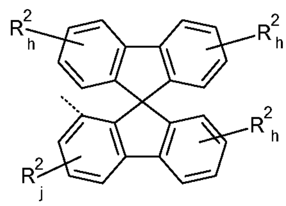

formule ($R^1$-39)

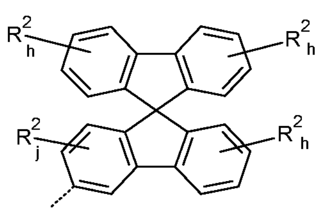

formule ($R^1$-40)

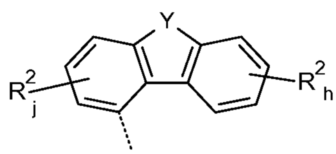

formule ($R^1$-41)

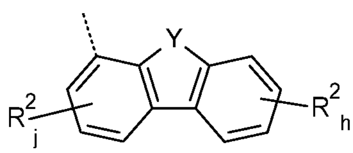

formule ($R^1$-42)

| | | |
|---|---|---|
| 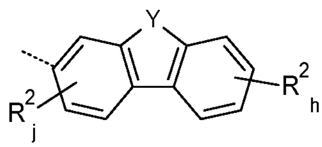 | 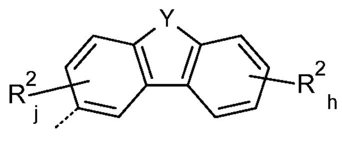 | 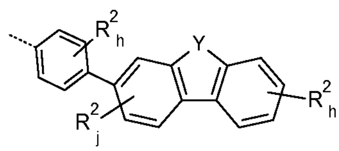 |
| formule ($R^1$-43) | formule ($R^1$-44) | formule ($R^1$-45) |
| 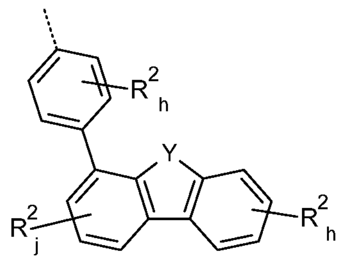 | 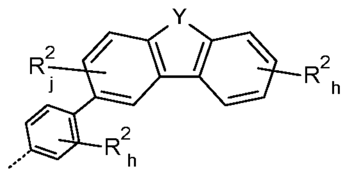 | 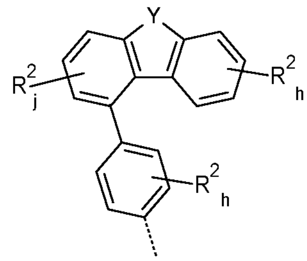 |
| formule ($R^1$-46) | formule ($R^1$-47) | formule ($R^1$-48) |
| 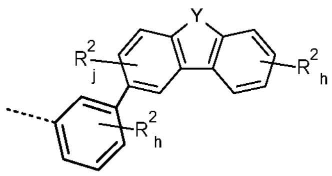 | 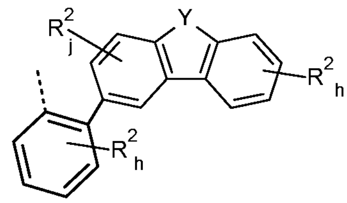 | 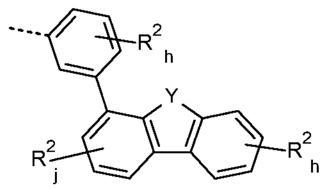 |
| formule ($R^1$-49) | formule ($R^1$-50) | formule ($R^1$-51) |
| 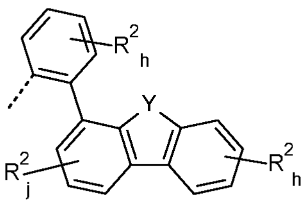 | 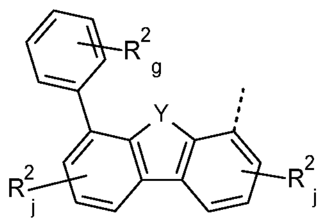 | 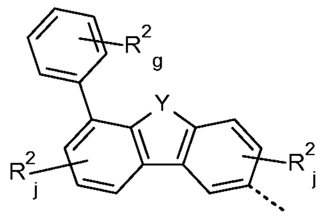 |
| formule ($R^1$-52) | formule ($R^1$-53) | formule ($R^1$-54) |
| 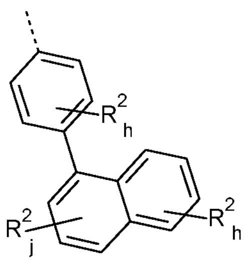 | 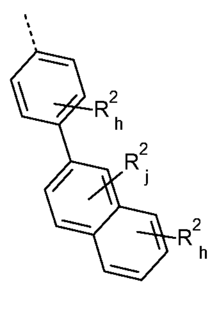 | 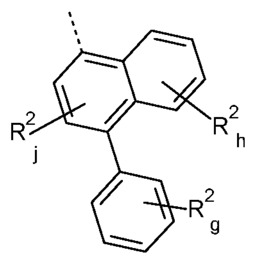 |
| formule ($R^1$-55) | formule ($R^1$-56) | formule ($R^1$-57) |

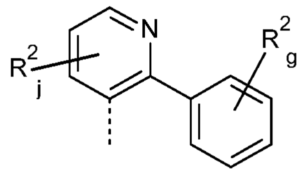

formule ($R^1$-58)

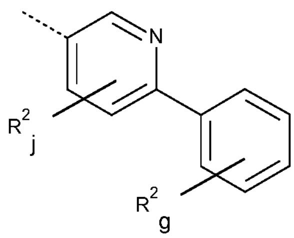

formule ($R^1$-59)

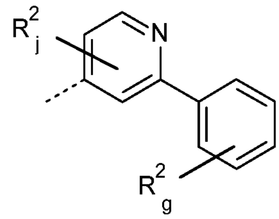

formule ($R^1$-60)

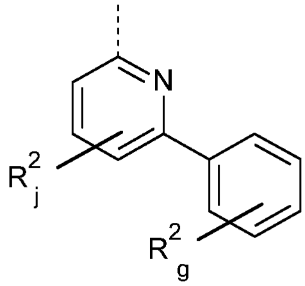

formule ($R^1$-61)

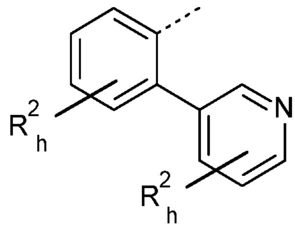

formule ($R^1$-62)

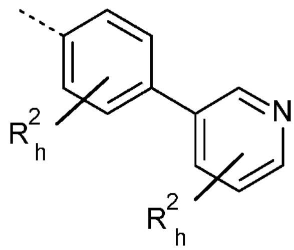

formule ($R^1$-63)

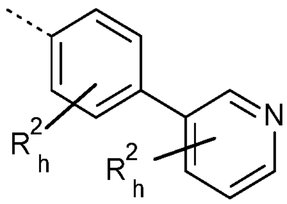

formule ($R^1$-64)

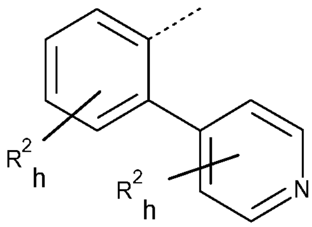

formule ($R^1$-65)

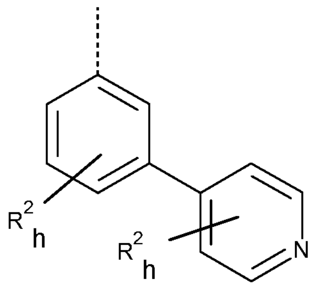

formule ($R^1$-66)

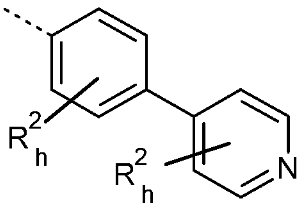

formule ($R^1$-67)

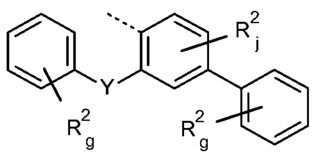

formule ($R^1$-68)

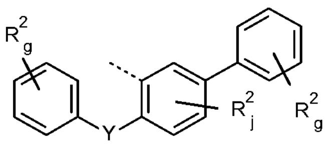

formule ($R^1$-69)

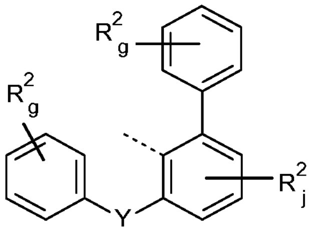

formule ($R^1$-70)

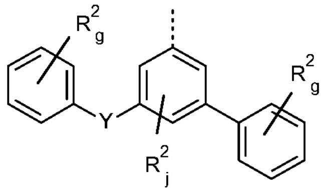

formule ($R^1$-71)

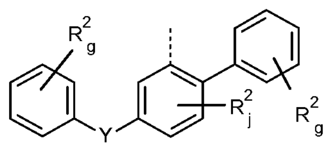

formule ($R^1$-72)

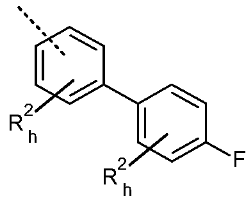

formule ($R^1$-73)

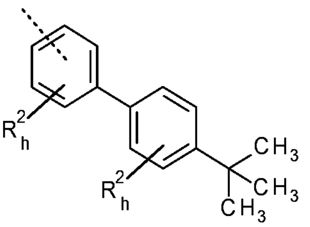

formule ($R^1$-74)

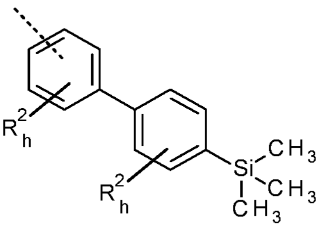

formule ($R^1$-75)

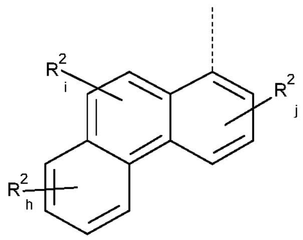

formule ($R^1$-76)

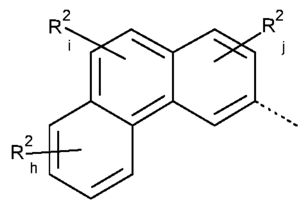

formule ($R^1$-77)

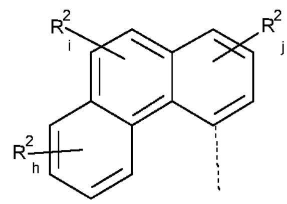

formule ($R^1$-78)

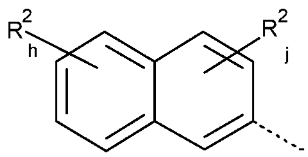

formule ($R^1$-79)

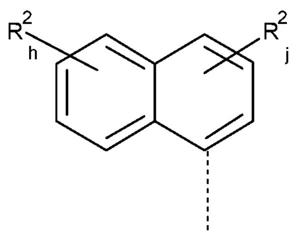

formule ($R^1$-80)

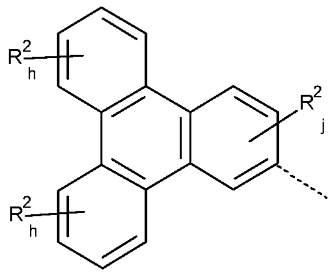

formule ($R^1$-81)

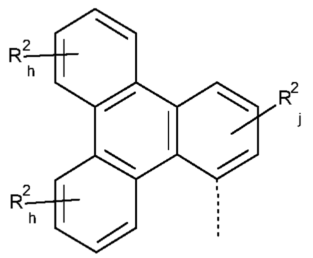

formule ($R^1$-82)

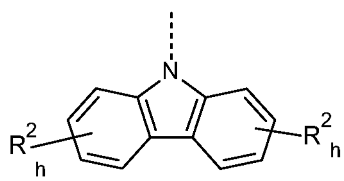

formule ($R^1$-83)

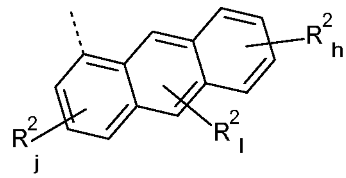

formule ($R^1$-84)

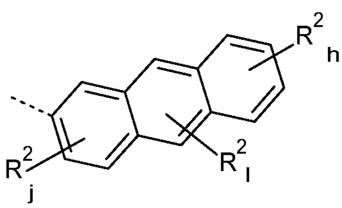

formule ($R^1$-85)

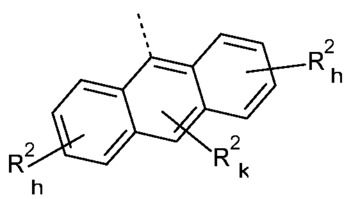

formule ($R^1$-86)

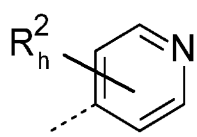

formule ($R^1$-87)

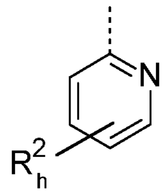
formule ($R^1$-88)

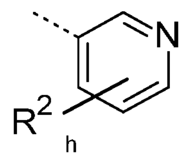
formule ($R^1$-89)

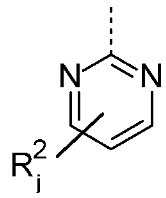
formule ($R^1$-90)

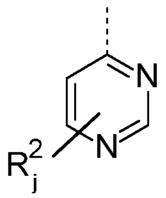
formule ($R^1$-91)

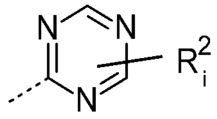
formule ($R^1$-92)

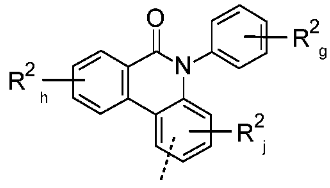
formule ($R^1$-93)

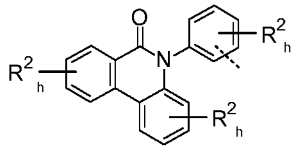
formule ($R^1$-94)

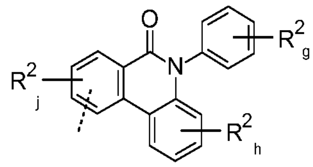
formule ($R^1$-95)

dans lesquelles ce qui suit s'applique aux symboles utilisés:

Y est O, S ou $NR^2$, préférablement O ou S ;
i vaut à chaque occurrence, indépendamment, 0, 1 ou 2 ;
j vaut à chaque occurrence, indépendamment, 0, 1, 2 ou 3 ;
h vaut à chaque occurrence, indépendamment, 0, 1, 2, 3 ou 4 ;
g vaut à chaque occurrence, indépendamment, 0, 1, 2, 3, 4 ou 5 ; et

la ligne en pointillés marque la position de liaison ;

$R^1$ est choisi à chaque occurrence, de manière identique ou différente, dans le groupe constitué par H, D, F, CN, $N(Ar^1)_2$, un groupement alkyle à chaîne linéaire ayant de 1 à 8 atomes de C, ou un groupement alkyle ramifié ou cyclique ayant de 3 à 8 atomes de C, ou un groupement alcényle ayant de 2 à 8 atomes de C, qui peut dans chaque cas être substitué par un ou plusieurs radicaux $R^2$, ou un noyau aromatique ou hétéroaromatique ayant de 6 à 24 atomes de cycle aromatique, qui peut dans chaque cas être substitué par un ou plusieurs radicaux non aromatiques $R^1$ ;
$Ar^1$ est, de manière identique ou différente à chaque occurrence, un noyau aromatique ou hétéroaromatique ayant de 5 à 40 atomes de cycle aromatique, qui peut dans chaque cas être substitué par un ou plusieurs radicaux $R^2$ ;
$R^2$ est choisi à chaque occurrence, de manière identique ou différente, dans le groupe constitué par H, D, un radical hydrocarboné aliphatique ayant de 1 à 10 atomes de C, ou un noyau aromatique ou hétéroaromatique ayant de 5 à 30 atomes de cycle aromatique, qui peut être substitué par un ou plusieurs groupements alkyle ayant dans chaque cas de 1 à 4 atomes de carbone ;

où, dans la formule (I) au plus 2 parmi les symboles $X^1$, $X^2$, $X^3$, $X^4$, $X^5$, $X^6$, $X^7$, $X^8$, $X^9$, $X^{10}$, $X^{11}$, $X^{12}$, $X^{13}$, $X^{14}$, $X^{15}$, $X^{16}$ ne sont pas égaux à CH ou CD.

2. Oligomère, polymère ou dendrimère contenant un ou plusieurs composés selon la revendication 1, où, au lieu d'un atome d'hydrogène ou d'un substituant, une ou plusieurs liaisons sont présentes entre les composés et le polymère, l'oligomère ou le dendrimère.

3. Composition comprenant au moins un composé selon la revendication 1 ou un oligomère, polymère ou dendrimère selon la revendication 2 et au moins un autre composé choisi dans le groupe constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

4. Formulation comprenant au moins un composé selon la revendication 1 ou un oligomère, polymère ou dendrimère selon la revendication 2 ou une composition selon la revendication 3, et au moins un solvant.

5. Utilisation d'un composé selon la revendication 1, d'un oligomère, polymère ou dendrimère selon la revendication 2 ou d'une composition selon la revendication 3, dans un dispositif électronique comme émetteur fluorescent, matériau hôte pour les émetteurs phosphorescents, matériau de transport d'électrons ou matériau de transport de trous, préférablement comme matériau hôte pour les émetteurs phosphorescents ou comme matériau de transport de trous.

6. Dispositif électronique comprenant au moins un composé selon la revendication 1, un oligomère, polymère ou dendrimère selon la revendication 2 ou une composition selon la revendication 3, le dispositif électronique étant préférablement choisi dans le groupe constitué par les dispositifs électroluminescents organiques, les circuits intégrés organiques, les transistors organiques à effet de champ, les transistors organiques en couche mince, les transistors organiques émetteurs de lumière, les cellules solaires organiques, les détecteurs optiques organiques, les photorécepteurs organiques, les dispositifs organiques à extinction de champ, les cellules électrochimiques émettrices de lumière ou les diodes laser organiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2000022026 A
- EP 707020 A
- EP 894107 A
- WO 2006061181 A
- WO 9218552 A
- WO 2004070772 A
- WO 2004113468 A
- EP 1028136 A
- WO 2005014689 A
- WO 2004041901 A
- WO 2004113412 A
- WO 2005040302 A
- WO 2005104264 A
- WO 2007017066 A
- US 7294849 B
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2016124304 A
- WO 2016015815 A
- WO 2016000803 A
- WO 2015117718 A
- WO 2015104045 A
- WO 2015036074 A
- WO 2005011013 A
- WO 2004013080 A
- WO 2004093207 A
- WO 2006005627 A
- WO 2010006680 A
- WO 2014015935 A
- WO 2005039246 A
- US 20050069729 A
- JP 2004288381 A
- EP 1205527 A
- WO 2008086851 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 005111172 A
- WO 2006117052 A
- WO 2010015306 A
- EP 652273 A
- WO 2009062578 A
- WO 2010054729 A
- WO 2010054730 A
- US 20090136779 A
- WO 2010050778 A
- WO 2011042107 A
- WO 2011088877 A
- WO 2012143080 A
- WO 2012048781 A
- WO 2011116865 A
- WO 2011137951 A
- WO 2013064206 A
- WO 2014094963 A
- WO 2015192939 A
- WO 2010108579 A
- WO 2005053051 A
- WO 2009030981 A
- WO 2004058911 A

**In der Beschreibung aufgeführte Nicht-Patentliteratur**


- **D. M. KOLLER et al.** *Nature Photonics,* 2008, 1-4
- **B. M. S. ARNOLD et al.** *Appl. Phys. Lett.,* 2008, vol. 92, 053301